# EUROPEAN PATENT APPLICATION

(11) **EP 4 345 167 A1**
(43) Date of publication of application: **03.04.2024**
(21) Application number: 22382917.7
(22) Date of filing: 30.09.2022
(51) Int. Cl.: C12Q 1/6806, C12N 15/10

(54) **METHOD TO ANALYZE TRNA USING DIRECT SEQUENCING**

(71) Applicant: Fundació Centre de Regulació Genòmica, 08003 Barcelona (ES)
(72) Inventor: NOVOA PARDO, Eva, María, 08003 Barcelona (ES); LUCAS, Morghan, Charlotte, Page, Barcelona, 08003 (ES); PRYSZCZ, Leszek, Piotr, 08003 Barcelona (ES)
(74) Representative: Freyria Fava, Cristina

(57) **Abstract**

The present invention discloses a method to quantify tRNA abundance and tRNA modifications in an RNA sample that comprises contacting RNA with oligonucleotides in the presence of a ligating agent and performing nanopore direct sequencing. It also discloses a kit to perform said method.

## Description

### Field of the invention

Provided is a direct and fast method for detecting and quantifying modifications present in Transfer RNA (tRNAs) in a biological sample. A kit to perform said method is also provided

### Background

tRNAs are highly-modified small non-coding RNAs that play a pivotal role in protein translation. Modifications present in tRNAs are key for their stability, and act as identity elements for correct aminoacylation, ensuring that the fidelity of the genetic code is maintained. Despite their central role in protein translation, tRNA modifications are reversible and can be dynamically regulated upon environmental exposures, across cell cycle stages, and upon tumorigenesis. Similarly, tRNA abundances are also dysregulated upon environmental exposures, as well as in several human diseases. Therefore, quantifying both tRNA abundances as well as tRNA modification dynamics is essential to understand the function and regulation of tRNAs, as well as to understand how and why tRNA dysregulation is linked to diverse human diseases.

Quantification of tRNA modifications is typically achieved by using Mass Spectrometry-based methods (LC-MS/MS), which are highly sensitive but do not provide positional information. On the other hand, quantification of tRNA abundances is achieved using next generation sequencing (NGS)-based methods, which requires an initial conversion of the tRNA molecules into cDNA. Consequently, NGS-based methods are blind to most tRNA modifications.

A promising alternative to the use of NGS-based technologies to characterize the tRNAome is the direct RNA nanopore sequencing (DRS) platform developed by Oxford Nanopore Technologies (ONT), which can sequence the native tRNA molecules, and can in principle detect and measure both tRNA modifications and tRNA abundances, without the need of reverse transcription or PCR. Previous works have demonstrated the feasibility of sequencing native tRNAs using nanopores, using either solid-state or biological (from Oxford Nanopore Technologies) nanopores.

A limitation of the method described in those articles is that the tRNA reads could not be base-called, and consequently, could not be mapped.

The scientific article Thomas NK, Poodari VC, Jain M, Olsen HE, Akeson M, Abu-Shumays RL. Direct Nanopore Sequencing of Individual Full Length tRNA Strands. ACS Nano. 2021;15(10):16642-16653. doi:10.1021/acsnano.1c06488 provides a method to directly quantify and sequence individual tRNA. However, this method is much lengthier and inefficient compared to the method of the current invention. This document discloses a gel purification step which increases the required input amount (gel purification has low RNA recovery), causes fragmentation of the RNA molecules, and increases the complexity and time required for the library preparation. Moreover, the oligonucleotide design of said document (5' and 3' oligonucleotides that ligate to the tRNA molecule) is incompatible with linearization of the tRNA molecule, thus leading to decreased sequencing yields, partly due to pore clogging and the need for gel-mediated tRNA selection, which contributes to tRNA fragmentation. In addition, the yield of tRNA reads obtained per sequencing run was extremely low (about 50,000 reads, compared to 1-2M reads that are typically obtained from DRS runs). Moreover, it is not disclosed whether exact in vivo tRNA abundances and/or tRNA modifications were recapitulated using this method. The present invention demonstrates that this method is insufficient to correctly recapitulate the tRNA abundances present in a given sample. Finally, this previous method does not allow for sample multiplexing, limiting its applicability to samples where RNA amounts are not limiting.

The present invention relates to a nanopore-based method that allows fast, accurate and direct measurement of tRNA abundances and modifications with at least about 10 times more tRNA reads than the previous works, recapitulates tRNA modifications at their sites, simplifies the library preparation steps and consequently the time required for the library preparation, and allows for sample multiplexing.

Collectively, the method of the invention is a sensitive and accurate method for quantification of tRNA abundance and modification profiles with single-transcript resolution. The robust and simple library preparation workflow can be completed within a day, and sequencing within one to two days, being faster than the methods disclosed in the state of the art.

### Description

In the present specification the term "complementary" and "complementarity" are interchangeable and refer to the ability of polynucleotides to form base pairs with one another. Base pairs are typically formed by hydrogen bonds between nucleotide units in antiparallel polynucleotide strands or regions. Complementary polynucleotide strands or regions can base pair in the Watson-Crick manner (e.g., A to T, A to U, C to G). 100% (or total) complementary refers to the situation in which each nucleotide unit of one polynucleotide strand or region can hydrogen bond with each nucleotide unit of a second polynucleotide strand or region. Less than perfect (or partial) complementarity refers to the situation in which some, but not all, nucleotide units of two strands or two regions can hydrogen bond with each other and can be expressed as a percentage.

In the present specification the term "hybridization" is used to refer to the structure formed by 2 independent strands of RNA that form a double stranded structure via base pairings from one strand to the other. These base pairs are considered to be G-C, A-U and G-U. (A - Adenine, C -Cytosin, G-Guanine, U - Uracil). As in the case of the complementarity, the hybridization can be total or partial

In the present specification the expression "basecalling errors' are mismatches, insertions and deletions that are generated in DRS.

In the present specification the expression "5' RNA adapter" is synonym to "first splinted oligonucleotide" and to "first splinted RNA oligonucleotide" and can be used interchangeably.

In the present specification the expression "3' RNA adapter" is synonym to "second splinted oligonucleotide", and to "second splinted RNA oligonucleotide" and when this oligonucleotide has at least 1 DNA nucleotide starting from its 3' end or extrme is termed "second splinted RNA:DNA oligonucleotide" and can be used interchangeably.

In the present specification the expression "ONT RTA adapter A" is synonym to "first adapter DNA nucleotide" and can be used interchangeably.

In the present specification the expression "ONT RTA adapter B" is synonym to "second adapter DNA oligonucleotide" and can be used interchangeably.

In the present specification the term "oligonucleotide" refers to RNA, DNA, or RNA:DNA oligonucleotides.

In the present specification the term "nucleotide" can refer to both, ribonucleotide or deoxyribonucleotide, unless otherwise explained.

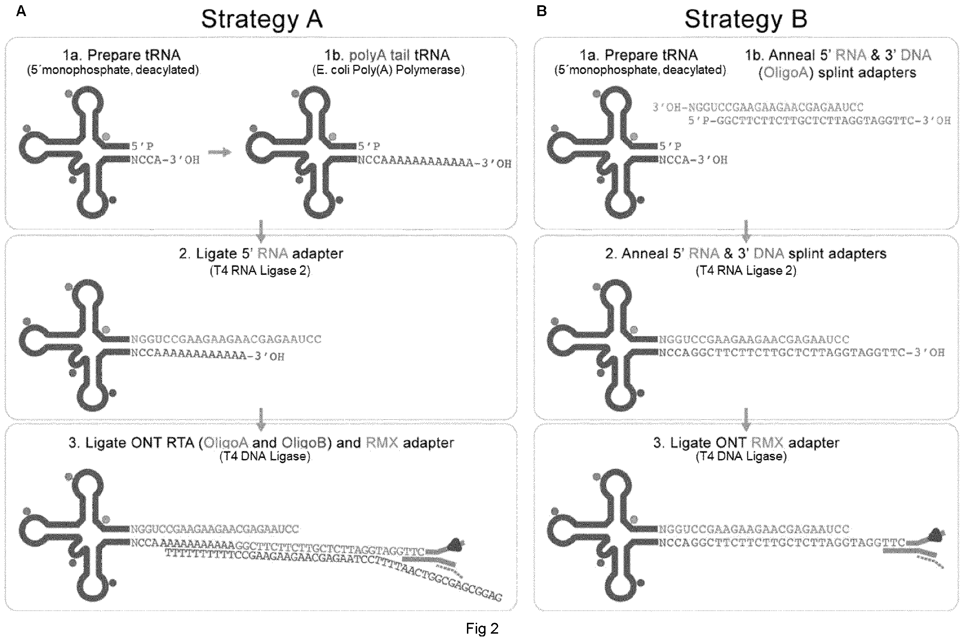

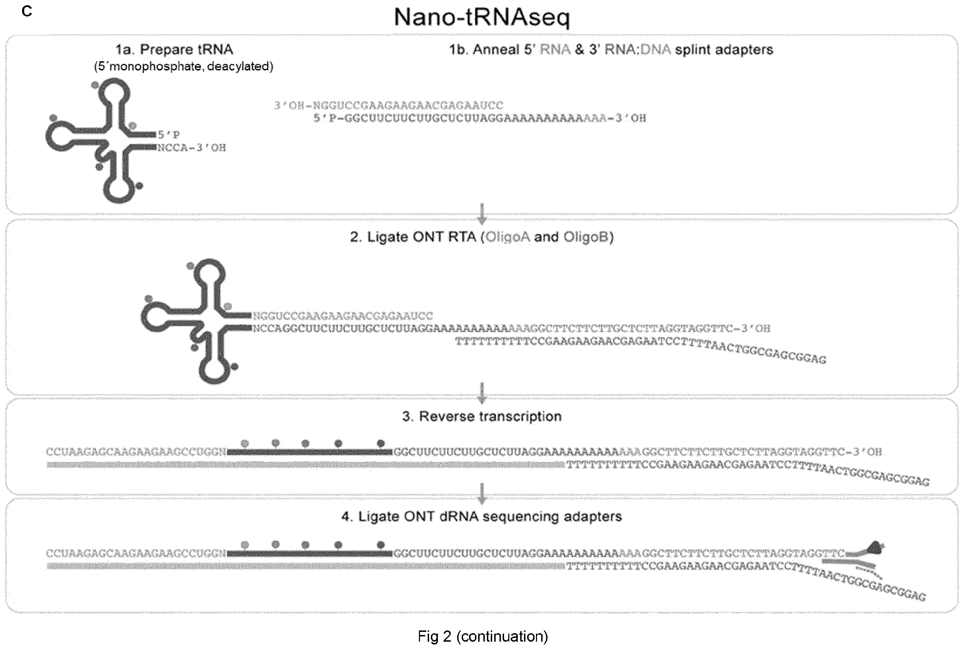

A first object of the invention relates to a method to quantify tRNA abundance and tRNA modifications that comprises the following steps:
a) contacting an RNA sample containing tRNA, in the presence of an agent with ligating activity, with:
   a pre-annealed splinted double-stranded oligonucleotide comprising:
   - a first splinted oligonucleotide, comprising a second splinted oligonucleotide hybridization region and a tRNA hybridization region, said tRNA hybridization region being located on its 3' end,
   - a second splinted oligonucleotide, comprising a first splinted oligonucleotide hybridization region, a second adapter DNA oligonucleotide hybridization region,
   such that at the end of the step, the first splinted oligonucleotide is adjacent to the 5' end of the tRNA and complementary annealed to both the 3' end of the tRNA by the tRNA hybridization region and to the second splinted oligonucleotide by the second splinted oligonucleotide hybridization region. Fig 2, Nano-tRNA seq, box 1
b) contacting the product of step a) in the presence of an agent with ligating activity with
   a pre-annealed adapter DNA double-stranded oligonucleotide comprising:
   - a first adapter DNA oligonucleotide, comprising a second DNA adapter oligonucleotide hybridization region, and
   - a second adapter DNA oligonucleotide, comprising a first DNA adapter oligonucleotide hybridization region and a second splinted oligonucleotide hybridization region, being said second splinted oligonucleotide hybridization region complementary to the second adapter DNA oligonucleotide hybridization region of the second splinted oligonucleotide.
   such that at the end of the step, the first adapter DNA oligonucleotide is adjacent to the 3' end of the terminal region of the second splinted RNA oligonucleotide, and the second DNA oligonucleotide is complementary annealed to both the second splinted RNA oligonucleotide and the first adapter DNA oligonucleotide. See Fig 2, Nano-tRNA seq, box 2
c) performing reverse transcription to linearize the product of step b) to obtain a library (Fig 2, Nano-tRNA seq, box 3) and
d) carrying out a direct nanopore direct sequencing of the library of step c) to obtain the abundance of tRNA and its modifications in said sample (Fig 2, Nano-tRNA seq, box 4).

In a particular embodiment, step d) is divided in the following sub-steps:
e) contacting the library of step c), in the presence of an agent with ligating activity, with an oligonucleotide adapter configured to perform nanopore direct sequencing
f) loading the product of the previous step to a flow cell which contains a membrane in which is present a nanopore that provides a channel through said membrane, coupled to a current intensity, wherein the product of the previous step passes through the nanopore, causes disruptions in the current intensity, and
g) analyzing said sequences to obtain the abundance of tRNA and its modifications in said sample.

tRNAs are generally between 60-120 nucleotides, while messenger RNAs (mRNAs) are much longer; there are other small RNAs in the cell but tRNAs usually account for the largest proportion of small RNA species. Thus, in another particular embodiment, the RNA sample containing in tRNA is an RNA sample wherein at least 10%, preferably at least 20%, more preferably at least 30%, even more preferably at least 40%, even more preferably at least 50%, even more preferably at least 60%, even more preferably at least 70%, even more preferably at least 80%, even more preferably at least 85%, even more preferably at least 90%, even more preferably at least 95%, even more preferably 100% of the RNAs have a length of less than 300 nucleotides, preferably less than 200 nucleotides, more preferably less than 150 nucleotides, but in any case, equal or more than 40 nucleotides. Therefore, the RNA sample to be sequenced contains tRNA species.

In a particular embodiment, an RNA sample containing tRNA is an RNA sample enriched in tRNA.

An RNA sample enriched in tRNA is an RNA sample in which at least 1% of the RNA molecules of said sample are tRNA, preferably at least 2%, more preferably at least 3%, even more preferably at least 5%, even more preferably at least 7%, even more preferably at least 10% of the RNA molecules of said sample are tRNA, even more preferably at least 20% of the RNA molecules of said sample are tRNA, even more preferably at least 30% of the RNA molecules of said sample are tRNA, even more preferably at least 40% of the RNA molecules of said sample are tRNA, even more preferably at least 50% of the RNA molecules of said sample are tRNA, even more preferably at least 60% of the RNA molecules of said sample are tRNA, even more preferably at least 70% of the RNA molecules of said sample are tRNA, even more preferably at least 80% of the RNA molecules of said sample are tRNA, even more preferably at least 90% of the RNA molecules of said sample are tRNA and even more preferably 100% of the RNA molecules of said sample are tRNA.

In a particular embodiment, the agent with ligating activity can be an enzyme or a chemical agent, preferably an enzyme. In other aspects, the ligating is by enzymatic ligation. Suitable reagents (e.g., ligases and corresponding buffers, etc.) and kits for performing enzymatic ligation reactions are known and available, e.g., the Instant Sticky-end Ligase Master Mix available from New England Biolabs (Ipswich, MA). Ligases that may be employed include, e.g., T4 RNA ligase 2 from NEB (NEB #M0239). Conditions suitable for performing the ligation reaction will vary depending upon the type of ligase used. Information regarding such conditions is readily available.

However, commercially available ligases have a generally low concentration for the purpose of this specific ligation. Thus, in a preferred embodiment the ligase enzyme is E. *coli* T4 Rnl2 (gp24.1) its nucleotide sequence is SEQ ID N° 1 and the codon optimized sequence SEQ ID N° 2 and the concentration is in the range of 1 to 20 mg/ml, preferably from 2 to 11 mg/ml, preferably from 2 to 10 mg/ml, more preferably from 4 to 9 mg/ml. The concentration of the ligase is important to reduce the time required for an effective ligation of the tRNA and the pre-annealed splinted double-stranded oligonucleotide, while keeping the reaction volume to a minimum.

In another particular embodiment, step a) is between 10 min and 6hr, preferably between 20 min and 5 hr, more preferably between 30 min and 4 hr, even more preferably between 45 min and 4 hr, even more preferably between 1hr and 3hr at room temperature, this is, between 17°C and 29°C, preferably between 18°C and 28°C, more preferably between 19°C and 27°C, even more preferably between 20°C and 26°C.

In a preferred embodiment step a) is between 1hr and 3hr at a temperature, between 20°C and 26°C. In another particular embodiment, step a) is between 8hr and 16hr at a temperature between 2°C and 6°C.

is between 4hr and 24hr, preferably between 5hr and 22hr, more preferably between 6hr and 20hr, even more preferably between 7hr and 18hr, even more preferably between 8hr and 16hr at a temperature between 1°C and 15°C, preferably between 1,5°C and 12°C, more preferably between 2°C and 9°C, even more preferably between 2°C and 6°C.

In another preferred embodiment step a) is between 8hr and 16hr at a temperature, between 2°C and 6°C.

In an additional particular embodiment, a crowding agent is added during the ligation to maximize its efficiency, and the crowding agent is selected from the group consisting of Polyethylene glycol) (PEG) 8000, PEG 6000, or any other commercial crowding agent and combinations thereof. In a particular embodiment the crowding agent is PEG8000 about 10% w/v, or about 20% w/v, or about 30% w/v or about 40% w/v or about 50% w/v, preferably between 15% w/v and 25 % w/v

In a particular embodiment, before step a) the RNA composition containing tRNA can be contacted with an agent comprising a deacylation activity. The advantage of this step is to increase the ligation yield of the following step, as it prevents the ligation of the second splinted oligonucleotide.

From the original deacylation protocol disclosed in Dittmar KA, Goodenbour JM, Pan T (2006) Tissue-Specific Differences in Human Transfer RNA Expression. PLoS Genet 2(12): e221. https://doi.org/10.1371/journal.pgen.0020221 (See Material and Methods, section tRNA microarrays, second paragraph), the deacylation step was optimized by reducing the reaction volume (final 10uL RNA in dH20, and 95 uL Tris-HCI pH9.0), omitting the neutralisation step, and increasing the ethanol volume added during the column clean-up to collect the deacylated RNA composition containing tRNA.

The RNA sample containing tRNA used in the methods described herein may be from any source including, human beings, animals, plants, bacteria and fungi or yeast. For example, a body fluid (blood or plasma), tissue sample, organ, organelle, or single cells obtained using methods known in the art. Commercial kits are available for isolation of RNA including, for example, RNeasy Kits (Qiagen). Approaches, reagents and kits for isolating, purifying and/or concentrating RNA from sources of interest are known in the art and commercially available. For example, kits for isolating RNA from a source of interest include the nucleic acid isolation/purification kits by Qiagen, Inc. (Germantown, Mc); the ChargeSwitch^{®}, Purelink^{®}, nucleic acid isolation/purification kits by Life Technologies, Inc. (Carlsbad, CA). In certain aspects, the nucleic acid is isolated from a fixed biological sample, e.g., formalin-fixed, paraffin-embedded (FFPE) tissue. RNE from FFPE tissue may be isolated using commercially available kits - such as the AllPrep^{®} DNA/RNA FFPE kit by Qiagen, Inc. (Germantown, Me), and the RecoverAII^{®} Total Nucleic Acid Isolation kit for FFPE by Life Technologies, Inc. (Carlsbad, CA).

In a particular embodiment the RNA composition containing tRNA is treated with a DNAse before the method of the invention.

Both splinted and adapter oligonucleotides need to be pre-annealed before steps a) and b) of the method of the invention in order to obtain double-stranded oligonucleotides.

For the pre-annealing of the splinted oligonucleotides, both the first splinted oligonucleotide and the second splinted oligonucleotide are mixed in a molar ratio about 3:1, 2.5:1, 2:1, 1,5:1, or about 1:1 molar ratio first splinted oligonucleotide: second splinted oligonucleotide. In a particular embodiment said first and second splinted oligonucleotides are mixed in a molar ratio about 1:1, 1:1.5, 1:2, 1:2.5, or about 1:3 first splinted oligonucleotide: second splinted oligonucleotide, preferably a 1:1 molar ratio.

For the pre-annealing of the adapter oligonucleotides, both the first adapter DNA oligonucleotide and the second DNA oligonucleotide are mixed in a molar ratio about 3:1, 2.5:1, 2:1, 1,5:1, 1.2:1 or about 1:1 first adapter DNA oligonucleotide: DNA oligonucleotide. In a particular embodiment said first and second adapter DNA oligonucleotides are mixed in a molar ratio about 1:1, 1:1.2, 1:1.5, 1:2, 1:2.5, or about1:3 first adapter DNA oligonucleotide: DNA oligonucleotide, preferably a 1:1 molar ratio.

The conditions for the annealing can be the general conditions used in the field following regular protocols known by a skilled person. In a particular embodiment the above-mentioned splinted or adapter oligonucleotides are pre-annealed in a solution of about 8-12 mM Tris-HCI (pH 7.5), 45-55 mM NaCl and RNasin^{®} Ribonuclease Inhibitor (Promega,N251A), with a final concentration of about 50 ng/µL, and heated to 65°C to 85C for about 15 s and cooled to 18 to 27°C at a rate of about 0.1°C/s to hybridize said adapters.

In a particular embodiment, the nucleotides of the first splinted oligonucleotide are selected from RNA, DNA or combinations thereof, preferably RNA. When the nucleotides are RNA nucleotides, in the present description this oligo can be termed as first splinted RNA oligonucleotide.

In another particular embodiment, the tRNA hybridization region of the first splinted oligonucleotide has at least 3 nucleotides (preferably RNA nucleotides) matching the CCA overhang of the tRNA, this is UGG plus a random nucleotide (N) in the 3' end.

In a particular embodiment, the 3' end sequence of the first splinted oligonucleotide is 5'-rUrGrG(rN)-3', being N any ribonucleotide selected form rA, rU, rG or rC.

In another particular embodiment, the nucleotides of the second splinted oligonucleotide are selected from RNA, DNA or combinations thereof.

In a particular embodiment, the nucleotides of the second splinted oligonucleotide are RNA. Thus, in the present description this oligo can be termed as second splinted RNA oligonucleotide.

In a preferred embodiment, the nucleotides of the second splinted oligonucleotide are both RNA and DNA; concretely, there is at least 1 terminal DNA base starting from its 3' end (preferably 3 terminal DNA bases). Thus, in the present description this oligo can be termed as second splinted RNA:DNA oligonucleotide. The disadvantage of a second splinted DNA oligonucleotide is that the mapping step after the sequencing will be worse. The disadvantage of a second splinted RNA oligonucleotide is that it will self-hybridize during the first ligation step, thus reducing the yield of step a).

The second splinted RNA:DNA oligonucleotide may have at least 1 terminal DNA nucleotide, preferably 2 or 3 terminal DNA nucleotides, preferably 10 or even up to 15 DNA nucleotides starting from the 3' end of said oligonucleotide. The technical effect of these DNA nucleotides is to prevent self-ligation, thus increasing the overall ligation yield.

The terminal DNA nucleotide is selected from the group consisting of A, T, C, G and combinations thereof.

In another particular embodiment, the second adapter DNA oligonucleotide hybridization region of the second splinted oligonucleotide can be any sequence of at least 10 nucleotides, preferably a poly-A tail of at least 10 A nucleotides. This overhang of at least 10 nucleotides ensures that the library (this is, the RNA sample containing tRNA with the first and second splinted oligonucleoide) can be coupled with default ONT oligonucleotides known in the state of the art for direct RNA library preparation (RTA ligation step).

The size of at least 10 nucleotides, preferably 20 nucleotides in the adapter design improves the mappability of the reads. The size has to be as long as possible to improve the mapping of the tRNAs, and it has to be as small as possible so that it can be removed by the clean-up; any commercial RNA cleanup kit can be used, preferably one that retains any size larger than 17nt, such as the Zymo cleanup kit. For this reason, bead clean up can be used as alternative to these clean up kits, which gives more flexibility in terms of which RNAs are lost during the clean-up step.

In a particular embodiment, the terminal DNA nucleotide(s) is part of the second adapter DNA oligonucleotide hybridization region of the second splinted RNA:DNA oligonucleotide. For instance, it can be one, or two, or three "A" of the poly-A tail of 10 A nucleotides.

The first and second splinted oligonucleotides are designed such that when the first and second splint oligonucleotides hybridization regions of those oligos are hybridized to their respective regions, an end of the second splint oligonucleotide is adjacent to the 3' end of the tRNA, and the opposite end of the second splint RNA:DNA oligonucleotide is adjacent to the first adapter DNA oligonucleotide. These adjacent ends may be covalently linked (e.g., by enzymatic ligation, for instance, by T4 RNA ligase 2) which may then be used in a downstream application of interest (e.g., PCR amplification, nano-pore direct sequencing, next-generation sequencing, and/or the like) facilitated by one or more sequences in the oligonucleotides employed.

When both the first and second splinted oligonucleotides are RNA nucleotides steps a) and b) can be performed simultaneously, and the agent with ligating activity can be any commercial T4RNA ligase 2, or preferably SEQ ID No 1, more preferably SEQ ID No 2, or a variant having at least 80 % identity, more preferably 85% identity, even more preferably at least 90 % identity, and even more preferably 91 % or 92 % or 93 % or 94 % or 95 % or 96 % or 97 % or 98 % or even up to 99 % identity with respect to the SEQ ID No 1 or SEQ ID No 2 polynucleotide sequence. In any case the concentration of the ligase must be from 2 to 11 mg/ml, preferably from 2 to 10 mg/ml, more preferably from 4 to 9 mg/ml.

When the first splinted oligonucleotide is RNA and the second splinted oligonucleotide is RNA:DNA step b) needs to be performed after step a) and the agent with ligating activity for step a) can be any commercial T4 RNA ligase 2, or preferably SEQ ID No 1, more preferably SEQ ID No 2 or a variant having at least 80 % identity, more preferably 85% identity, even more preferably at least 90 % identity, and even more preferably 91 % or 92 % or 93 % or 94 % or 95 % or 96 % or 97 % or 98 % or even up to 99 % identity with respect to the SEQ ID No 1 or SEQ ID No 2 polynucleotide sequence. In any case the concentration of the enzyme must be from 2 to 11 mg/ml, preferably from 2 to 10 mg/ml, more preferably from 4 to 9 mg/ml. Any commercial DNA ligase can be used for step b).

In another particular embodiment, the second splinted oligonucleotide hybridization region of the second adapter DNA oligonucleotide can be any sequence as long as it is complementary to the second adapter DNA oligonucleotide hybridization region of the second splinted oligonucleotide. In a particular embodiment, it has at least 10 nucleotides, preferably a poly-T tail of 10 T nucleotides.

Generally, it is important to keep the oligonucleotides as short as possible to reduce costs, but large enough to be correctly mapped during a direct RNA sequencing method such as Nanopore direct sequencing. RNA lengths below 150 nucleotides are badly detected by this technology and RNA lengths below 60 nucleotides are not even detected.

In another particular embodiment, the size of the first splinted oligonucleotide is between 15 and 40 nucleotides, preferably between 20 and 30 nucleotides.

In another particular embodiment, the size of the second splinted oligonucleotide is between 25 and 50 nucleotides, preferably between 28 and 35 nucleotides. A size of 24nt or more is required for efficient basecalling and mapping, to capture the maximal number of tRNA reads.

The second splinted oligonucleotide should be 25nt of RNA or more for optimal read mappability, in a preferred embodiment it is 30nt RNA, to give buffer for potential basecalling issues related to the homopolymer of the 10X polyA tail at the 3' end of the oligo.

In a particular embodiment, the second RNA:DNA splinted oligonucleotide has 3nt DNA bases starting from the 3' end to reduce self-ligation. Even only 1 DNA nucleotide would still avoid self-ligation. A second RNA splinted oligonucleotide would not avoid self-ligation, it but would avoid a cleanup step.

As the first splinted oligonucleotide cannot be basecalled, it is not possible to confirm the minimum length requirement as with the second splinted oligonucleotide, but it should be similar to the 3' oligo, meaning that there should be a 20-25nt RNA buffer at the 5' end. In a preferred embodiment, the 5' first splinted oligonucleotide is 24nt long.

In another particular embodiment, the size of the first adapter DNA oligonucleotide is between 15 and 40 nucleotides, preferably between 20 and 35 nucleotides.

In another particular embodiment, the size of the second adapter DNA oligonucleotide is between 25 and 60 nucleotides, preferably between 30 and 50 nucleotides.

**Table 1 shows a particular example of oligonucleotides that can be used for the method of the invention TABLE 1: Splinted and Adapter oligonucleotides sequences**

| Oligonucleotides | SEQ ID | Sequence |
|---|---|---|
| first splinted RNA oligonucleotide | SEQ ID No 3 | rCrCrUrArArGrArGrCrArArGrArArGrArArGrCrCrUrGrGrN |
| second splinted RNA oligonucleotide | SEQ ID No 4 | /5Phos/rGrGrCrUrUrCrUrUrCrUrUrGrCrUrCrUrUrA rGrGrArArArArArArArArArA |
| second splinted RNA:DNA oligonucleotide | SEQ ID No 5 | /5Phos/rGrGrCrUrUrCrUrUrCrUrUrGrCrUrCrUrUrArGrG rArArArArArArArArArAAAA |
| first adapter DNA oligonucleotide | SEQ ID No 6 | /5Phos/GGCTTCTTCTTGCTCTTAGGTAGTAGGTTC |
| second adapter DNA oligonucleotide | SEQ ID No 7 | GAGGCGAGCGGTCAATTTTCCTAAGAGCAAG AAGAAGCCTTTTTTTTTT |

| | | |
|---|---|---|
| Underlined nucleotides are DNA N can be any nucleotide selected from A, C, T,G | | |

In a particular embodiment, the first RNA splinted oligonucleotide is SEQ ID No 3 and the second splinted oligonucleotide is SEQ ID No 4 or SEQ ID No 5
or a variant having at least 80%, preferably at least 85%, more preferably at least 90 % and even more preferably 91 % or 92 % or 93 % or 94 % or 95 % or 96 % or 97 % or 98 % or even up to 99 % identity with respect to SEQ ID No 3, SEQ ID No 4, SEQ ID No 5.

In a particular embodiment, one or more of the oligonucleotides are modified to have a 5'monophospahte group, because said oligonucleotides, after being chemically synthesized, have a 5'triphosphate group, preferably the second splinted oligonucleotide and the first adapter DNA oligonucleotide. A skilled person would know how to carry on sad modification using common general knowledge.

In a particular embodiment of the invention, one or more oligonucleotides of the invention have a shuffled string of nucleotides that act as a unique identifier of the sample to allow multiplexing, this is, several samples analyzed together simultaneously in steps e) and f) of the method of the invention

This barcode or unique identifier uniquely identifies the first adapter DNA oligonucleotide, the second adapter DNA oligonucleotide or both and/or uniquely identifies the sample source of the RNA containing tRNA being sequenced to enable sample multiplexing by marking every molecule from a given sample with a specific barcode or "tag"); a barcode sequencing primer binding domain (a domain to which a primer used for sequencing a barcode binds); a molecular identification domain (e.g., a molecular index tag, such as a randomized tag of 4, 6, or other number of nucleotides) for uniquely marking molecules of interest, e.g., to determine expression levels based on the number of instances a unique tag is sequenced; a complement of any such domains; or any combination thereof. In certain aspects, a barcode domain (e.g., sample index tag) and a molecular identification domain (e.g., a molecular index tag) may be included in the same nucleic acid.

In a particular embodiment the barcode or unique identifier is in both, the first adapter DNA oligonucleotide and the second adapter DNA oligonucleotide being complementary between said oligonucleotides. As the nanopore direct sequencing will only sequence the nucleic acid chain bound to the first DNA oligonucleotide (and not its complementary chain), this is the barcode (concrete sequence) contained in said oligonucleotide that will be used later for demultiplexing the signal in step f) of the method of the invention.

Although barcodes for a similar approach have been shown before (e.g. Smith MA, Ersavas T, Ferguson JM, et al. Molecular barcoding of native RNAs using nanopore sequencing and deep learning. Genome Res. 2020;30(9):1345-1353. doi:10.1101/gr.260836.120), the present invention expands the repertoire of barcodes, and also includes the possibility of longer sequences. Importantly, the barcodes of the present invention cannot be demultiplexed afterwards using the DeePlexiCon algorithm that was used in said publication.

In this particular embodiment, the oligonucleotide hybridization region of both DNA adapter oligonucleotides can be replaced by the barcode sequence. Thus, it is essential that the barcodes are complementary and have the same size.

An example of a first adapter DNA oligonucleotide for multiplex run is: N(15-45)TAGTAGGTTC. SEQ ID No 8 comprise the last 10 nucleotides, of said first adapter DNA oligonucleotide for multiplex run TAGTAGGTTC, which are also the last 10 nucleotides of SEQ ID No 6

Wherein "N" is a nucleotide selected from the group consisting of A, C, T, G and will contain the barcode.

An example of a second adapter DNA oligonucleotide for multiplex run is: GAGGCGAGCGGTCAATTTTN(15-45)TTTTTTTTTT. SEQ ID No 9 comprise the first 19 nucleotides, of said second adapter DNA oligonucleotide for multiplex run GAGGCGAGCGGTCAATTTT, which are also the first 19 nucleotides of SEQ ID No 7

Wherein "N" is a nucleotide selected from the group consisting of A, C, T, G and will contain the barcode.

The string of 10 "T" of the above mentioned example is the second splinted RNA:DNA oligonucleotide hybridization region, this it can be any other sequence as long as it is complementary with the second adapter DNA oligonucleotide hybridization region of the second splinted RNA:DNA oligonucleotide

In a particular embodiment the barcodes are:
>Barcode_2 SEQ ID No 10: GTGATTCTCGTCTTTCTGCG
>Barcode_3 SEQ ID No 11: GTACTTTTCTCTTTGCGCGG
>Barcode_4 SEQ ID No 12: GGTCTTCGCTCGGTCTTATT
>Barcode_23 SEQ ID No 13: GCAGCTGGATGCGAGAGGGAAGCTGGTTGAAATAATA
>Barcode_48 SEQ ID No 14: GTTCCGCGCACGCTTTTCTCGTTTTGTCTTTTACACT
>Barcode_82 SEQ ID No 15: GGGAAAGAGCGGTACACTCTCCGAGGGAGAGGCCCAC

The reverse transcription of step c) allows for a linearization of the product and this linearization decreases the pore clogging that occurs when sequencing structured RNA molecules during the nanopore direct sequencing and thus increases sequencing yield by 50% compared to other methods without linearization step but with gel purification steps. In addition, gel purification is labor intensive, requires a lot of time and leads to fragmentation of tRNAs.

The conditions for the reverse transcription are known by anyone skilled in the art; for instance, using a temperature comprised between 55°C and 65°C for a period of time between 45 min and 75 min. Any commercial reverse transcriptase can be used. For instance, Maxima Reverse Transcriptase from ThermoFisher, Superscript^{™} II reverse transcriptase or Superscript^{™} IV reverse transcriptase.

The helicase protein locates in one strand of the double-stranded sequencing adapter RNA oligonucleotide possibilities such particular RNA strand to be translocated by the nanopore of the following step at a constant speed and thus sequenced.

In step d), the sequencing of the linearized product of step c) can be performed by any direct sequencing method that comprises a nanopore, for instance Oxford Nanopore technologies. The nanopore direct sequencing and the materials and protocols to perform it are known in the art. For instance, in EP0815438B1.

In a particular embodiment, the oligonucleotide adapter configured to perform nanopore direct sequencing is a double-stranded sequencing adapter DNA oligonucleotide with a helicase protein bound to one of the strands and having the complementary strand, a first DNA adapter oligonucleotide hybridization region (See Fig 2, Nano-tRNA seq, box 3).

In a particular embodiment the nanopore direct sequencing comprises a membrane, said membrane can be either solid-state or biological membranes.

Any known nanopore direct sequencing method or product can be used to perform step f) of the current invention, for instance the one disclosed in EP0815438B1 or EP1238275B1.

The analysis or performing algorithm used in step g) can be any commercial one known by a skilled of many performing algorithms known in the art suitable for nanopore direct RNA sequencing.

The first step is extracting the reads. This step can be done by a commercial software, for instance MinKNOW or any software configured to analyze the sequencing results of the nanopore direct sequencing.

Next step of the analysis is the basecalling, which can be done by several known performing algorithms in the field by a skilled person such as Guppy or Bonito.

Last step of the analysis is to mapping which can be done by several known performing algorithms. For example, Minimap2 or BWA which is a versatile sequence alignment program that aligns nucleic acid sequences against a large reference database.

In a particular embodiment the performing algorithm is configured to capture (and sequence) more tRNAs in a quantitative way compared to the default or adjusted parameters of MinKnow.

The method of the invention coupled to a known algorithm to analyze the nanopore sequencing step provides 10-12 times more reads, of which around 5 times more tRNA reads are mapped, compared to other methods of the state of the art such as Thomas NK, Poodari VC, Jain M, Olsen HE, Akeson M, Abu-Shumays RL. Direct Nanopore Sequencing of Individual Full Length tRNA Strands. ACS Nano. 2021;15(10):16642-16653. doi:10.1021/acsnano.1c06488. and with improved recovery of shorter tRNA molecules, which are otherwise largely lost, leading to biased representations of the tRNA abundances.

In another particular embodiment the performing algorithm in MinKNOW is configured to capture (and sequence) more tRNAs and in a quantitative way.

The current version of MinKNOW (21/07/2022), as well as prior versions of MinKNOW, is optimised to capture long RNA reads (typically >200 nucleotides). It detects valid reads in two steps. A molecule passing through a pore is assigned as:
- an adapter for up to 5 seconds (this may be shorter if signal variability of a molecule is larger than the variability in the adapter definition) and only then
- as strand if strand definition is fulfilled for at least 2 seconds

Only reads passing strand criteria are reported in Fast5 files. Thus, the read has to spend 2-7 seconds in the pore in order to be reported. This corresponds roughly to 150-490 bases.

In an additional particular embodiment, a custom sequencing script was prepared in the commercial software used to carry on the sequencing, MinKNOW version 1.11.5. which is the standard software that drives nanopore sequencing devices.

The parameters of the software configured to analyze the sequencing results of the nanopore direct sequencing (preferably *MinKNOW*) are adjusted for up to 1 second definition for adapter and a maximum of 2 seconds for the strand. By reducing both times the chances of an RNA molecule to be classified as a read are increased and thus reported in Fast5 files.

In a particular embodiment the performing algorithm uses (Burrow-Wheeler Aligner) BWA configuration, and its parameters are selected from the group consisting of:
i) bwa mem -W13 -k6 -xont2d,
ii) bwa mem -W13 -k6 -xont2d -T20,
iii) bwamem -W13 -k6 -xont2d -T10,
iv) bwamem -W9 -k5 -xont2d -T10 and
v) bwasw -z10 -a2 -b1 -q2 -r1.

Preferably bwa mem -W13 -k6 -xont2d -T20

In an additional particular embodiment, the performing algorithm for the multiplexing running comprises minimap2 with following adjusted parameters: -xmap-ont -k6 -w3 -n1 -m10 -s13 -A1 -B1 - O1 -E1. These adjusted configuration allows for a higher sensitivity and better alignments.

Altogether, the present invention provides a simple, cost-effective, high-throughput and reproducible method to accurately reproduce tRNA abundances and capture tRNA modification changes simultaneously, using native tRNA nanopore sequencing, providing a novel framework to study the tRNAome at single molecule resolution, whilst retaining all the tRNA modification information.

Another object of the present invention relates to a Kit for quantifying tRNA abundance and tRNA modifications in a RNA sample containing tRNA.

Such kit comprises reagents for quantifying tRNA abundance and tRNA modifications in a RNA sample containing tRNA disclosed by the methods described herein.

In a particular embodiment, the kit comprises:
- a first splinted oligonucleotide, comprising a second splinted oligonucleotide hybridization region and a tRNA hybridization region, said tRNA hybridization region being located on its 3' end,
- a second splinted oligonucleotide, comprising a first splinted oligonucleotide hybridization region, a second adapter DNA oligonucleotide hybridization region,
- a first adapter DNA oligonucleotide, comprising a second DNA adapter oligonucleotide hybridization region, and
- a second adapter DNA oligonucleotide, comprising a first DNA adapter oligonucleotide hybridization region and a second splinted oligonucleotide hybridization region, being said second splinted oligonucleotide hybridization region complementary to the second adapter DNA oligonucleotide hybridization region of the second splinted oligonucleotide.

In a particular embodiment the nucleotides of the first splinted oligonucleotide are selected from RNA, DNA or combinations thereof, preferably RNA with a 3' end sequence of 5'-rUrGrG(rN)-3'.

In a particular embodiment the nucleotides of the second splinted oligonucleotide are RNA. In another particular embodiment the nucleotides of the second splinted oligonucleotide are both RNA and DNA, concretely, there is at least 1 terminal DNA base starting from its 3' end.

The second splinted RNA:DNA oligonucleotide may have at least 1 terminal DNA nucleotides, preferably 2 or 3 terminal DNA nucleotides, even up to 10 or 15 DNA nucleotides starting from the 3' end of said oligonucleotide.

In another particular embodiment the sequence of the oligonucleotides is the sequence depicted on Table 1 or a variant having at least 80%, preferably at least 85%, more preferably at least 90 % and even more preferably 91 % or 92 % or 93 % or 94 % or 95 % or 96 % or 97 % or 98 % or even up to 99 % identity with respect to one or more of the sequences depicted in Table 1.

In another particular embodiment, the Kit further comprises a double-stranded sequencing adapter DNA oligonucleotide with a helicase protein bound to one of the strands and having the complementary strand, a first DNA adapter oligonucleotide hybridization region.

The Kit further comprises one or more of the following reagents an agent with ligating activity, an agent to perform reverse transcription, solutions for performing deacylation and buffers and solutions required for reverse transcription, ligation and/or deacylation reaction.

In a particular embodiment the agent with ligating activity is an enzyme, preferably a DNA ligase, RNA ligase or both. In a preferred embodiment the RNA ligase is T4 RNA ligase 2. In a more preferred embodiment the nucleotide sequence of the T4 RNA ligase 2 is SEQ ID No 1 or SEQ ID No 2 or a variant having at least 80%, preferably at least 85%, more preferably at least 90 % and even more preferably 91 % or 92 % or 93 % or 94 % or 95 % or 96 % or 97 % or 98 % or even up to 99 % identity with respect to SEQ ID No 1 or SEQ ID No 2.

The kit may further include reagents and solutions to obtain a RNA sample containing tRNA and/or to enrich the amount of tRNA in such RNA sample.

The kit further includes instructions and the required plastic were to perform the method of the invention.

Components of the subject kit may be present in separate containers, or multiple components may be present in a single container. A suitable container includes a single tube (e.g., vial), one or more wells of a plate (e.g., a 96-well plate, a 384-well plate, etc.), or the like.

The instructions for using any of the reagents of the above-mentioned kit to perform the method of the invention may be recorded on a suitable recording medium. For example, the instructions may be printed on a substrate, such as paper or plastic, etc. As such, the instructions may be present in the kits as a package insert, in the labeling of the container of the kit or components thereof (i.e., associated with the packaging or sub-packaging), etc. In other embodiments, the instructions are present as an electronic storage data file present on a suitable computer- readable storage medium, e.g., portable flash drive, DVD, CD-ROM, diskette, etc. In yet other embodiments, the actual instructions are not present in the kit, but means for obtaining the instructions from a remote source, e.g. via the internet, are provided. An example of this embodiment is a kit that includes a web address where the instructions can be viewed and/or from which the instructions can be downloaded. As with the instructions, the means for obtaining the instructions is recorded on a suitable substrate.

Each of the terms "comprising," "consisting essentially of," and "consisting of" may be replaced with either of the other two terms. The term "a" or "an" can refer to one of or a plurality of the elements it modifies (e.g., "a reagent" can mean one or more reagents) unless it is contextually clear either one of the elements or more than one of the elements is described. The term "about" as used herein refers to a value within 10% of the underlying parameter (i.e., plus or minus 10%; e.g., a weight of "about 100 grams" can include a weight between 90 grams and 110 grams). Use of the term "about" at the beginning of a listing of values modifies each of the values (e.g., "about 1, 2 and 3" refers to "about 1, about 2 and about 3"). When a listing of values is described the listing includes all intermediate values and all fractional values thereof (e.g., the listing of values "80%, 85% or 90%" includes the intermediate value 86% and the fractional value 86.4%). When a listing of values is followed by the term "or more," the term "or more" applies to each of the values listed (e.g., the listing of "80%, 90%, 95%, or more" or "80%, 90%, 95% or more" or "80%, 90%, or 95% or more" refers to "80% or more, 90% or more, or 95% or more"). When a listing of values is described, the listing includes all ranges between any two of the values listed (e.g., the listing of "80%, 90% or 95%" includes ranges of "80% to 90%", "80% to 95%" and "90% to 95%"). Certain implementations of the technology are set forth in examples below.

The present invention also discloses the following clauses:
1. A method to quantify tRNA abundance and tRNA modifications that comprises the following steps:
   a) contacting an RNA sample containing tRNA, in the presence of an agent with ligating activity, with:
      a pre-annealed splinted double-stranded oligonucleotide comprising:
      - a first splinted oligonucleotide, comprising a second splinted oligonucleotide hybridization region and a tRNA hybridization region, said tRNA hybridization region being located on its 3' end,
      - a second splinted oligonucleotide, comprising a first splinted oligonucleotide hybridization region, a second adapter DNA oligonucleotide hybridization region,
      such that at the end of the step, the first splinted oligonucleotide is adjacent to the 5' end of the tRNA and complementary annealed to both the 3' end of the tRNA by the tRNA hybridization region and to the second splinted oligonucleotide by the second splinted oligonucleotide hybridization region,
   b) contacting the product of step a) in the presence of an agent with ligating activity with
      a pre-annealed adapter DNA double-stranded oligonucleotide comprising:
      - a first adapter DNA oligonucleotide, comprising a second DNA adapter oligonucleotide hybridization region, and
      - a second adapter DNA oligonucleotide, comprising a first DNA adapter oligonucleotide hybridization region and a second splinted oligonucleotide hybridization region, being said second splinted oligonucleotide hybridization region complementary to the second adapter DNA oligonucleotide hybridization region of the second splinted oligonucleotide,
      such that at the end of the step, the first adapter DNA oligonucleotide is adjacent to the 3' end of the terminal region of the second splinted RNA oligonucleotide, and the second DNA oligonucleotide is complementary annealed to both the second splinted RNA oligonucleotide and the first adapter DNA oligonucleotide,
   c) performing reverse transcription to linearize the product of step b) to obtain a library and
   d) carrying out nanopore direct sequencing with the library of step c), to obtain the abundance of tRNA and its modifications in said sample.
2. The method according to the preceding clause, wherein step d) comprises:
   - contacting the library of step c), in the presence of an agent with ligating activity, with an oligonucleotide adapter configured to perform nanopore direct sequencing,
   - loading the product of the previous step to a flow cell which contains a membrane in which is present a nanopore that provides a channel through said membrane, coupled to a current intensity, wherein the product of the previous step passes through the nanopore, causes disruptions in the current intensity, and
   - analyzing said sequences to obtain the abundance of tRNA and its modifications in said sample.
3. The method according to the any of the preceding clauses, wherein at least 85% of the RNA molecules of the RNA sample containing tRNA have a size comprised between 300 nucleotides and 40 nucleotides
4. The method according to any of the preceding clauses, wherein at least 10% of the RNA molecules of the RNA sample containing tRNA are tRNA.
5. The method according to any of the preceding clauses, wherein the ligating agent of step a) is an enzyme or a chemical agent, preferably an enzyme, more preferably E. *coli* T4 RNA ligase2 being its polynucleotide sequence SEQ ID N° 1 or SEQ ID N° 2 or a variant having at least 80%, preferably at least 85%, more preferably at least 90 % and even more preferably 91 % or 92 % or 93 % or 94 % or 95 % or 96 % or 97 % or 98 % or even up to 99 % identity with respect to SEQ ID No 1 or SEQ ID No 2.
6. The method according to the preceding clause, wherein the concentration of E. *coli* T4 RNAligase2 SEQ ID N° 1 or SEQ ID N° 2 is between 1 to 20 mg/ml.
7. The method according to any of the preceding clauses, wherein the duration of step a) is between 10 min and 6hr at a temperature comprised between 17°C and 29°C.
8. The method according to any of the preceding clauses, wherein the duration of step a) is between 4hr and 24hr at a temperature between 1°C and 15°C.
9. The method according to any of the preceding clauses comprising polyethylene glycol between 15% w/v and 25% w/v.
10. The method according to any of the preceding clauses, wherein the RNA sample containing tRNA is subjected to a deacylation reaction before step a)
11. The method according to any of the preceding clauses, wherein the RNA sample is obtained from any source including, human beings, animals, plants, bacteria and fungi or yeast.
12. The method according to any of the preceding clauses, wherein the RNA composition containing tRNA is treated with a DNAse before step a)
13. The method according to any of the preceding clauses, wherein the first splinted oligonucleotide and the second splinted oligonucleotide are annealed before step a) with a molar ratio selected from 3:1 to 1:3, preferably 1:1.
14.The method according to any of the preceding clauses, wherein the first adapter DNA oligonucleotide and the second DNA oligonucleotide are annealed before step a) with a molar ratio selected from 3:1 to 1:3, preferably 1.2:1
15. The method according to any of the preceding clauses, wherein the first splinted oligonucleotide is selected from RNA, DNA or combinations thereof, preferably RNA.
16. The method according to any of the preceding clauses, wherein the tRNA hybridization region of the first splinted oligonucleotide is 5'-rUrGrG(rN)-3'.
17. The method according to any of the preceding clauses, wherein the second splinted oligonucleotide is selected from RNA, DNA or combinations thereof, preferably RNA:DNA
18. The method according to any of the preceding clauses, wherein the nucleotides of the second splinted oligonucleotide are both RNA and DNA, being at least 1 terminal DNA base starting from its 3' end, preferably 2 or 3 terminal DNA nucleotides, preferably 10 or even up to 15 DNA nucleotides starting from the 3' end of said oligonucleotide.
19. The method according to any of the preceding clauses, wherein the second RNA:DNA splinted oligonucleotide has 3nt DNA bases starting from the 3' end
20. The method according to the preceding clauses, wherein the terminal DNA nucleotide(s) is selected from the group consisting of A, T, C, G and combinations thereof
21. The method according to any of the preceding clauses, wherein the second adapter DNA oligonucleotide hybridization region of the second splinted oligonucleotide is any sequence of at least 10 nucleotides, preferably 20 nucleotides as long as it is complementary to the second adapter DNA oligonucleotide hybridization region of the second splinted oligonucleotide, preferably a poly-A tail of at least 10 A nucleotides.
22. The method according to any of the preceding clauses, wherein the size of the first splinted oligonucleotide is between 15 and 40 nucleotides, preferably between 20 and 30 nucleotides.
23. The method according to any of the preceding clauses, wherein when the first and second splinted oligonucleotides are RNA nucleotides steps a) and b) can be performed simultaneously, and the agent with ligating activity is a commercial T4RNA ligase 2, or SEQ ID No 1, or SEQ ID No 2 or a variant having at least 80%, preferably at least 85%, more preferably at least 90 % and even more preferably 91 % or 92 % or 93 % or 94 % or 95 % or 96 % or 97 % or 98 % or even up to 99 % identity with respect to SEQ ID No 1 or SEQ ID No 2.
24. The method according to any of the preceding clauses 1 to 22, wherein when the first splinted oligonucleotide is RNA and the second splinted oligonucleotide is RNA:DNA step b) needs to be performed after step a) and the agent with ligating activity is T4RNA ligase 2 or SEQ ID No 1, or SEQ ID No 2 for step a) and any commercial DNA ligase for step b).
25. The method according to any of the preceding clauses, wherein the second splinted oligonucleotide hybridization region of the second adapter DNA oligonucleotide is a complementary sequence to the second adapter DNA oligonucleotide hybridization region of the second splinted oligonucleotide.
26. The method according to any of the preceding clauses, wherein the first splinted oligonucleotide is between 15 and 40 nucleotides, preferably between 20 and 30 nucleotides.
27. The method according to any of the preceding clauses, wherein the second splinted oligonucleotide is between 25 and 50 nucleotides, preferably between 28 and 35 nucleotides.
28. The method according to any of the preceding clauses, wherein the oligonucleotides are selected from one or more of the group consisting of: SEQ ID No 3, SEQ ID No 4, SEQ ID No 5, SEQ ID No 6, and SEQ ID No 7 or a variant having at least 80%, preferably at least 85%, more preferably at least 90 % and even more preferably 91 % or 92 % or 93 % or 94 % or 95 % or 96 % or 97 % or 98 % or even up to 99 % identity with respect to SEQ ID No 3, SEQ ID No 4, SEQ ID No 5, SEQ ID No 6, and/or SEQ ID No 7.
29. The method according to any of the preceding clauses 2 to 28, wherein the parameters of the software configured to analyze the sequencing results of the nanopore direct sequencing are adjusted for up to 1 second definition for adapter and a maximum of 2 seconds for the strand.
30. The method according to any of the preceding clauses 2 to 29, wherein the parameters for the analysis of the nanopore direct sequencing results use the BWA configuration, and the parameters are selected from the group consisting of:
   i) bwa mem -W13 -k6 -xont2d,
   ii) bwa mem -W13 -k6 -xont2d -T20,
   iii) bwamem -W13 -k6 -xont2d -T10,
   iv) bwamem -W9 -k5 -xont2d -T10 and
   v) bwasw -z10 -a2 -b1 -q2 -r1.
   Preferably bwa mem -W13 -k6 -xont2d -T20
31. The method according to any of the preceding clauses 1 to 27, wherein the hybridization region of both first and second DNA adapter oligonucleotide is a random nucleotide sequence between 15 and 45 nucleotides, unique for each pair of first and second DNA adapter oligonucleotide.
32. The method according to the preceding clause wherein more than one RNA sample containing tRNA are processed simultaneously through step d).
33. The method according to any of the preceding clauses, wherein the hybridization region of the first DNA adapter oligonucleotide is SEQ ID No 8, and the hybridization region of the second DNA adapter oligonucleotide is SEQ ID No 9
34. The method according to any of the preceding clauses 31 to 33, wherein the performing algorithm for the analysis of the nanopore direct sequencing results is minimap2 with adjusted parameters: - xmap-ont -k6 -w3 -n1 -m10-s13-A1 -B1 -O1-E1
35. A kit for performing the method described in any of the clauses 1 to 34.
36. The Kit according to the preceding clause, comprising:
   - a first splinted oligonucleotide, comprising a second splinted oligonucleotide hybridization region and a tRNA hybridization region, said tRNA hybridization region being located on its 3' end,
   - a second splinted oligonucleotide, comprising a first splinted oligonucleotide hybridization region, a second adapter DNA oligonucleotide hybridization region,
   - a first adapter DNA oligonucleotide, comprising a second DNA adapter oligonucleotide hybridization region, and
   - a second adapter DNA oligonucleotide, comprising a first DNA adapter oligonucleotide hybridization region and a second splinted oligonucleotide hybridization region, being said second splinted oligonucleotide hybridization region complementary to the second adapter DNA oligonucleotide hybridization region of the second splinted oligonucleotide,
37. The Kit according to the preceding clauses 35 to 36, wherein the first splinted oligonucleotide is an RNA oligonucleotide.
38. The Kit according to any of the preceding clauses 35 to 37, wherein the first RNA splinted oligonucleotide is SEQ ID No 3 and the second splinted oligonucleotide is SEQ ID No 4 or SEQ ID No 5
   or a variant having at least 80%, preferably at least 85%, more preferably at least 90 % and even more preferably 91 % or 92 % or 93 % or 94 % or 95 % or 96 % or 97 % or 98 % or even up to 99 % identity with respect to SEQ ID No 3, SEQ ID No 4, SEQ ID No 5.
39. The Kit according to any of the preceding clauses 35 to 37, wherein the second splinted oligonucleotide is an RNA:DNA oligonucleotide with at least 1 terminal DNA base on its 3' end.
40. The Kit according to any of the preceding clauses 35 to 39, comprising a T4 RNA ligase 2 with an oligonucleotide sequence of SEQ ID No 1 or SEQ ID No 2 or a variant having at least 80%, preferably at least 85%, more preferably at least 90 % and even more preferably 91 % or 92 % or 93 % or 94 % or 95 % or 96 % or 97 % or 98 % or even up to 99 % identity with respect to SEQ ID No 1 or SEQ ID No 2.

### Brief description of the figures

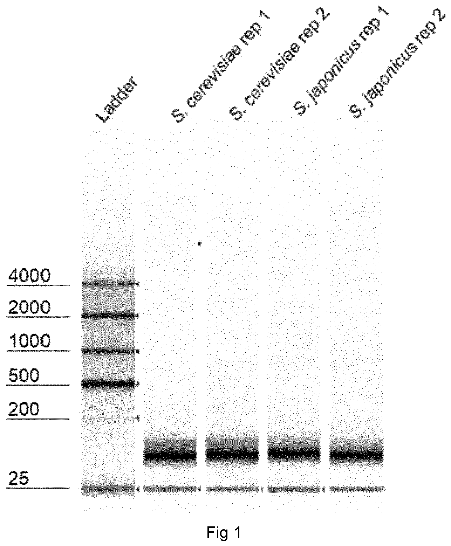

**Figure 1** **Tapestation gel of small RNA extracted from yeast strains.**
**Figure 2****. Comparison of the strategies tested to sequence tRNA molecules using nanopore DRS.** (A) Schematic overview of the 3 distinct library preparations, Strategy A, Strategy B and the method of the present invention, tested to sequence tRNA molecules

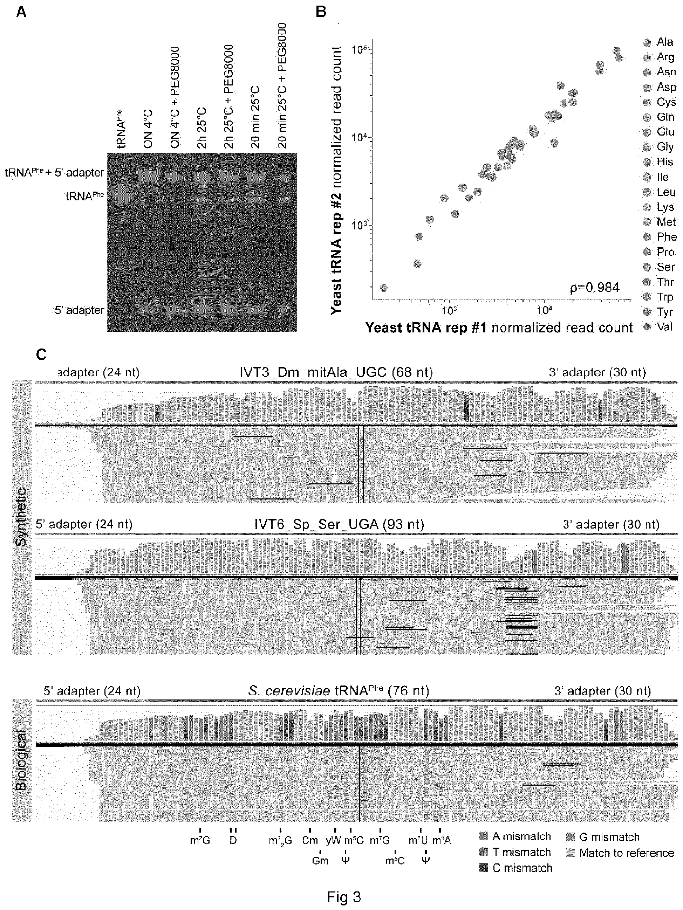

**Figure 3****. The method of the invention (Nano-tRNA seq) can efficiently sequence both in vitro transcribed and native tRNA populations** (A) TBE-UREA gel showing the effect of reaction duration and the addition of 20% PEG8000 on ligation efficiency (ON = overnight). (B) IGV snapshots of NanotRNAseq mapped reads from synthetic in vitro transcribed tRNAs (upper panels) or biological tRNAs (lower panel). Positions with a mismatch frequency greater than 0.2 are colored, whereas those showing mismatch frequencies lower than 0.2 are shown in gray. (C) Scatterplot of tRNA abundances showing the replicability of Nano-tRNAseq when WT S. *cerevisiae* tRNA biological replicates are sequenced. The correlation strength is indicated by Spearman's correlation coefficient (ρ).

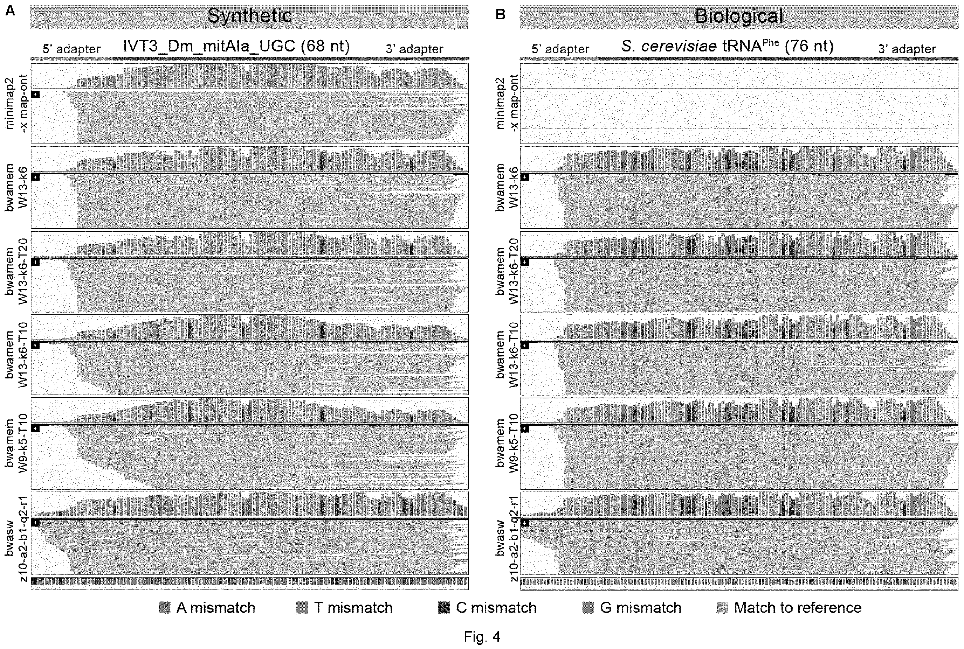

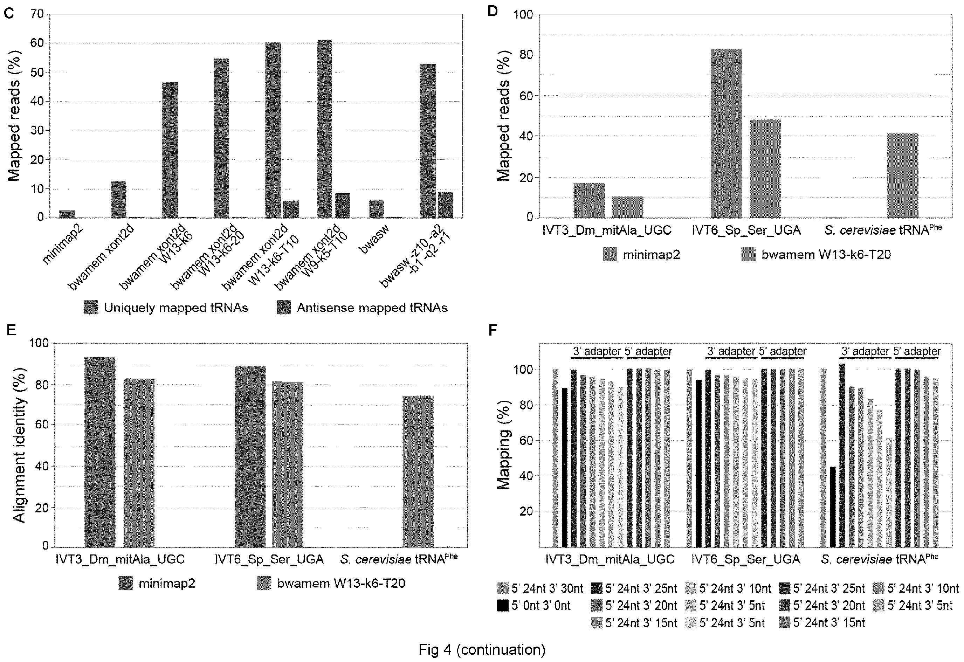

**Figure 4****. Choice of mapping software and parameters drastically affects the number of mapped tRNA reads.** (A,B) IGV snapshots of reads mapped to in vitro transcribed D. melanogaster mitochondrial tRNA^{Ala(UGC)} (A) and S. *cerevisiae* tRNA^{Phe} (B), mapped using different mapping algorithms (minimap2, bwa-mem, bwa-sw) and parameters. The 5' and 3' RNA adapter regions that were ligated to the ends of the tRNA molecule were included in the references for mapping, and are represented by an orange and red bar, respectively. Positions with a mismatch frequency greater than 0.2 are colored, whereas those showing mismatch frequencies lower than 0.2 are shown in gray. (C) Barplot depicting the effect of algorithm and parameter choice on the relative proportion of uniquely mapped reads (green) and mismapped reads (purple, reads mapping to antisense strands was used as a proxy to assess mismapping) (Table 5). (D) The proportion of mapped reads and (E) alignment identity for each individual template from barplot in (C), using either minimap2 or bwa-mem (W13-k6-T20). We should note that minimap2 alignment identity in S. cerevisiae tRNA^{Phe} was not computed because no reads were mapped to this tRNA using minimap2 with -xmap-ont parameters (Table 6). (F) Barplot showing the effect of trimming the length of the 5' RNA adapter (blues) and 3' RNA adapter (reds) on tRNA read mappability (Table 7). The conditions used by Nano-tRNAseq are shown in gray, whereas the effect of not using RNA adapters is shown in black.

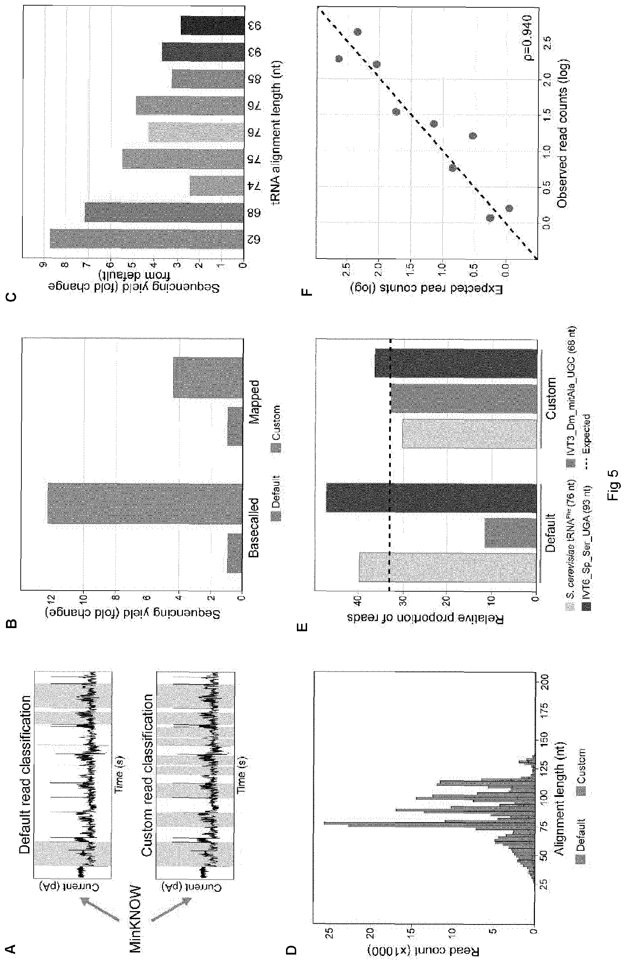

**Figure 5****. Adjustment of MinKNOW parameters increases the number of sequenced and mapped tRNA reads.** (A) Diagram showing the conceptual difference between default and custom MinKNOW read classification. (B) Barplot of sequencing yield in terms of basecalled and uniquely mapped reads obtained with default custom configuration (Table 8). (C) Barplot of relative fold change of uniquely mapped reads with respect to tRNA length (Table 9). (D) Histogram of read count and alignment length of in vitro transcribed (IVT) tRNAs reads captured with default and custom configuration. (E) Barplot of the relative proportion of IVT tRNA molecules *D. melanogaster* mitochondrial tRNA^{Ala(UCG)} and S. *pneumoniae* tRNA^{Ser(UGA)}, and native S. *cerevisiae* tRNA^{Phe} reads recovered with default and custom settings (Table 10), where the dotted line indicates the expected proportion. (F) Expected versus observed log read counts of 9 IVT tRNA molecules captured using the custom MinKNOW configuration (Table 11). Spearman correlation (ρ) is shown.

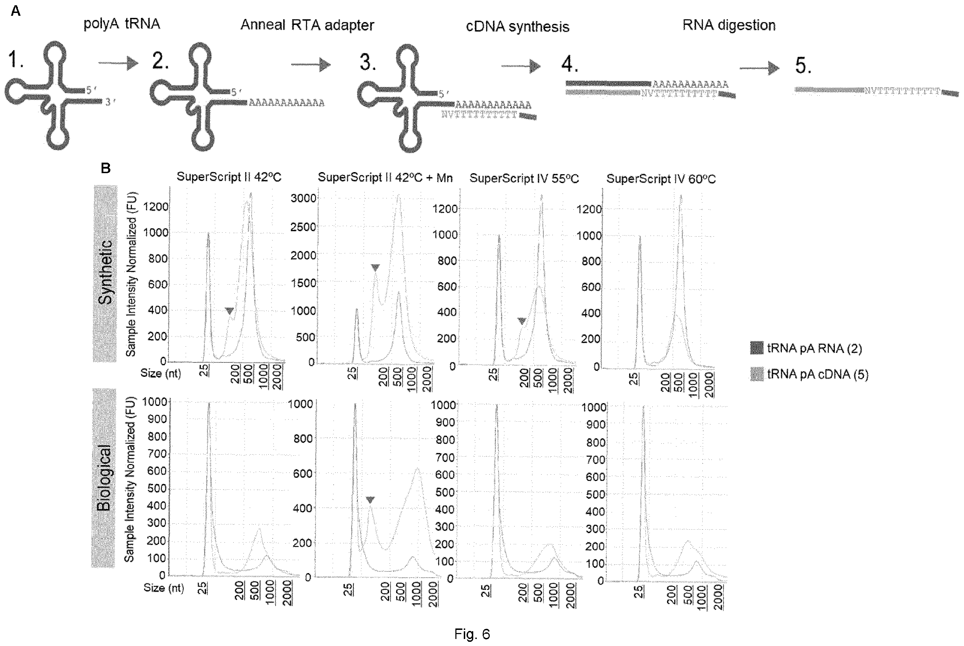

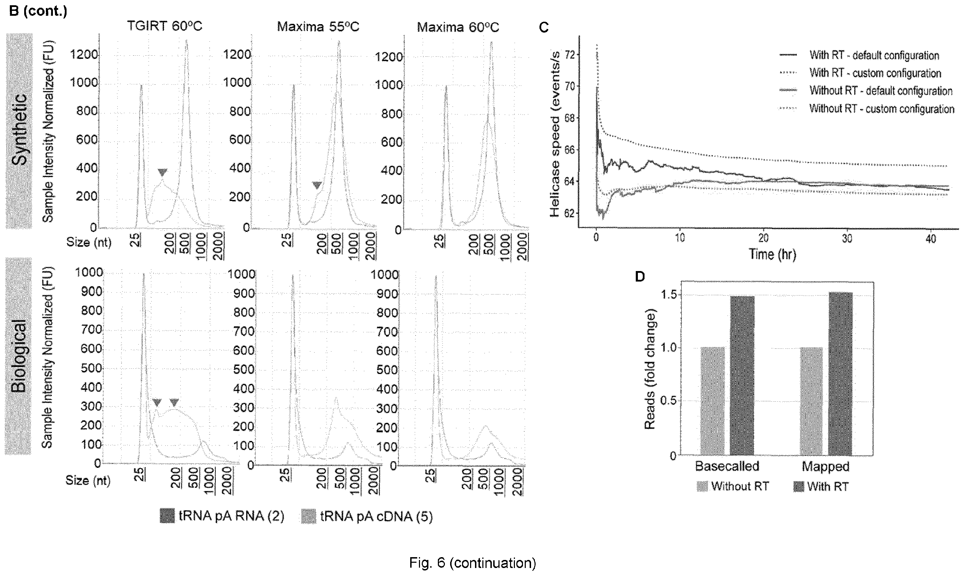

**Figure 6****. Comparison of the activity of diverse reverse transcriptase enzymes for tRNA linearization.** (A) Strategy used to test the reverse transcription activity of different enzymes. Starting from either in vitro transcribed (IVT) or native tRNA (1), the tRNA was polyadenylated (2) and annealed with an oligodT adapter (3), which was used to initiate the cDNA synthesis using different RT enzymes and conditions. The RNA strand of the linearized product (4) was digested, leaving the cDNA strand (5), which was checked via TapeStation. (B) TapeStation profiles depicting the original polyA (pA) tRNA product (blue) and the cDNA product (orange) that is produced by reverse transcription of the template using diverse reverse transcriptases and incubation conditions. Truncated cDNA products are shown with a gray triangle. The 25 nt peak that is present in all samples corresponds to the loading size marker. The upper panel is IVT tRNA, and the lower panel is commercial S. cerevisiae tRNAPhe. (C) Helicase speed (events/s roughly corresponds to nt/s sequenced) over time of wild type (WT) S. *cerevisiae* total tRNA sequenced with or without reverse transcription (RT) and classified using the default or custom MinKNOW configuration. (D) Barplot showing the fold change of basecalled and uniquely mapped reads when WT S. cerevisiae total tRNA is linearized with reverse transcription, compared to without reverse transcription.

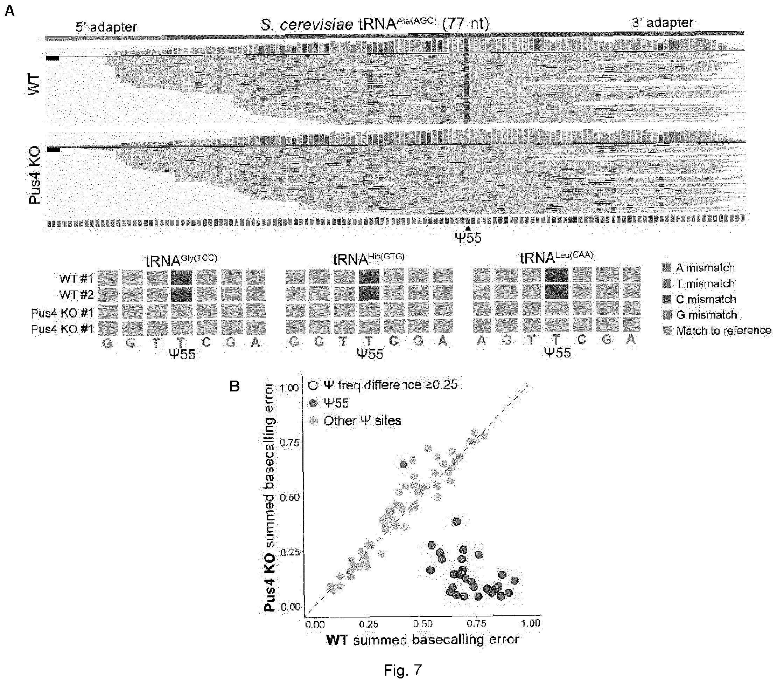

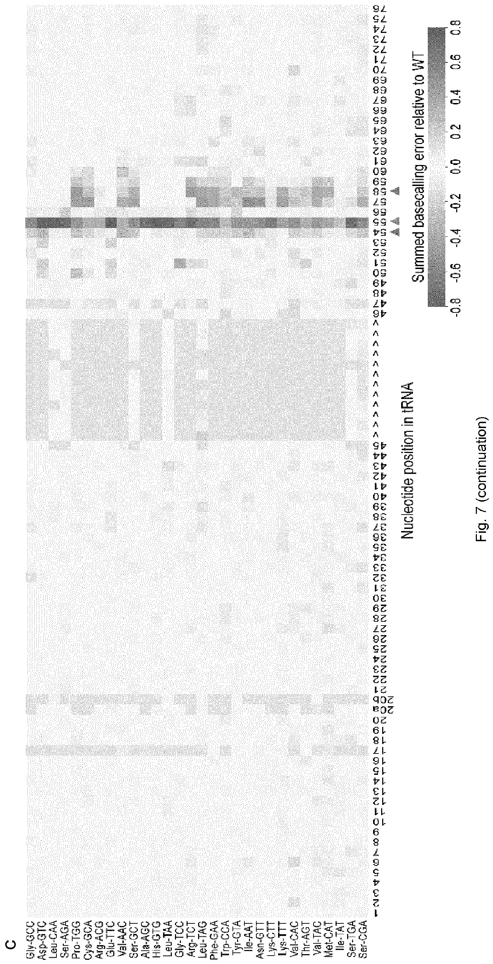

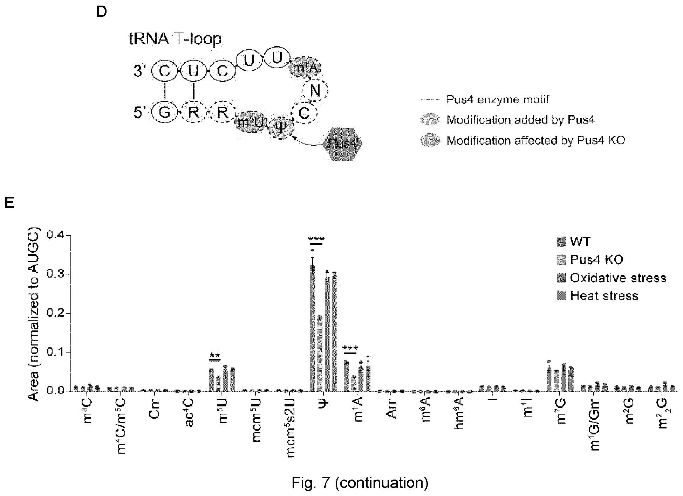

**Figure 7****. Nano-tRNAseq can quantify tRNA abundance and RNA modifications as well as capture modification interdepencies** (A) IGV tracks of tRNA^{Ala(AGC)} from WT and Pus4 KO S. *cerevisiae* strains. Positions with a mismatch frequency greater than 0.2 are colored, whereas those showing mismatch frequencies lower than 0.2 are shown in gray. Below are zoomed IGV snapshots of the Ψ55 region of select tRNAs, where the upper lanes correspond to WT biological replicates, and the lower panels are Pus4 KO biological replicates. (B) Comparison of mismatch frequencies for known Ψ sites in S. cerevisiae WT vs Pus4 KO tRNA molecules. Each data point represents a known tRNA Ψ site, red indicates Ψ55 sites, and the black outline indicates sites that have a summed basecalling error of ≥ 0.25 compared to WT, which serves as a proxy for Ψ modification frequency. (C) Heatmap of summed basecalling error of Pus4 KO relative to WT, for each nucleotide (x-axis), and for each tRNA isoacceptor (y-axis, ordered from most to least abundant in descending order).. Nucleotides with a higher summed basecalling error frequency in the KO strain, relative to WT, are shown in red tones, and those with a lower summed basecalling error frequency in the KO are shown in blue tones, as seen with Pus4 target Ψ55 (green arrow head), as well as in positions m⁵U54 and m¹A58 (pink arrow heads), indicating that these RNA modifications are down-regulated in Pus4 KO strains, relative to WT. (D) Schematic of the T-loop of tRNAs which is targeted by the Pus4 enzyme. Nucleotides with a dotted outline represent the Pus4 binding motif (RRUUCNA), Ψ55, which is added by Pus4, is highlighted in green. RNA modifications m5U54 and m¹A58, which are indirectly affected by Pus4 pseudouridylation of position, are highlighted in pink. (E) LC-MS/MS validation of RNA modification levels in tRNAs from S. *cerevisiae* cultured in standard conditions (WT), exposed to oxidative or heat stress, as well as Pus4 KO . Bars represent mean ± SEM for n=3 biological replicates per condition. P values were determined using a one-way analysis of variance (ANOVA) with Tukey correction for multiple comparisons, and significance was compared to WT. *P < 0.05, **P < 0.01, and ***P < 0.001.

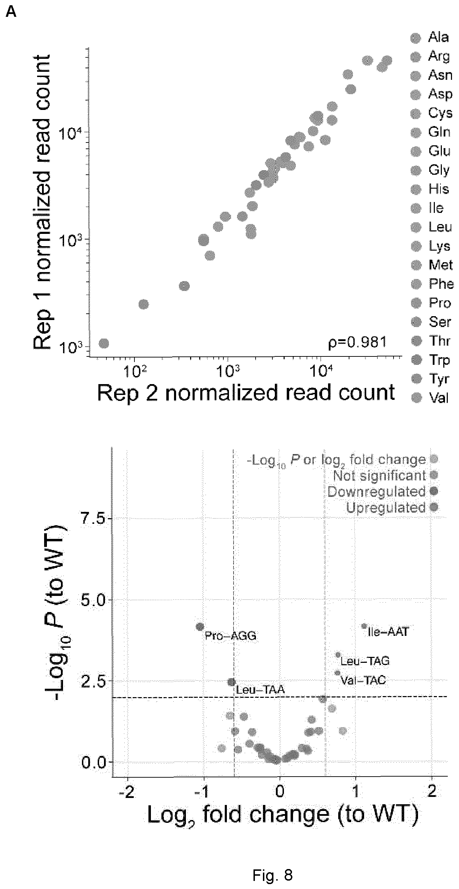

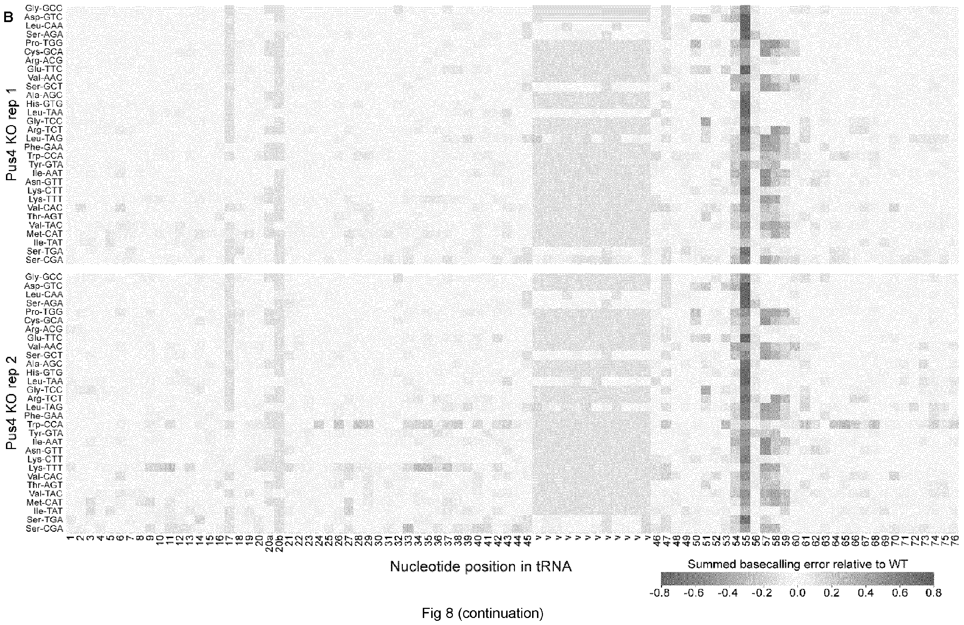

**Figure 8****. Nano-tRNAseq tRNA abundance and modification quantification is highly replicable.** (A) Scatterplots showing tRNA abundances of S. cerevisiae Pus4 knockout (KO) across biological replicates. See also Table 14. Each point represents a tRNA alloaccepter and are colored based on alloaccepter type. Differential expression volcano plot of pus4KO versus WT. Differentially expressed tRNAs were defined as having an adjusted -log10 P-value of <0.01 and an absolute log2 fold change greater than 0.6. (B) Heatmap of summed basecalling error frequency of Pus4 KO biological replicate 1 (as in Figure 7C) and replicate 2.. Nucleotides with a higher summed basecalling error frequency relative to WT are in red tones, and those with a lower summed basecalling error frequency in blue tones.

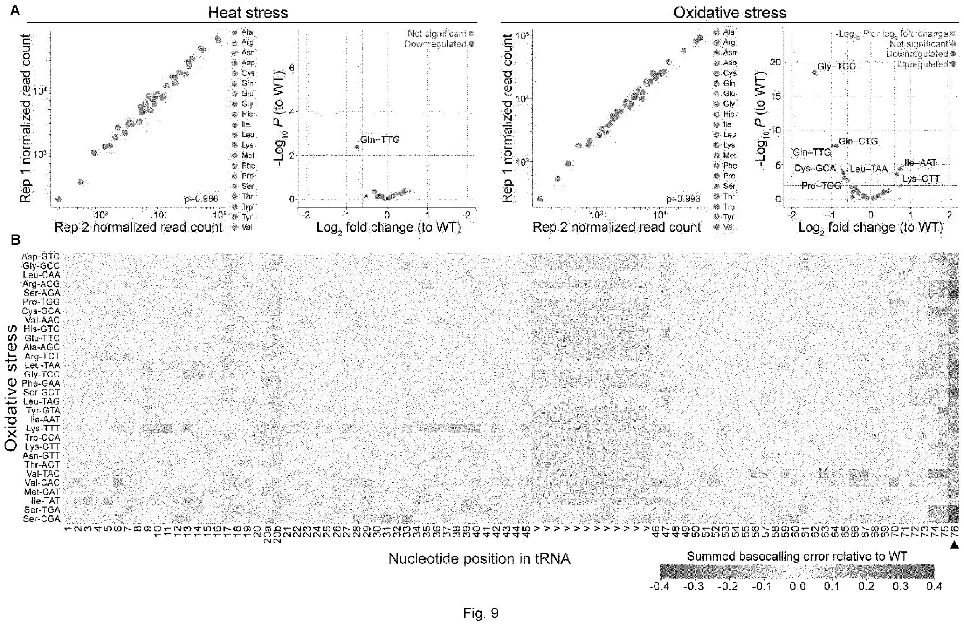

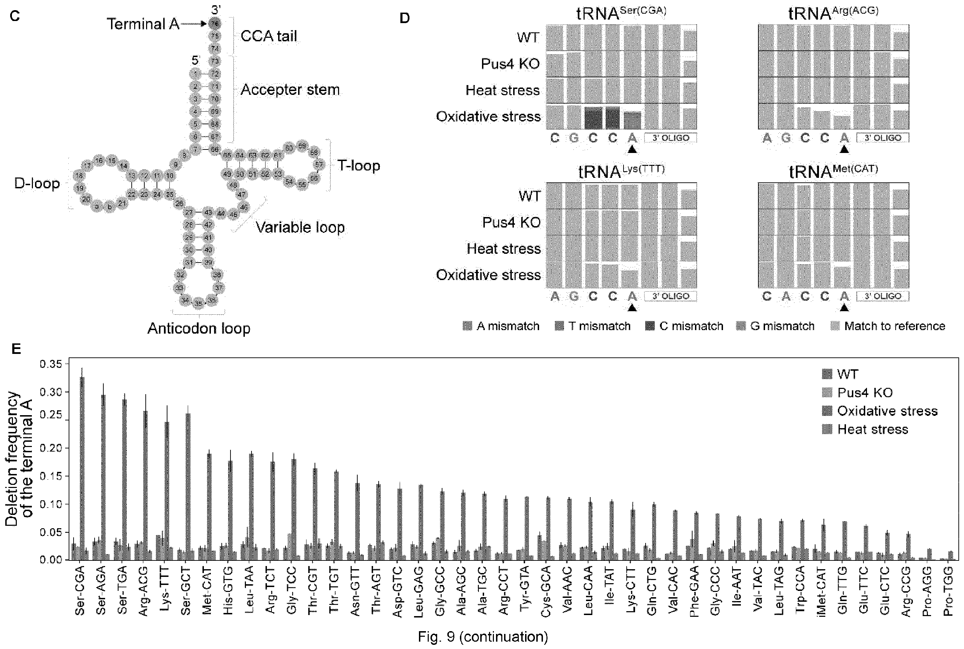

**Figure 9****. Characterization of tRNA abundance and modification dynamics upon exposure to stress reveals that the CCA tail is deadenylated in oxidative stress.** (A) Scatterplots of tRNA abundances of S. cerevisiae heat stress (45°C for 1 hour) and oxidative stress (2 mM H202 for 1 hour) biological replicates. Each point represents a tRNA alloaccepter and are colored based on alloaccepter type. The correlation strength is indicated by Spearman's correlation coefficient (ρ). See also Table 14. Differential expression volcano plots of heat stress or oxidative stress versus WT. See also Table 15-16. Differentially expressed tRNAs were defined as having an adjusted -Iog10 P-value of <0.01 and an absolute log2 fold change greater than 0.6. (B) Heatmap of summed basecalling error of oxidative stress relative to WT, for each nucleotide (x-axis), for each tRNA (y-axis, ordered from most to least abundant in descending order).. Nucleotides with a lower summed basecalling error frequency relative to WT are in blue tones, and those with a higher summed basecalling error frequency in red tones, as seen with the terminal A at position 76 (black arrow head). (C) Schematic of a generic S. *cerevisiae* cytoplasmic tRNA in its usual secondary structure with the terminal A nucleotide of the CCA tail highlighted in red. (D) Zoomed snapshots of IGV tracks featuring the terminal A (black arrow head). (E) Barplot of the deletion frequency of the terminal A base for each S. cerevisiae tRNA isoacceptor, under oxidative stress (red), Pus4 knockout (KO)(orange) or heat stress (purple), or in WT conditions (blue).

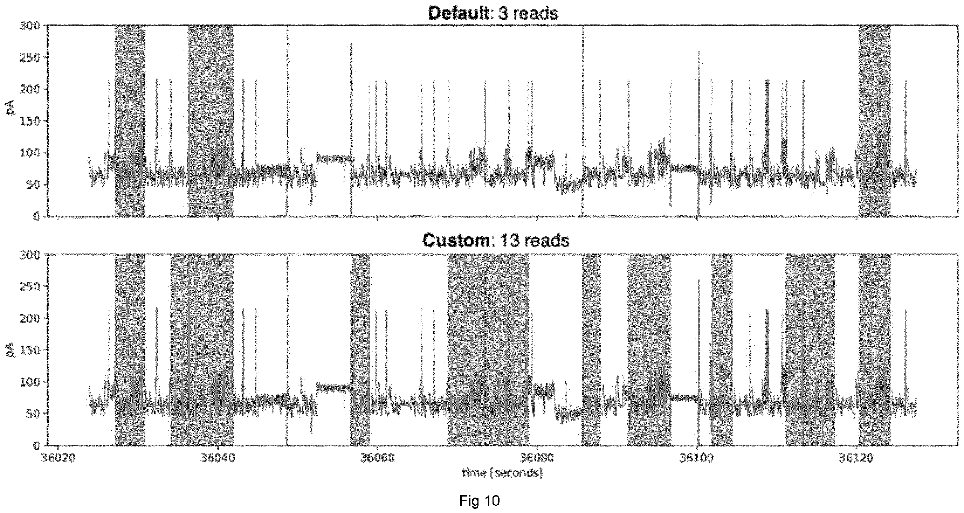

**Figure 10****. Nanopore signal from channel 300 is shown.** Comparison of reads captured using MinKNOW with `default' parameters (upper panel), and using 'custom' parameters, optimized to capture tRNA reads (lower panel). In each panel, the current intensity (units: pA y axis) captured at a specific nanopore channel (channel 300) during a given time duration (shown in seconds, x axis) is depicted.

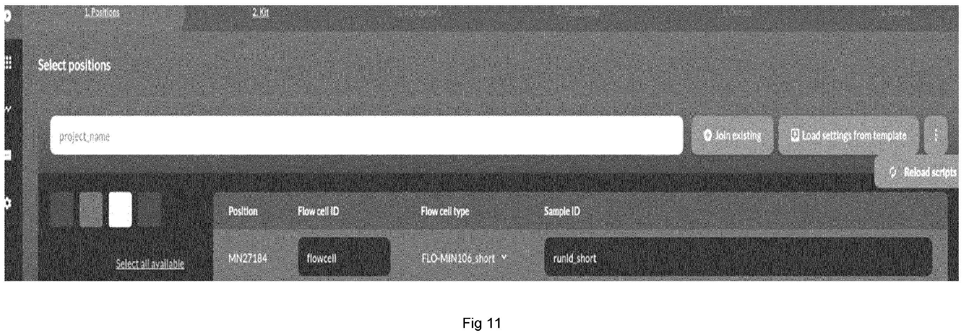

**Figure 11****.** Snapshot of MinKNOW illustrating the "reload scripts" button.

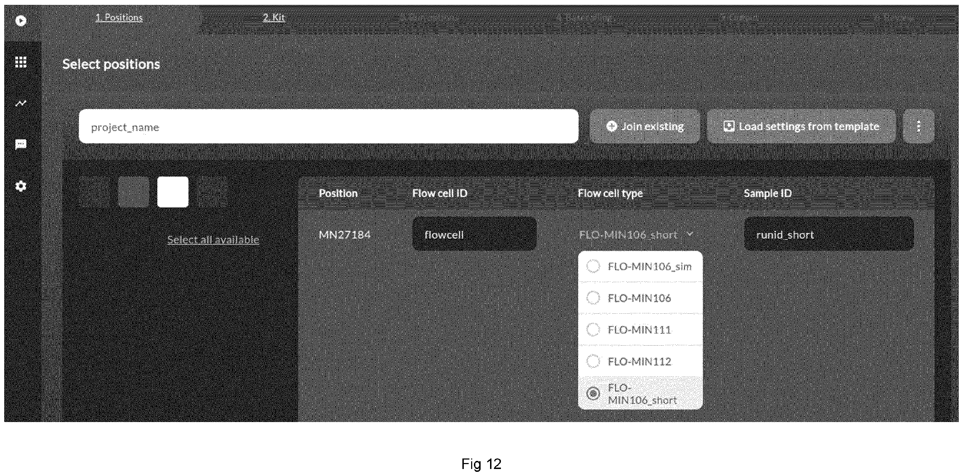

**Figure 12****.** Snapshot of MinKNOW once the alternative configuration (in this case, "FLO-MIN106-short") has been enabled.

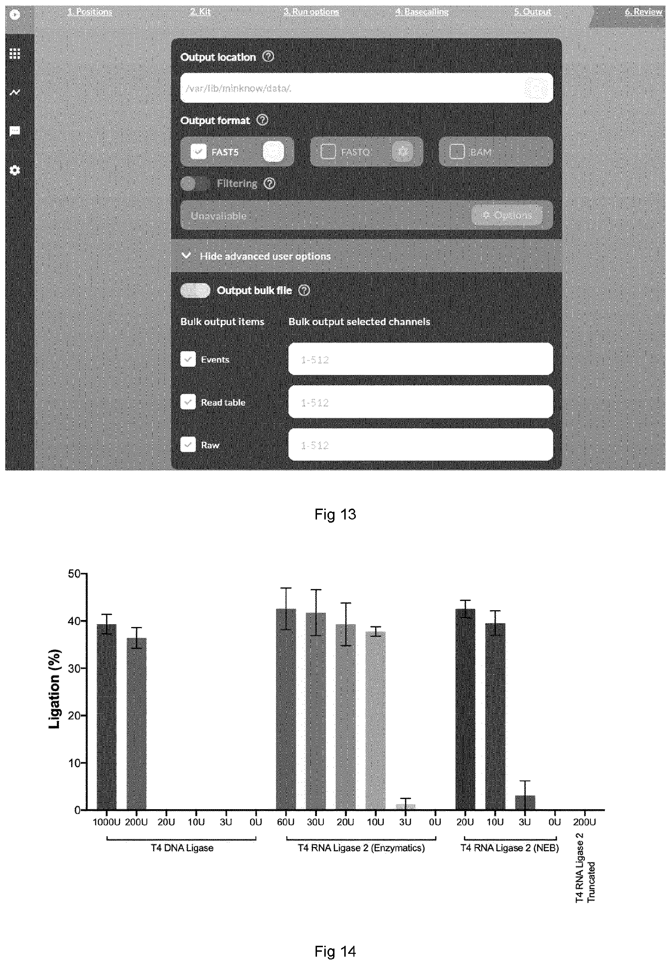

**Figure 13****.** Snapshot of MinKNOW illustrating how to save bulk files.
**Figure 14****.** Comparison of ligancy efficiency of 3 ligases at different concentrations

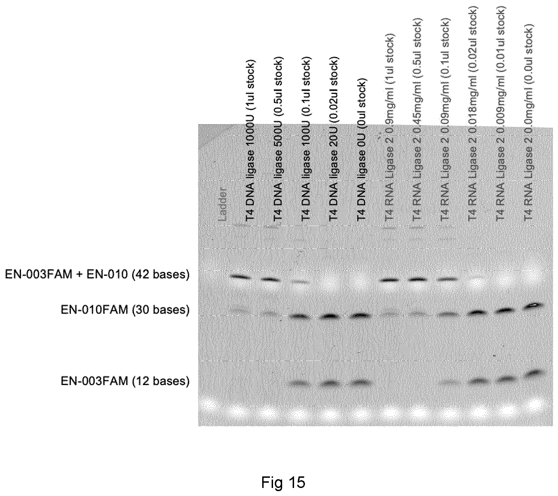

**Figure 15****.** Gel image of new vs old oligo and ligation times

### Examples

### Materials and Methods

### Preparation of In Vitro Transcription (IVT) transcribed tRNAs

A total of 9 unmodified in vitro transcribed tRNAs (see Table 2) were prepared as previously described [Saint-Léger A, Bello C, Dans PD, Torres AG, Novoa EM, Camacho N, et al. Saturation of recognition elements blocks evolution of new tRNA identities. Sci Adv. 2016;2: e1501860]. Briefly, each tRNA was assembled using six DNA oligonucleotides that were first annealed and then ligated between Hindlll and BamHl restriction sites of the plasmid pUC19. BstNI-linearized plasmids were used to perform the in vitro transcription with T7 RNA polymerase, according to standard and known protocols [Sampson JR, Uhlenbeck OC. Biochemical and physical characterization of an unmodified yeast phenylalanine transfer RNA transcribed in vitro. Proc Natl Acad Sci USA. 1988;85: 1033-1037.]. Transcripts were separated by 8 M urea/10% polyacrylamide gel electrophoresis. The tRNA was identified by UV shadowing, electroeluted and ethanol precipitated. The tRNA pellet was resuspended in RNAse-free water, and the integrity of the IVT tRNA products was checked using 8 M urea/15% polyacrylamide gel and stored until further use.

**Table 2 IVT tRNA and commercial tRNA references. Reference of each IVT tRNA and the commercial S. cerevisiae tRNA^{Phe} with the splinted oligonucleotides**

| **Name** | **Cloned gene** | **nt Length (adapters)** | **Reference** |
|---|---|---|---|
| IVT1_Pf_Lys_ UUU SEQ ID N° 16 | *Plasmodium falciparum* apicoplast tRNA Lys UUU | 76 (130) | CCTAAGAGCAAGAAGAAGCCTGGNGAATTACTAGCT TAATTGGTAGAGTACTCGACTTTTAATCGAAT GGTTCTGAGTTCAAATCTCAGGTAGTTCACCA GGCTTCTTCTTGCTCTTAGGAAAAAAAAAA |
| IVT2_Dm_Ser_ GCU SEQ ID N° 17 | *Drosophila melanogaster* tRNA Ser GCU | 85 (139) | CCTAAGAGCAAGAAGAAGCCTGGNGACGAGGUGGC CGAGAGGUUAAGGCGUUGGACUGCUAAUCCAAUGU GCUCUGCACGCGUGGGUUCGAAUCCCAUCCUCGUC GCCAGGCTTCTTCTTGCTCTTAGGAAAAAAAAAA |
| IVT3_Dm_mitA la_UGC SEQ ID N° 18 | *Drosophila melanogaster* mitochondrial tRNA Ala UGC | 68 (122) | CCTAAGAGCAAGAAGCCTGGNAGGGTTGTAGTT AAATATAACATTTGATTTGCATTCAAAAAGTATTGAATA TTCAATCTACCTTACCAGGCTTCTTCTTGCTCTTAGGA AAAAAAAAA |
| IVT4_Ec_Ser_ GCU SEQ ID N° 19 | *Escherichia coli* tRNA Ser GCU | 93 (147) | CCTAAGAGCAAGAAGCCTGGNGGTGAGGTGGC CGAGAGGCTGAAGGCGCTCCCCTGCTAAGGGAGTAT GCGGTCAAAAGCTGCATCCGGGGTTCGAATCCCCGC CTCACCGCCAGGCTTCTTCTTGCTCTTAGGAAAAAAA AAA |
| IVT5_Nc_Thr_ AGU SEQ ID N° 20 | *Neurospora crassa* tRNA Thr AGU | 75 (129) | CCTAAGAGCAAGAAGAAGCCTGGNGCCCGCATGGC TCAGTGGTAGAGCGCATCACTAGTAATGATGAGGTCG TCAGTTCGATTCTGGCTGTGGGCACCAGGCTTCTTCT TGCTCTTAGGAAAAAAAAAA |
| IVT6_Sp_Ser_ UGA SEQ ID N° 21 | *Streptococcus pneumoniae* tRNA Ser UGA | 93 (147) | CCTAAGAGCAAGAAGCCTGGNGGAGGATTACC CAAGTCCGGCTGAAGGGAACGGTCTTGAAAACCGTC AGGCGTGTAAAAGCGTGCGTGGGTTCGAATCCCACA TCCTCCTCCAGGCTTCTTCTTGCTCTTAGGAAAAAAA AAA |
| IVT7_Hs_Gly_ ACC SEQ ID N° 22 | *Homo sapiens* tRNA Gly ACC | 74 (128) | CCTAAGAGCAAGAAGAAGCCTGGNGCATTGGTGGTT CAGTGGTAGAATTCTCGCCTACCACGCGGGAGGCCC GGGTTCGATTCCCGGCCAATGCACCAGGCTTCTTCTT GCTCTTAGGAAAAAAAAAA |
| IVT8_Hs_mitS er_GCU SEQ ID N° 23 | *Homo sapiens* mitochondrial tRNA Ser GCU | 62 (116) | CCTAAGAGCAAGAAGCCTGGNGAGAAAGCTCA CAAGAACTGCTAACTCATGCCCCCATGTCTAACAACA TGGCTTTCTCACCAGGCTTCTTCTTGCTCTTAGGAAA AAAAAAA |
| IVT9_Hs_Gly_ CCC SEQ ID N° 24 | *Homo sapiens* tRNA Gly CCC | 74 (128) | CCTAAGAGCAAGAAGAAGCCTGGNGCGCCGCTGGT GTAGTGGTATCATGCAAGATTCCCATTCTTGCGACCC GGGTTCGATTCCCGGGCGGCGCACCAGGCTTCTTCT TGCTCTTAGGAAAAAAAAAA |
| Sc_Phe SEQ ID N° 25 | NA | 76 (130) | CCTAAGAGCAAGAAGAAGCCTGGNGCGGATTTAGCT CAGTTGGGAGAGCGCCAGACTGAAGATCTGGAGGTC CTGTGTTCGATCCACAGAATTCGCACCAGGCTTCTTC TTGCTCTTAGGAAAAAAAAAA |

| | | | |
|---|---|---|---|
| Nucleotides in bold correspond to first splinted RNA oligonucleotide and second splinted RNA:DNA oligonucleotide. | | | |

### Removal of 5' triphosphate of IVT tRNAs

The 5' triphosphate was converted to 5' monophosphate by incubating 1 µL of RppH enzyme (NEB, M0356S) per 100 ng of input IVT tRNAs, with 1X Thermopol Buffer (NEB, B9004S), in a total reaction volume of 30 µL, at 37°C for 2 h. The reaction was inactivated by adding 0.6 µL of 500 mM EDTA and incubating at 65°C for 5 min, followed by clean up using a Zymo RNA Clean and Concentrator-5 kit (Zymo, R1016), following the manufacturer's instructions to retain RNAs ≥17 nt. This step is required only for IVT RNAs, because they have three phosphates at their 5' end. In vivo tRNAs have only one phosphate, so this step is not needed for an RNA sample containing tRNA.

### Yeast strains and culturing

*Saccharomyces cerevisiae* parental strain (BY4741) and Pus4 knockout strain (BY4741 MATa pus4::KAN) were obtained from the Yeast Knockout Collection (Dharmacon) and grown under standard conditions overnight in 4 mL yeast extract peptone dextrose (YPD) medium (1% yeast extract, 2% Bacto Peptone and 2% dextrose) at 30°C. The following day, cultures were diluted to 0.0001 OD600 in 200 mL of YPD and grown overnight at 30°C with shaking (250 r.p.m.). When cultures reached mid-exponential growth phase (between OD600 0.5), the WT culture was divided into 3x 50 mL subcultures, which were then incubated for 1 h at 30°C (control), 45°C (heat stress) or in 2 mM H202 (oxidative stress). The Pus4 culture was divided into 1x 50 mL culture and incubated at 30°C. Following incubation, cultures were quickly transferred into a pre-chilled 50 mL Falcon tube and centrifuged at 3000 g for 5 min at 4°C, followed by two washes with water, then pellets were snap-frozen at -80°C. Replicates were performed on consecutive days.

### RNA extraction from yeast cultures

Snap-frozen yeast pellets were resuspended in 660 µL of TRIzol^{®} Reagent (Thermo Fisher Scientific, 15596018) with 340 µL of acid-washed and autoclaved 425-600 µm glass beads (Sigma-Aldrich, G8772). The cells were disrupted by vortexing on top speed for seven cycles of 15 s and chilling the samples on ice for 30 s between cycles. The samples were then incubated at room temperature for 5 min and 200 µL of chloroform was added. After briefly vortexing the suspension, the samples were incubated for 5 min at room temperature. The samples were then centrifuged at 14,000 g for 15 min at 4°C, and the upper aqueous phase was transferred to a new tube. To precipitate RNA, 1X volume of molecular-grade isopropanol and 1 µL of GlycoBlue^{™} coprecipitant (Thermo Fisher Scientific, AM9515) was added and mixed by inverting and incubated for 10 min at room temperature. The samples were centrifuged at 14,000 g for 15 min at 4°C and the pellet was then washed with ice cold 70% ethanol. The pellet was resuspended in nuclease-free water after air drying for 5 min on the benchtop and the RNA purity was measured using a NanoDrop 1000 spectrophotometer. The samples were treated with Turbo DNase (Thermo Fisher Scientific, no. AM2238) and subsequently cleaned up using a Zymo RNA Clean and Concentrator-5 kit (Zymo, R1016) following the manufacturer's instructions to retain RNAs ≤200 nt. Briefly, 1X volume of RNA Binding Buffer was combined with 1X volume of 100% ethanol. 2X volume of the RNA Binding Buffer and ethanol solution was added to the reaction, transferred to a Zymo-IC column and spun at ≥12,000 g at room temperature for 1 min. 1X volume of 100% ethanol was added to the flow-through, which contains the 17-200 nt fraction, and this was transferred to a new Zymo-IC column and spun at ≥12,000 g at room temperature for 1 min. 400 µL of RNA Prep Buffer was added to the column and spun at at ≥12,000 g at room temperature for 1 min, then 800 µL of RNA Wash Buffer was added and the column was spun at >12,000 g at room temperature for 2 mins, transferred to a fresh collection tube, and spun for 1 min. The RNA was eluted in nuclease free water.(Fig 1) RNA concentration was determined using Qubit Fluorometric Quantitation, RNA purity was measured with a NanoDrop 1000 spectrophotometer, and the RNA electropherogram was obtained using Agilent 4200 TapeStation RNA HS Screentape Assay.

### RNA extraction and size selection of small RNAs from Human cell lines

Snap-frozen pellets from the different cell lines were resuspended in 500 µL of TRIzol^{®}Reagent (Thermo Fisher Scientific, 15596018) and incubated at room temperature for 5 min and 100 µL of chloroform was added. After briefly vortexing the suspension, the samples were incubated for 3 min at room temperature. The samples were then centrifuged at 16,000 g for 15 min at 4°C, and the upper aqueous phase was transferred to a new tube. To precipitate RNA, 0.7X volume of molecular-grade isopropanol and 1 µL of GlycoBlue^{™} coprecipitant (Thermo Fisher Scientific, AM9515) was added and mixed by inverting and incubated for 15 min at room temperature. The samples were centrifuged at 12,000 g for 30 min at 4°C and the pellet was then washed with ice cold 70% ethanol. After a 5 min centrifugation at 7,500 g, the pellet was resuspended in nuclease-free water after air drying for 8 min on the benchtop and the RNA purity was measured using a NanoDrop 1000 spectrophotometer. The samples were treated with Turbo DNase (Thermo Fisher Scientific, no. AM2238) and subsequently cleaned up using a Qiagen RNeasy MinElute Cleanup Kit (Qiagen, no. 74204) following the manufacturer's instructions to retain RNAs ≤200 nt, but adding some steps to retain also RNAs >200 nt. Briefly, 350 µL of RLT Buffer was added to the sample, 250 µL of 100% ethanol and then, the samples were transferred to an RNeasy MinElute spin column and centrifuged at >8,000 g at room temperature for 40 s to retain RNAs > 200 nt. 450 µL of 100% ethanol was added to the flow-through, which contains the 17-200 nt fraction, transferred to a new RNeasy MinElute spin column and centrifuged at >8,000 g at room temperature for 40 s. 500 µL of RPE wash buffer was added to all the columns: the ones retaining RNAs >200 nt and the ones retaining RNAs ≤200 nt and spun at ≥10,000 g at room temperature for 40 s, then 500 µL of 80% ethanol was added and the columns were spun at >10,000 g at room temperature for 40 s first, and for 2 min after in order to dry the possible ethanol retains. The RNA was eluted in 14 µL nuclease free water. RNA concentration and purity were determined using NanoDrop 1000 spectrophotometer, and the RNA electropherogram was obtained using Agilent 4200 TapeStation RNA HS Screentape Assay.

### tRNA deacylation

In vivo-purified tRNAs may be aminoacylated, which can inhibit 3' ligation and polyA tailing reactions. Commercial S. *cerevisiae* tRNAPhe (Sigma Aldrich, R4018), commercial S. *cerevisiae* total tRNA (Sigma Aldrich, AM7119) and tRNAs purified from S. *cerevisiae* BY4741 WT and Pus4 knockout cultures were resuspended in 10 µL nuclease-free water and deacylated in 95 µL 100 mM Tris-HCI (pH 9.0) at 37°C for 30 min. Deacylated tRNAs were recovered using Zymo RNA Clean and Concentrator-5 kit (Zymo, R1016), following the manufacturer's instructions to retain RNAs ≥17 nt, confirmed using Agilent 4200 TapeStation RNA HS Screentape Assay.

### Nanopore direct tRNA sequencing library preparation (Nano-tRNAseq)

tRNA libraries were prepared using the SQK-RNA002 kit (Oxford Nanopore Technologies) with some protocol alterations as described here. All oligonucleotides used in this study were obtained from *Integrated DNA Technologies* (IDT) (see Table 1 for sequences). The 5' RNA splint adapter first splinted RNA oligonucleotide, SEQ ID NO 3 was designed to be complementary to the 3' NCCA overhang of mature tRNAs, and the 3' splint RNA:DNA adapter: second splinted oligonucleotide SEQ ID NO 5 was designed to be complementary to the rest of the 5' RNA splint adapter (first splinted RNA oligonucleotide), with a short polyA segment for the ONT RTA adapter (second adapter DNA oligonucleotide) to anneal to (Figure 2). The 5' and 3' RNA splint adapters were prepared at a 1:1 molar ratio in a solution of 10 mM Tris-HCI (pH 7.5), 50 mM NaCl and 1 µL RNasin^{®} Ribonuclease Inhibitor (Promega,N251A), with a final concentration of 50 ng/µL, and heated to 75°C for 15 s and cooled to 25°C at a rate of 0.1°C/s to hybridize the adapters. DNA oligos with the same sequence as ONT RTA adapters were ordered from IDT and annealed in the same manner as the 5' and 3' splint adapters. Deacylated tRNAs were ligated to the pre-annealed 5' and 3' splint adapters at a molar ratio of 1.2:1 (assuming an average tRNA length of 90 nt). The ligation was carried out at room temperature for 2 h in a total reaction volume of 50 µL with 20% PEG 8000 (NEB, B10048), 1X T4 RNA Ligase 2 Buffer (NEB, B0239S), 4 µL 6 mg/mL recombinant E. *coli* T4 RNA 2 Ligase (made in-house, see below) and 1 µL RNasin^{®} Ribonuclease Inhibitor (Promega, N251A). A 2X volume of room temperature equilibrated Ampure RNAClean XP beads (Beckman-Coulter, A63987) was then added to the reaction and pipetting gently up and down, and incubated for 15 minutes at room temperature on a Hula mixer The beads were washed with freshly prepared 70% ethanol and left to air dry. The samples were eluted by resuspending the beads in nuclease-free water and incubating for 10 minutes at room temperature on a Hula mixer.

The RNA concentration was determined using RNA HS Qubit Fluorometric Quantification. 200 ng of 5' and 3' ligated tRNAs were ligated to the pre-annealed RTA adapters at a molar ratio of 1:2 (roughly 4.3 pmol tRNAs to 8.6 pmol of RTA adapter). The ligation was carried out at room temperature for 30 min in a total reaction volume of 15 µL with 1X Quick Ligation Reaction buffer (NEB, B6058S), 1.5 µL T4 DNA Ligase (NEB, M0202M, 2,000,000 units/mL) and 0.5 µL RNasin^{®} Ribonuclease Inhibitor (Promega, N251A).

After ligation, a reverse transcription master mix of 13 µL nuclease-free water, 2 µL 10 mM dNTPs, 8 µL 5X Maxima H Minus Reverse Transcriptase Buffer and 2 µL Maxima H Minus Reverse Transcriptase (Life Technologies, EP0751) was added directly to the reaction, mixed well by pipetting and incubated at 60°C for 1 h, 85°C for 5 min and then brought to 4°C. The linearized tRNAs were cleaned up using 2X Ampure RNAClean XP beads as described for the ligation reaction. RNA concentration was not measured in the proceeding steps as it is below the detectable range for the Qubit RNA HS Assay. Finally, the ONT RMX sequencing adapters were ligated at room temperature for 30 min in a total reaction volume of 40 µL with 1X Quick Ligation Reaction buffer (NEB, B6058S), 3 µL T4 DNA Ligase (NEB, M0202M, 2,000,000 units/mL) and 6 µL RMX adapters. A 2X volume of Ampure RNAClean XP beads was then added and mixed into the reaction by pipetting gently up and down, and incubated for 10 min at room temperature on a Hula mixer. The sample was washed twice with 150 µL WSB (Wash Buffer), in which the pellet was resuspended by flicking the tube. The sample was eluted in 20 µL ELB (Elution Buffer) and incubated for 10 minutes at room temperature on a Hula mixer. The final library was prepared by adding 17.5 µL of nuclease-free water and 37.5 µL of vortexed RRB, and kept on ice until loading. The MinION flow cell (FLO-MIN-106) in which Direct Nanopore RNA is to be performed was Quality Checked, primed and loaded as per the standard ONT SQK-RNA002 protocol.

### Alternative (failed) Nanopore direct tRNA sequencing library strategies

Alternative (failed) tRNA DRS libraries tested were prepared using the SQK-RNA002 kit (Oxford Nanopore Technologies) with some protocol alterations as described here for the following library preparation protocol strategies (Figure 2).

*Strategy A:* Deacylated tRNAs were polyadenylated using Escherichia coli poly(A) polymerase (NEB, M0276S) at 37°C for 30 min following manufacturer's instructions. The 5' RNA splint adapter, as used in Nano-tRNAseq and all library preparation strategies described, was ligated to poly(A)-tailed tRNAs at a molar ratio of 2:1. The reaction was carried out overnight at 4°C with 20% PEG 8000, 1X T4 RNA Ligase 2 Buffer, 4 µL 6 mg/mL recombinant E. *coli* T4 RNA 2 Ligase, 1 µL RNaseOUT^{™} (Invitrogen, 18080051), in a total reaction volume of 50 µL. A 1.8X volume of Ampure RNAClean XP beads was then added and mixed into the reaction by pipetting gently up and down, and incubated for 15 minutes at room temperature on a Hula mixer. The beads were washed with freshly-prepared 70% ethanol and left to air dry. To elute, the beads were resuspended in nuclease-free water and incubated for 10 minutes at room temperature on a Hula mixer. RNA concentration was determined using Qubit Fluorometric Quantification. The ligation of RTA and RMX adapters, final library preparation steps, and flowcell QC and loading is as described above.

*Strategy B:* The 5' splint RNA adapter; (first splinted RNA oligonucleotide SEQ ID No 3), and ONT RTA adapter oligo A (first adapter DNA oligonucleotide) were annealed in a molar ratio of 1:1 as described above. The annealed 5' splint RNA adapter and 3' splint DNA adapter were ligated to 5' monophosphate, deacylated tRNAs and cleaned up using the same protocol as in Strategy A. The ligation of RMX adapters, final library preparation steps, and flowcell QC and loading is as described in Nano-tRNAseq.

### Recombinant protein expression of E. coli T4 RNA Ligase 2

Unlike T4 DNA Ligase, commercial T4 RNA Ligase 2 is only available at low concentrations. To improve the efficiency of our library preparation ligations, we produced a highly concentrated recombinant *E. coli* T4 RNA Ligase 2 in-house. Specifically, the codon-optimized sequence of *E. coli* T4 RNA Ligase 2 SEQ ID No2 (T4RNL2) ORF DNA was synthetically ordered from IDT, and was cloned into the expression plasmid pETM14 in frame, with a coding sequence of a hexa-histidine tag followed by a 3C precision cleavage recognition sequence. The protein expression and purification were performed in the Protein Technologies Unit at the Center for Genomic Regulation, following previously described procedures [Bullard DR, Bowater RP. Direct comparison of nick-joining activity of the nucleic acid ligases from bacteriophage T4. Biochem J. 2006;398: 135-144.] (see Figures 14 and 15 for ligation tests). For long-term storage at -80°C, glycerol was added to a final concentration of 10%.

### Conditions for the production of T4 RNA ligase 2

Protein expression conditions of SEQ_ ID No 1 or SEQ ID No 2 where as follows: For 1L BL21 (DE3) in 2TY growth medium; growth at a temperature range from 32°C to 40°C preferably about 37°C until OD600 0,4; and protein expression was induced with 0,4mM IPTG; for about 3h 37°C. The maximum amount of protein obtained per 1L is about 7mg

Suggested protein purification info, although any other known protocol from protein purification can be used
Cell lysate of yeast expressing either SEQ ID No 1 or SEQ ID No 2 as mentioned above
- Add 50mL of a buffer containing about 50mM TRIS pH 7.4, about 200 mM NaCl, about 50mM Imidazol and about 10% Glycerol, plus protease inhibitors (complete EDTA free); Triton X100 and PMSF
- Resuspend pellet; cell lysate with french press, and clarify by ultracentrifugation Purification
- Affinity column: HiTrap 5mL Ni²⁺ column
- SEC Hiload 16/60 200

The ready to use ligase T4 RNA ligase SEQ_ ID NO 1 or SEQ ID NO has a concentration from: about 2 mg/ml to 11 mg/ml preferably about 9mg/mL in 10 mM Tris-HCI, 50 mM KCI, 35 mM (NH4)2SO4, 0.1 mM DTT, 0.1 mM EDTA, 50% Glycerol pH 7.5 at 25°C. However, any other solution commonly known in the field which does not interfere with the ligase activity can be used.

### Gel purification of tRNAs and LC-MS/MS

Gel purified tRNAs were only used for LC-MS/MS. 5 µg of the 17-200 nt fraction of each sample, and commercial S. *cerevisiae* tRNA^{Phe} and total tRNA which served as markers, were prepared in 2X RNA loading dye (NEB, B0363A) and heat denatured at 94°C for 5 min. Running samples were loaded into 15% 7M TBE-Urea gels (Life Technologies, EC6885BOX) with a lane left free between each sample to avoid cross-contamination and run in 1X TBE at 100V until bromophenol blue marker is at three quarters of the way down the gel. The gel was poststained in the dark in 1X TBE with 1X SYBR^{™} Gold (Invitrogen, S11494) for 5 min. Gels were transferred to copier transparency film (Niceday, 607510) and using UV underlighting, the gel region corresponding to tRNAs (around 70 to 110 nt) was excised using a sterile scalpel and transferred into a Zymo-Spin^{™} IV Column from the ZR small-RNA PAGE Recovery kit (Zymo, R1070). tRNAs were extracted from the gel as per manufacturer's instructions, and the extracted tRNA profiles were confirmed using Agilent 4200 TapeStation RNA HS Screentape Assay. 500 ng of gel purified tRNAs were digested at 37°C for 1 h using Nucleoside Digestion Mix (NEB, M0649), following manufacturer's instructions. The nucleoside digestion solution was then desalted using HyperSep^{™} SpinTip Column (ThermoFisher, 60109-404); first the column was washed with 40 µL of 60% acetonitrile by centrifuging at 100 g for 10 min, then washed with 40 µL of 0.1% formic acid by centrifuging at 100 g for 5 min. The digested sample was combined with 30 µL of formic acid, added to the column and collected in a fresh collection tube by centrifuging at 100 g for 10 min. The flow-through was re-applied to the column and centrifuged at 100 g for 10 min. The sample, now bound to the column, was washed with 40 µL of 0.1% formic acid by centrifuging at 100 g for 5 min. The collection tube was replaced to prepare for elution and 40 µL of 60% acetonitrile was added to the column and the sample eluted by centrifuging at 100 g for 5 min. LC-MS/MS of S. *cerevisiae* tRNA modifications was conducted by the CRG/UPF Proteomics Facility; briefly 125ng of each digested and desalted sample was analyzed by LC-MS.MS using a 40 min gradient on a Orbitrap XL. As a quality control, ribonucleoside standards were run between samples to prevent carry-over and to assess the instrument performance. Heat stress replicate 2 had an altered chromatographic profile with significantly less Ψ than all other samples and was therefore discarded from the analysis.

### tRNA reverse transcription optimization

IVT tRNAs and commercial *S. cerevisiae* tRNA^{Phe} were polyA tailed as described in Strategy A. Importantly, only synthetic RNAs have a 5' monophosphate. For SuperScript II reverse transcription tests, 100 ng of poly A tailed RNA, 1 µL of 100 µM 3' RT test adapter (SEQ ID No 26 /5Phos/ACT TGC CTG TCG CTC TAT CTT CTT TTT TTT TTT TTT TTT TTTVN), "N" can be any nucleotide selected from A, C, T, G, while "V" can be any nucleotide selected from A, C and G. 1 µL of 10 mM dNTP (Promega, M750B) were combined in a total reaction volume of 12 µL, incubated at 65°C for 5 mins then chilled on ice. Then, 4 µL of either 5X first strand buffer (ThermoFisher, 18064014) or 5X first strand (FS) buffer supplemented with 65 mM MnCl2, 1 µL of 0.1 M DTT, 1 µL of RNaseOUT^{™}, 1 µL of Superscript^{™} II reverse transcriptase (ThermoFisher, 18064014) were added, and the reaction was incubated at 42°C for 1 hour, inactivated by heating at 70°C for 15 minutes, followed by RNAse digestion. For SuperScript IV reverse transcription tests, 100 ng of poly A tailed RNA, 1 µL of 100 µM 3' RT test adapter, 1 µL of 10 mM dNTP were combined in a total reaction volume of 12 µL, incubated at 65°C for 5 mins then chilled on ice. Then, 4 µL of 5X Superscript^{™} IV RT buffer (ThermoFisher, 18090010), 1 µL of 0.1 M DTT, 1 µL of RNaseOUT^{™}, 1 µL of Superscript^{™} IV reverse transcriptase (ThermoFisher, 18090010) were added, and the reaction was incubated at 55°C or 60°C for 1 hour, inactivated by heating at 85°C for 5 minutes, followed by RNAse digestion. For TGIRT reverse transcription tests, 100 ng of poly A tailed RNA, 1 µL of 100 µM 3' RT test adapter, 4 µL of 5X TGIRT RT buffer, 1 µL of 0.1 M DTT, 1 µL of TGIRT^{™}-III (InGex, TGIRT50), 1 µL of RNaseOUT^{™} were combined in a total reaction volume of 19 µL and incubated at room temperature for 30 mins. Then, 1 µL of 10 mM dNTPs was added, and the reaction was incubated 60°C for 1 hour, inactivated by heating at 75°C for 15 minutes, followed by RNAse digestion. For Maxima reverse transcription tests, 100 ng of poly A tailed RNA, 1 µL of 100 µM 3' RT test adapter, 1 µL of 10 mM dNTP were combined in a total reaction volume of 12 µL, incubated at 65°C for 5 mins then chilled on ice. Then, 4 µL of 5X Maxima RT buffer, 1 µL of RNaseOUT^{™}, 1 µL of Maxima H Minus reverse transcriptase (ThermoFisher, EP0751) were added, and the reaction was incubated at 55°C or 60°C for 1 hour, inactivated by heating at 85°C for 5 minutes, followed by RNAse digestion. The rationale behind a higher incubation temperature is that it better unwinds the tRNA structure and allows access to the reverse transcriptase. Following reverse transcription, RNA was digested by adding 1.5 µL of RNase Cocktail^{™} Enzyme Mix (ThermoFisher, AM2286) to the reaction and incubating at 37°C for 10 mins. The reactions were cleaned up using 1.5X Ampure XP beads as described, and the tRNA cDNA and input polyA tRNA was run on TapeStation using the RNA HS assay. While we cannot be sure that cDNA runs at the same speed as RNA on the TapeStation, we reasoned that we could still observe truncated cDNA templates as peaks in the electropherogram.

### S. cerevisiae tRNA reference set

Reference sequences for mature S. cerevisiae tRNAs were retrieved from GtRNAdb2 [Chan PP, Lowe TM. GtRNAdb 2.0: an expanded database of transfer RNA genes identified in complete and draft genomes. Nucleic Acids Res. 2016;44: D184-9.]. GtRNAdb2 reports 275 tRNA sequences annotated in the *S. cerevisiae* genome but there are only 43 unique isoacceptor-anticodon pairs (including Und-NNN). Most tRNA isoacceptors (i.e. with the same anticodon) have multiple copies, e.g. Asp-GTC and Gly-GCC have 16 copies each. Most of those copies are identical - there are only 55 unique, mature tRNA sequences. The remaining 12 sequences are highly similar copies, having 95-99% identity. For example, Asp-GTC-1 and Asp-GTC-2 have an identity of 96.9%. In order to facilitate reliable alignment and accurate tRNA quantification, we decided to keep only a single representative sequence for each isoacceptor-anticodon pair. We selected the first annotated sequence for each isoacceptor-anticodon pair in GtRNAdb2 (ie. Gly-GCC-1-1).

### Basecalling and mapping tRNA reads

Reads were basecalled using Guppy basecaller v3.6.1 in high-accuracy mode. All Us were converted to Ts before mapping. Basecalled reads were mapped using minimap2 v2.17-r941 with recommended parameters (-xmap -ont) or sensitive parameters (-ax map-ont -k5) or BWA v0.7.17-r1188. For BWA, two modes (MEM and SW) were tested, and several sets of parameters were invoked as follows (ordered from the most stringent to the least stringent settings): i) bwa mem -W13 -k6 -xont2d, ii) bwa mem -W13 -k6 -xont2d -T20, iii) bwamem -W13 -k6 -xont2d -T10, iv) bwamem -W9 -k5 -xont2d -T10 and v) bwasw -z10 -a2 -b1 -q2 -r1. Reads mapping to the reverse strand (antisense) were assigned as 'wrong alignments'. We selected the best performing algorithm and parameters (bwa mem -W13 - k6 -xont2d -T20) by comparing the number of uniquely aligned reads and the number of wrong alignments (Table 5). We should note that the sequence of 5' and 3' RNA adapters were included in the respective references when mapping the tRNA reads. The effect of the presence and the length of 5' and 3' RNA adapters on the mappability of the reads by shortening the respective adapter sequence from the alignment reference with a step of 5 nucleotides (Table 7).

### Analysis of tRNA abundances

Only unique (mapping quality above 0) primary alignments were considered. Differentially expressed tRNAs were inferred using DESeq2. Volcano plots were generated using EnhancedVolcano package [Blighe, Rana, Lewis. EnhancedVolcano: Publication-ready volcano plots with enhanced colouring and labeling. R package version.]. Differentially expressed tRNAs were defined as those having adjusted P-value <0.01 and absolute log2 expression fold change greater than 0.6.

### Analysis of differential tRNA modifications

Differential tRNA modifications were measured by using differential basecalling errors (mismatch, insertion, deletion), for each tRNA nucleotide. The sum of basecalling errors was calculated by subtracting the frequency of the reference base from 1. The frequency of reference base equals the number of reads with a basecalled equivalent to reference base, divided by the depth of coverage for that position. Only uniquely aligned reads (primary alignment with mapping quality above 0) were considered for downstream analyses.

### Adjusting MinKNOW parameters to capture small RNA molecules,

MinKNOW configurations may be adjusted as follows for enhanced capture small, RNA molecules such as tRNA, reads from direct RNA nanopore sequencing runs (if these parameters are not adjusted, MinKNOW will incorrectly discard small RNA reads, or tRNA reads as "adapter-only" reads, and these reads will never be stored in a FAST5 file or FASTQ file). Therefore, this step reinforces the recovery of small RNA or tRNA populations. We should note that "default" MinKNOW parameters will still capture small RNA or tRNAs, but this will be ~10X less reads (see Figure 10), and with biased representations of tRNAs (longer small RNA or tRNAs will be over-represented, compared to short tRNAs - see Figure 5E):
Sequencing runs can be conducted with the option of "bulk dump" raw file turned on, for all 512 channels. The sequencing simulations were performed with default and custom MinKNOW configurations. By default, MinKNOW defines the duration of the adapter up to 5 seconds and the strand (an actual read) at least 2 seconds. Thus, the RNA molecule has to spend up to 7 seconds in the pore in order to be classified and reported as an actual read. The motor protein (RNA helicase) used in DRS experiments has an average speed of 70 nt per second, thus 7 seconds corresponds to roughly 490 nt. Such definition makes sense for long molecule sequencing, as it filters out the adaptoronly reads. However, for short RNA sequencing, it would be reasonable to shorten both the adapter and strand definitions. We evaluated several configurations, shortening the duration of the adapter to 1 second and the strand to: 1, 2, 3 or 4 seconds. Subsequently, the number of reported, basecalled, aligned and uniquely aligned reads generated by default and custom MinKNOW configurations were compared. We observed that using the 1 second definition for adapter and 2 seconds for the strand resulted in the highest number of aligned and uniquely aligned reads (Table 8). Therefore, those settings are used across the manuscript unless stated otherwise.

### Step 1: Set up alternative MinKNOW configuration files

To set up alternative MinKNOW configurations, you must copy and enable the configuration files via rsync to using a terminal. This can be done using the following commands (root privileges needed):
$ rsync -a conf/package/flow_cells.toml /opt/ont/minknow/conf/package
$ rsync -a conf/package/sequencing/*.toml /opt/ont/minknow/conf/package/sequencing

### Step 2: Reload the scripts in MinKNOW,

Every time the scripts are reloaded, MinKNOW reads the configuration from:
- flowcells.toml (typically found in opt/ont/minknow/conf/packages/flowcells.toml)
- sequencing*.toml (typically found in /opt/minknow/conf/packages/sequencing/sequencing_*.toml)

To reload the scripts, you should just click the "reload scripts" button as shown in Figure 11:
You should now see alternative configurations in the flowcell type field (see Figure 12). MinKNOW with alternative configurations (such as "FLO-MIN106_short") can be now run which will allows to capture short tRNAs during the sequencing run, without having to save the bulk FAST5 file.

### Step 3. Launch sequencing (or simulation of sequencing run once you saved the "bulk" fast5 from a previous run) with the new MinKNOVV configuration:

You should run MinKNOW as for a normal sequencing run, but this time choosing the alternative configuration (FLO-MIN106-short) that has been set up. We should note that this can so far only be used on "bulk" dumps (raw current intensity files) that have been previously saved from your sequencing runs. To do this, you must ensure that you save the bulk file during your sequencing run (this is optional in MinKNOW, you must click this option). The bulk file should be specified in the configuration file, under the section `custom settings' of the ".toml" file. An example of this file is embedded below:

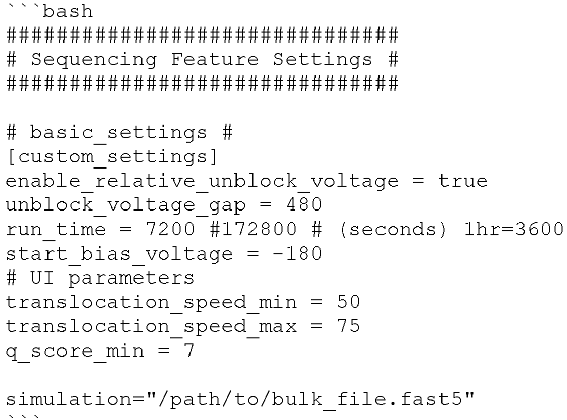

### Additional information: how to save the bulk file

This is an option given by MinKNOW software when you set up the parameters in thr run. You must tick this option and specify how many pores/channels you would like to save the current intensity information from (see Figure 13). A bulk file for 72h for all pores may reach 250-300Gb.

To enable bulk file saving, the user should tick the option "Output bulk file" as shown above, which will then save the continuous raw current intensity from a sequencing run. This bulk file can then be reprocessed using the alternative MinKNOW configurations, using the steps described above.

### Comparisons with published datasets

The *S. cerevisiae* tRNA expression estimations obtained by the method of the present invention were compared to estimates reported by orthogonal Illumina-based tRNA sequencing methods ARM-seq [Cozen AE, Quartley E, Holmes AD, Hrabeta-Robinson E, Phizicky EM, Lowe TM. ARM-seq: AlkBfacilitated RNA methylation sequencing reveals a complex landscape of modified tRNA fragments. Nat Methods. 2015;12: 879-884.], Hydro-tRNAseq [Gogakos T, Brown M, Garzia A, Meyer C, Hafner M, Tuschl T. Characterizing Expression and Processing of Precursor and Mature Human tRNAs by Hydro-tRNAseq and PAR-CLIP. Cell Rep. 2017;20: 1463-1475.] and mim-tRNAseq [Behrens A, Rodschinka G, Nedialkova DD. High-resolution quantitative profiling of tRNA abundance and modification status in eukaryotes by mim-tRNAseq. Mol Cell. 2021;81: 1802-1815.e7.]. The published estimates were reported per tRNA isoacceptor-anticodon pair and included the same references as the ones used in this work, with exception of Hydro-tRNAseq, that missed two (Leu-GAG and iMet-CAT) and reported additional five references (Leu-AAG, Leu-CAG, Ala-CGC, Pro-CGG and Arg-TCG). These references were excluded from pairwise comparisons with Hydro-tRNAseq.

### Results

### Standard nanopore DRS of tRNA molecules results in low sequencing yields and poor 5' end coverage

Nanopore direct sequencing, or nanopore direct RNA sequencing (DRS) is a well-established long-read sequencing technology to study RNA molecules, typically polyadenylated mRNAs. DRS is inefficient at capturing RNA molecules shorter than 200 nt, and is generally considered unable to capture sequences shorter than around 100 nt, limiting its applicability to study short RNA populations such as tRNAs. In addition, the first about15 nucleotides at the 5' end of RNA molecules are typically lost in DRS runs [Workman RE, Tang AD, Tang PS, Jain M, Tyson JR. Nanopore native RNA sequencing of a human poly (A) transcriptome. Nature. 2019. Available: https://www.nature.com/articles/s41592-019-0617-2], as this portion cannot be adequately basecalled due to the increase in the RNA translocation speed when the 5' end of the molecule exits the helicase. To overcome these limitations, we reasoned that extension of the 5' and 3' ends of the tRNA would lead to improved sequencing of tRNA molecules, as these would now be beyond the about100 nt threshold, in addition to capturing the sequence and modification information of 5' tRNA ends.

We first attempted a modified tRNA DRS approach in which a 5' RNA adapter (first splinted RNA oligonucleotide), complementary to the 3' CCA overhang present in mature tRNA molecules, was ligated to the 5' end of tRNAs that had been previously invitro polyadenylated (Strategy A, see Figure 2). A set of 9 synthetic in vitro transcribed (IVT) tRNAs (Table 2) were sequenced using this strategy. However, this approach produced very low sequencing yields (56,002 reads) than what is generally expected from a DRS run (about1-2 million reads). Moreover, only 7.5% of reads mapped uniquely to the tRNA reference set using minimap2 with recommended DRS mapping parameters (Table 4). Relaxation of the mapping parameters (-ax map-ont-k5), which had previously been shown to improve mappability of highly modified RNAs [Begik O, Lucas MC, Pryszcz LP, Ramirez JM, Medina R, Milenkovic I, et al. Quantitative profiling of pseudouridylation dynamics in native RNAs with nanopore sequencing. Nat Biotechnol. 2021. doi:10.1038/s41587-021-00915-6 ], did not yield a significant increase in the number of mapped tRNA reads (Table 4). In addition, we observed poor coverage of the 5' ends of the tRNA molecules, suggesting that the 5' RNA adapter ligation was inefficient, possibly due to the sterical interference of the 3' polyA tail with the annealing of the 5' RNA adapter or ligase accessibility.

In light of these results, we altered the library preparation protocol to replace the polyA tail with a 3' DNA adapter that was complementary to the 5' RNA adapter (Strategy B), such that the two oligonucleotides could be pre-annealed and ligated to the tRNA without hindrance from a polyA tail (Figure 2). However, this strategy also yielded a low number of sequenced reads (63,502 reads) and low percentage of uniquely mapped reads (6.5%) (Table 4). Despite the low number of mapped reads in Strategy B, there was a small improvement in the 5' end coverage of the tRNA molecules.

### Ligase comparison

### Analysis of commercial ligases for 1 st step of Nano-tRNAseq library preparation

The correct ligase for the first adapter ligation step of Nano-tRNAseq is T4 RNA ligase 2. However, commercial T4 RNA ligase 2 (NEB or Enzymatics) are not concentrated (10.000U/mL and 30.000U/mL, respectively). By contrast, T4 DNA ligase (NEB) is sold in concentrated format (2.000.000U/mL). For this reason, even though T4 DNA ligase is less efficient at ligating this reaction, its increased concentration causes that with equivalent volume, more ligation is achieved. In Fig 14 we can see that at equal units, T4 RNA ligase 2 is more efficient at this reaction, but would require too large volumes for library preparation.

### Comparison of T4 RNA ligase 2 (in-house) and concentrated T4 DNA ligase (NEB)

Here we compare the performance of in-house T4 RNA ligase 2 and concentrated commercial T4 DNA ligase (NEB) for the 1st Nano-tRNAseq library prep reaction (Figure 15)

### Oligos

SEQ ID No 27 EN-003FAM: 56-FAM-rArArArArArArArArArArArA
SEQ ID No 6 EN-010FAM_OligoA_ONT_shuffle1:
   /5Phos/GGCTTCTTCTTGCTCTTAGGTAGTAGGTTC/36-FAM/
SEQ ID No 7 EN-014 oligoB ONT_polydT:
   5'-GAGGCGAGCGGTCAATTTTCCTAAGAGCAAGAAGAAGCCTTTTTTTTTT-3'
Solutions: Formamide stop solution : 95% formamide, 20 mM EDTA, pH 8.0, 0.03% BPB, and 0.03% xylene cyanol FF;Quick DNA ligase (1X) : 50 mM Tris-HCI 10 mM MgCl2 5 mM DTT 1 mM ATP pH 7.6 at 25°C
Enzymes: Commercial T4 DNA Ligase (NEB), T4 RNA ligase 2 with no glycerol, SEQ ID No 2 Reaction
Preannealed oligos EN010:EN014 : 72.7 pmol, 4.378 x 10¹³ copy number
EN-003FAM: 37.6 pmol, 2.264 x 10¹³ copy number
Ligation performed at RT for 10 minutes. Ran on 15% TBE-Urea gel.

### Different incubation times (ligation gel and/or flongles)

### Oligos

**5' oligo**
   ML-047_ONT_tRNA_oligoB_RNA - 24nt
   SEQ ID NO 3 rCrCrU rArArG rArGrC rArArG rArArG rArArG rCrCrU rGrGrN
**OLD oligo** = **3' RNA oligo**
   ML-065 ONT tRNA 3'adaptor long_RNA (OLD) - 30nt
   SEQ ID NO 4
      /5Phos/rGrGrCrUrUrCrUrUrCrUrUrGrCrUrCrUrUrArGrGrArArArArArArArArArA
**NEW oligo** = **3' RNA-DNA oligo -> oligo used in the final Nano-tRNAseq library**
   ML-068_ONT_tRNA_3'adaptor_long_RNA+DNA (NEW) - 33nt
   SEQ ID NO 5
      /5Phos/rGrGrCrUrUrCrUrUrCrUrUrGrCrUrCrUrUrArGrGrArArArArArArArArArAAAA

### Flongle runs

### WT yeast

1. OLD RNA oligo overnight ligation
   Run ID: RNA261266
   Flowcell ID: AEY401
2. OLD oligo 2h ligation
   Run ID: RNA037486
   Flowcell ID: AJG115
3. NEW RNA-DNA oligo 2h ligation
   Run ID: RNA816787
   Flowcell ID: AJI950

### Commercial yeast

1. OLD oligo ON ligation RNA345135
2. NEW oligo 2h ligation RNA980403

**TABLE 3 Results using different oligos and ligation times**

| | **Basecalled reads** | **mapped** | **%** | **mapped uniquel y tRNA** | **%** | **antisense** | **%** | **Oligo 3** | **%** | **Oligo 5** | % |
|---|---|---|---|---|---|---|---|---|---|---|---|
| S.cerWT tRNA overnight ligation old 3' RNA oligo | 25,249 | 412 | 1.63 | 317 | 76.94 | 5 | 1.5 8 | 13 | 3.16 | 0 | 0.00 |
| S.cer WT tRNA 2h ligation old 3' RNA oligo | 12,688 | 134 | 1.06 | 86 | 64.18 | 1 | 1.1 6 | 2 | 1.49 | 5 | 3.73 |
| S.cer WT tRNA 2h ligation new 3' RNA-DNA oligo | 125,448 | 32,201 | 25.67 | 22,283 | 69.20 | 24 | 0.1 1 | 471 | 1.46 | 3 | 0.01 |
| Commerci al S.cer OLD oligo overnight ligation | 44,376 | 3,568 | 8.04 | 2,680 | 75.11 | 6 | 0.2 2 | 107 | 3.00 | 0 | 0.00 |
| Commerci al S.ce NEW oligo 2h ligation | 83,495 | 11,475 | 13.74 | 8,462 | 73.74 | 9 | 0.1 1 | 86 | 0.75 | 0 | 0.00 |

### Double ligation of RNA adapters to the tRNA molecule ends improves basecalling of tRNAs

Based on the results of Strategy A and B, we rationalized that padding the 5' and 3' tRNA ends with RNA adapters which can be accurately basecalled and mapped would enable us to capture the entirety of the tRNA sequence. This approach is the method of the present invention, also termed NanotRNAseq (Figure 2) was the most successful at sequencing, basecalling and mapping both in vitro and native tRNA molecules using nanopore DRS (Table 3).

A 5' RNA adapter complementary to the CCA overhang of mature tRNAs is pre-annealed to a 3' RNA adapter containing 3 DNA bases at the 3' end (Figure 2). Surprisingly we observed that RNA-only 3' adapters led to increased self-ligation (Table 4), an issue that we later mitigated by adding DNA bases to the end of the adapter Next, the pre-annealed 5' RNA and 3' RNA:DNA splint adapters were ligated to deacylated tRNAs by T4 RNA Ligase 2. Diverse ligation times and the addition of a molecular crowding agent were tested, to ensure that conditions that maximized ligation efficiency were chosen. The addition of 20% PEG8000 improved ligation efficiency, as did running the reaction either overnight at 4°C, or for 2 hours at room temperature, compared to 20 minutes at room temperature (Figure 3A). As there was little difference in the overnight versus 2 hour ligation, the latter was selected, as it permitted us to complete the library preparation protocol within one day. Following this, ONT RTA adapter A and ONT RTA adapter B were pre-annealed and ligated using T4 DNA Ligase. This approach resulted in 208,000 basecalled reads (Table 4), thus significantly increasing sequencing output by 3-4 fold relative to the previous strategies, and also improved 5' and 3' coverage of both synthetic and biological tRNAs (Figure 3B). In addition, we found that the recapitulated abundances using Nano-tRNAseq were highly replicable both when sequencing in vitro as well as native tRNA datasets (ρ=0.984) (Figure 3C).

### Mapping parameters and adapter length significantly affect tRNA read mappability

The alignment of native tRNA reads is challenging due to their short and highly modified nature. Indeed, native tRNAs contain a large proportion of mismatched bases, which often originate from inaccurate basecalling of modified bases in DRS datasets [90,94,97]. As a consequence of these miscalled bases, the commonly used long-read mapper minimap2 with recommended settings (-x map-ont) aligned only a fraction (2.56%) of the reads (Figure 2A-C, see also Table 4).

To improve the mappability of Nano-tRNAseq reads, we first examined whether short-read mapping algorithms, such as *bwa* [Li H, Durbin R. Fast and accurate short read alignment with Burrows-Wheeler transform. Bioinformatics. 2009;25: 1754-1760.], might lead to an increased proportion of mapped reads. Using sequencing data from a Nano-tRNAseq run that contained 3 different tRNA constructs (IVT *Drosophila melanogaster* mit tRNA^{Ala(UGC)}, IVT *Streptococcus pneumoniae* tRNA^{Ser(UGA)} and native S. *cerevisiae* tRNA^{Phe)}, we found that the bwa-mem aligner outperformed minimap2 in terms of proportion of mapped reads (Figure 4C). While more relaxed configurations of bwa-mem aligned more reads, this also came at the expense of increased false alignments (antisense mapped reads were used as a proxy of mismapping) (Figure 4C, see also Table 5). A sweet spot was found when using bwa-mem -W13 -k6 -xont2d -T20, which mapped 54.63% of the reads, with very few false alignments (0.19%). When comparing mapping capabilities of bwa-mem with minimap2 in native tRNA molecules, the contrast was even more stark; while minimap2 mapped IVT tRNAs, it failed to map a single biological tRNA read (Figure 4D, see also Table 6). The alignment identity was comparable to minimap2 for reads that mapped to IVT tRNAs, but was slightly lower than typical identity obtained in nanopore DRS runs, suggesting that short reads, even without modifications, cause a drop in the basecalling accuracy (Figure 4E). Notably, the alignment identity of S. cerevisiae tRNA^{Phe} was lower (-74.5%) than in synthetic tRNAs (81.8%), presumably due to the presence of base modifications present on endogenously modified *S. cerevisiae* tRNA^{Phe} (Figure 4E, see also Table 6).

We then quantified whether the mappability of Nano-tRNAseq reads might be affected by the length of the 5' and 3' RNA adapters. In order to simulate different RNA adapter lengths, we trimmed one or both adapters from the reference sequences. We found that the absence of both RNA adapters only had a modest effect on mappability of reads originating from IVT tRNAs (decrease of 6-11%) (Figure 4F, see also Table 7), while 55% fewer reads from native S. *cerevisiae* tRNA^{Phe} aligned to the reference. Accordingly, short and unmodified sequences can be aligned efficiently even without RNA adapters in the reference sequences, while short and modified reads benefit greatly from the extension from adapters, demonstrating that extending molecules with RNA adapters is essential for guiding the correct alignment of short reads enriched in 'mismatches', such as those derived from native tRNAs. For native *S. cerevisiae* tRNA^{Phe}, the read mappability plateaued at a 3' adapter length of 25 nt (Figure 4F), demostrating that our 30 nt RNA portion of 3' RNA:DNA used in Nano-tRNAseq is more than sufficient to achieve optimal read mappability.

**Table 4: Sequencing, mimimap mapping and self-ligation statistics of Strategy A, Strategy B and the method of the invention**

| | | | | | | **Uniquely mapped reads (#)** | | **Uniquely mapped reads (% relative to base-called)** | | |
|---|---|---|---|---|---|---|---|---|---|---|
| **Run ID** | **Templates** | **Strategy** | **Basecalle d reads (#)** | **MinKNOW sequencin g mode** | **Seque ncing time (h)** | **minimap 2-xmap-ont** | **minimap 2 -ax map-ont -k5** | **minimap2 -xmap-ont** | **minimap 2 -ax map-ont -k5** | **Reads from self-ligated 3' RNA adapters (#)** |
| 1_strategyA_IVT | 9x IVT tRNAs | A | 56.002 | Default | 48 | 4.213 | 6.508 | 7,52 | 11,62 | NA |
| 2_strategyB_IVT | 4x IVT tRNAs | B | 63.502 | Default | 45 | 4.126 | 7.596 | 6,50 | 11,96 | NA |
| 3_NanotRNAseq_ IVT+tRNAphe | 9x IVT tRNAs & S. *cerevisiae* tRNA^{Phe} | NanotRNAseq (3' RNA adapter) | 208.000 | Default | 72 | 7.800 | 19.420 | 3,75 | 9,34 | 179 |
| 4_NanotRNAseq_ IVT+tRNAphe | 2x IVT tRNAs + S. *cerevisiae* tRNA^{Phe} | NanotRNAseq (3' RNA adapter) | 51.352 | Default | 20 | 1.315 | 6.640 | 2,56 | 12,93 | 2 |
| 5_NanotRNAseq_ WTyeast_rep1_noRT | *S*. *cerevisiae* WT total tRNA without RT | NanotRNAseq (3' RNA adapter) | 162.854 | Default | 42 | 304 | 1.250 | 0,19 | 0,77 | 54 |
| 6_NanotRNAseq_ WTyeast_rep1_RT | *S*. *cerevisiae* WT total tRNA with RT | NanotRNAseq (3' RNA adapter) | 153.263 | Default | 42 | 217 | 908 | 0,14 | 0,59 | 118 |
| 7_NanotRNAseq_ WTyeast_rep1 | *S*. *cerevisiae* WT total tRNA | NanotRNAseq | 380.959 | Default | 72 | 41.198 | 58.698 | 10,81 | 15,41 | 1 |
| 8_NanotRNAseq_ pus4KOyeast_rep1 | *S*. *cerevisiae* Pus4 KO total tRNA | NanotRNAseq | 376.606 | Default | 72 | 33.140 | 48.347 | 8,80 | 12,84 | 7 |
| 9_NanotRNAseq_ Heatstressyeast_rep1 | *S*. *cerevisiae* heat stress total tRNA | NanotRNAseq | 588.336 | Default | 72 | 66.798 | 91.252 | 11,35 | 15,51 | 8 |
| 10_NanotRNAseq_ Oxidativestressyeast_rep 1 | *S*. *cerevisiae* oxidative stress total tRNA | NanotRNAseq | 343.750 | Default | 72 | 20.712 | 31.339 | 6,03 | 9,12 | 1 |
| 11_NanotRNAseq_ WTyeast_rep2 | *S*. *cerevisiae* WT total tRNA | NanotRNAseq | 495.919 | Default | 72 | 35.304 | 54.448 | 7,12 | 10,98 | 16 |
| 12_NanotRNAseq_ pus4KOyeast_rep2 | *S*. *cerevisiae* Pus4 KO total tRNA | NanotRNAseq | 586.456 | Default | 72 | 35.183 | 56.433 | 6,00 | 9,62 | 6 |
| 13_NanotRNAseq_ Heatstressyeast_rep2 | *S*. *cerevisiae* heat stress total tRNA | NanotRNAseq | 614.586 | Default | 72 | 56.902 | 79.039 | 9,26 | 12,86 | 9 |
| 14_NanotRNAseq_ Oxidativestressyeast_rep 2 | *S*. *cerevisiae* oxidative stress total tRNA | NanotRNAseq | 497.752 | Default | 72 | 24.070 | 38.530 | 4,84 | 7,74 | 9 |

**Table 5. Mapping statistics of Nano-tRNAseq using different mapping algorithms and parameters.**

| **Run ID** | **Basecalled reads (#)** | **Mapping parameters** | **Mapped reads (#)** | **Mapped reads (%)** | **Mapped reads to tRNA (#)** | **Mapped reads to tRNA (%)** | **Uniquely mapped reads to tRNA (#)** | **Uniquely mapped reads to tRNA (%)** | **Antisense tRNA (#)** | **Antisense tRNA (%)** |
|---|---|---|---|---|---|---|---|---|---|---|
| 4_NanotRNAseq_ IVT+tRNAphe | 51.391 | minimap2 -xmap-ont | 1.317 | 2,56 | 1.317 | 2,56 | 1.315 | 2,56 | 0 | 0 |
| | | bwamem | 6.455 | 12,56 | 6.455 | 12,56 | 6.425 | 12,50 | 2 | 0,03 |
| | | bwamem -W13 -k6 -xont2d | 24.087 | 46,87 | 24.086 | 46,87 | 23.846 | 46,40 | 6 | 0,03 |
| | | bwamem -W13 -k6 -xont2d -T20 | 30.131 | 58,63 | 30.126 | 58,62 | 28.101 | 54,68 | 53 | 0,19 |
| | | bwamem -W13 -k6 -xont2d -T10 | 39.686 | 77,22 | 39.647 | 77,15 | 30.926 | 60,18 | 1.886 | 6,1 |
| | | bwamem -W9 -k5 -xont2d -T10 | 43.590 | 84,82 | 43.559 | 84,76 | 31.337 | 60,98 | 2.677 | 8,54 |
| | | bwasw | 3.284 | 6,39 | 3.284 | 6,39 | 3.219 | 6,26 | 2 | 0,06 |
| | | bwasw -z10 -a2 -b1 -q2 -r1 | 31.182 | 60,68 | 30.446 | 59,24 | 27.045 | 52,63 | 2.457 | 9,08 |
| 3_NanotRNAseq_ IVT+tRNAphe | 208.022 | minimap2 -xmap-ont | 7.976 | 3,83 | 7.976 | 3,83 | 7.795 | 3,75 | 2 | 0,03 |
| | | bwamem | 25.835 | 12,42 | 25.835 | 12,42 | 25.518 | 12,26 | 33 | 0,13 |
| | | bwamem -W13 -k6 -xont2d | 64.085 | 30,80 | 64.081 | 30,80 | 56.038 | 26,93 | 82 | 0,15 |
| | | bwamem -W13 -k6 -xont2d -T20 | 80.026 | 38,46 | 80.004 | 38,45 | 61.161 | 29,40 | 220 | 0,36 |
| | | bwamem -W13 -k6 -xont2d -T10 | 126.331 | 60,72 | 126.267 | 60,69 | 71.743 | 34,48 | 9.941 | 13,86 |
| | | bwamem -W9 -k5 -xont2d -T10 | 153.563 | 73,81 | 153.477 | 73,76 | 74.734 | 35,92 | 14.189 | 18,99 |
| | | bwasw | 15.629 | 7,51 | 15.627 | 7,51 | 15.162 | 7,29 | 18 | 0,12 |
| | | bwasw -z10 -a2 -b1 -q2 -r1 | 71.599 | 34,42 | 68.155 | 32,76 | 51.423 | 24,72 | 9.172 | 17,84 |

**Table 6. Alignment identity of IVT and native S. cerevisiae tRNA^{Phe} with different mapping algorithms and parameters.**

| | | | | **Mapping (%)** | | | **Alignment identity (%) including RNA adapters** | | | **Alignment identity (%) of tRNA body** | | |
|---|---|---|---|---|---|---|---|---|---|---|---|---|
| **Run ID** | **Aligner parameters** | **Align reads (#)** | **Align reads (%)** | **IVT3_Dm_ mitAla_UG C** | **IVT6_Sp Ser_UG A** | **Sc_Ph e** | **IVT3_Dm_ mitAla_UG C** | **IVT6_Sp_S er_UGA** | **Sc_Ph e** | **IVT3_Dm_ mitAla_UG C** | **IVT6_Sp Ser_UG A** | **Sc_Ph e** |
| 4_NanotRNAse q_ IVT+tRNAphe | minimap2 -x map-ont | 1.315 | 2,56 | 17,30 | 82,70 | 0,00 | 93,0 | 88,7 | 0,0 | 95,20 | 89,20 | 0,00 |
| | bwamem | 6.427 | 12,52 | 13,60 | 84,90 | 1,50 | 94,3 | 92,9 | 96,1 | 94,80 | 93,20 | 99,40 |
| | bwamem-W13-k6 - xont2d | 23.851 | 46,45 | 10,10 | 54,70 | 35,10 | 83,7 | 81,5 | 75,3 | 84,40 | 81,40 | 71,60 |
| | bwamem-W13-k6-xont2d-T20 | 28.138 | 54,79 | 10,20 | 48,40 | 41,30 | 82,4 | 81,2 | 74,5 | 82,90 | 81,00 | 71,10 |
| | bwamem-W13-k6-xont2d-T10 | 32.743 | 63,76 | 16,60 | 43,90 | 39,50 | 79,7 | 81,2 | 74,5 | 80,10 | 81,20 | 71,50 |
| | bwamem-W9-k5-xont2d-T10 | 33.951 | 66,11 | 18,70 | 42,80 | 38,50 | 79,1 | 81,3 | 74,5 | 79,60 | 81,20 | 71,50 |
| | bwasw | 3.221 | 6,27 | 15,90 | 82,30 | 1,70 | 96,1 | 95,0 | 97,7 | 96,50 | 95,10 | 98,70 |
| | bwasw-z10-a2- b1-q2-r1 | 28.387 | 55,28 | 9,60 | 45,90 | 44,50 | 73,3 | 78,0 | 70,4 | 69,40 | 73,90 | 66,20 |

**Table 7. Mappings statistics of different adapter regions and lengths.**

| | | | **Uniquely mapped reads (#)** | | | **Fraction of reads aligned (compared to full length oligos)** | | |
|---|---|---|---|---|---|---|---|---|
| **Run ID** | **5' adapter length (nt)** | **3' adapter length (nt)** | **IVT3_Dm_mitAla _UGC (68 nt)** | **IVT6_Sp_Ser _UGA (93 nt)** | **Sc_Phe (76 nt)** | **IVT3_Dm_mitAla _UGC (68 nt)** | **IVT6_Sp_Ser _UGA (93 nt)** | **Sc_Phe (76 nt)** |
| 4_NanotRNAseq_ IVT+tRNAphe | 24 | 30 | 17,818 | 20,010 | 16,455 | 1,000 | 1,000 | 1,000 |
| | 0 | 0 | 15,908 | 18,852 | 7,353 | 0,893 | 0,942 | 0,447 |
| | 24 | 25 | 17,634 | 19,800 | 16,890 | 0,990 | 0,990 | 1,026 |
| | 24 | 20 | 17,222 | 19,488 | 14,854 | 0,967 | 0,974 | 0,903 |
| | 24 | 15 | 17,101 | 19,378 | 14,587 | 0,960 | 0,968 | 0,886 |
| | 24 | 10 | 16,861 | 19,186 | 13,699 | 0,946 | 0,959 | 0,833 |
| | 24 | 5 | 16,526 | 19,043 | 12,679 | 0,927 | 0,952 | 0,771 |
| | 24 | 0 | 16,025 | 18,927 | 10,010 | 0,899 | 0,946 | 0,608 |
| | 20 | 30 | 17,814 | 20,011 | 16,462 | 1,000 | 1,000 | 1,000 |
| | 15 | 30 | 17,815 | 20,026 | 16,483 | 1,000 | 1,001 | 1,002 |
| | 10 | 30 | 17,751 | 20,026 | 16,228 | 0,996 | 1,001 | 0,986 |
| | 5 | 30 | 17,671 | 20,016 | 15,847 | 0,992 | 1,000 | 0,963 |
| | 0 | 30 | 17,623 | 20,014 | 15,676 | 0,989 | 1,000 | 0,953 |

### Custom MinKNOW parameters leads to a 12-fold increase in sequencing yield

A surprising feature of our initial tRNA sequencing runs was the low amount of sequenced reads. While pore clogging caused by tRNA structure might partially explain the low sequencing yield, we also noticed that the current MinKNOW software (22/07/2022 and previous versions) classified a high proportion of reads as "adapter-only" reads in real time. Hence, we hypothesized that a considerable fraction of tRNA reads might be discarded by the MinKNOW software due to their short signal lengths, as they resemble "adapter-only" reads.

The MinKNOW software is responsible for analyzing the continuous electrical current (signal intensity) measured at each pore, reporting the signal regions that correspond to 'reads' into FAST5 files, which are then basecalled to generate a FASTQ file. We noted that MinKNOW by default reports reads that last at least 2 seconds, which roughly corresponds to RNA molecules of 140 nt (assuming constant helicase processivity of 70 nt/s in DRS). Considering that canonical tRNA molecules are ~73 nt, this would imply that even after double RNA adapter ligation (where 24 and 30 RNA nucleotides are added to the 5' and 3' ends of the tRNA molecule, respectively), the size of the ligated tRNA molecule would still be below the threshold, possibly leading to misassignment of tRNA reads as "adapter-only" reads. To alleviate this issue, we tested whether alternative MinKNOW configurations would improve the classification of tRNA reads, and consequently, boost sequencing yields. To this end, the bulk dump files were saved during the sequencing run, and these were reprocessed using diverse MinKNOW configurations (Table 8).

**Table 8. Mapping and basecalling statistics of MinKNOW with standard and custom read capture configurations.**

| **Run ID** | **Strategy** | **Templates** | **MinKNO W config** | **Adaptor maximu m duratio n (s)** | **Strand minimu m duratio n (s)** | **Ru n tim e (h)** | **Pore s save d (#)** | **Basecall ed reads (#)** | **Fold change (basecall ed)** | **Uniquel y mapped reads (#)** | **Unique ly mappe d reads (%)** | **Fold change (mappe d)** |
|---|---|---|---|---|---|---|---|---|---|---|---|---|
| 1_strategyA_IVT | A | 9x IVT tRNAs | default | 5 | 2 | 48 | NA | 56,002 | 1,00 | 36,295 | 64,81 | 1,00 |
| 2_strategyB_IVT | B | 4x !VT tRNAs | default | 5 | 2 | 45 | NA | 63,502 | 1,00 | 45,123 | 71,06 | 1,00 |
| 3_NanotRNAseq_IVT +tRNAphe | NanotRNAseq (3' RNA adapter) | 9x !VT tRNAs & S. *cerevisiae* tRNA^{Phe} | default | 5 | 2 | 72 | 512 | 208,000 | 1,00 | 63,466 | 30,51 | 1,00 |
| | | | custom | 2 | 1 | 72 | 512 | 2,537,89 3 | 12,20 | 281,678 | 11,10 | 4,44 |
| | | | custom2 | 1 | 1 | 72 | 512 | 3,292,90 5 | 15,83 | 296,890 | 9,02 | 4,68 |
| 4_NanotRNAseq_ IVT+tRNAphe | NanotRNAseq (3' RNA adapter) | 2x IVT tRNAs + S. *cerevisiae* tRNA^{Phe} | default | 5 | 2 | 12 | 50 | 31,458 | 1,00 | 17,605 | 55,96 | 1,00 |
| | | | custom | 2 | 1 | 12 | 50 | 211,786 | 6,73 | 54,389 | 25,68 | 3,09 |
| | | | custom2 | 1 | 1 | 12 | 50 | 257,932 | 8,20 | 46,245 | 17,93 | 2,63 |
| 5_NanotRNAseq_WTyea st _rep1_noRT | NanotRNAseq (3' RNA adapter) | S. *cerevisiae* WT total tRNA without RT | default | 5 | 2 | 42 | 512 | 162,854 | 1,00 | 31,819 | 19,54 | 1,00 |
| | | | custom | 2 | 1 | 42 | 512 | 1,568,00 0 | 9,63 | 59,408 | 3,79 | 1,87 |
| 6_NanotRNAseq_ WTyeast_rep1_RT | NanotRNAseq (3' RNA adapter) | S. *cerevisiae* WT total tRNA with RT | default | 5 | 2 | 42 | 512 | 153,263 | 1,00 | 25,810 | 16,84 | 1,00 |
| | | | custom | 2 | 1 | 42 | 512 | 2,316,26 6 | 15,11 | 90,866 | 3,92 | 3,52 |
| 7_NanotRNAseq_ WTyeast_rep1 | NanotRNAseq | S. *cerevisiae* WT total tRNA | default | 5 | 2 | 72 | 512 | 380,959 | 1,00 | 248,683 | 65,28 | 1,00 |
| | | | custom | 2 | 1 | 72 | 512 | 1,162,29 3 | 3,05 | 542,771 | 46,70 | 2,18 |
| 8_NanotRNAseq_ pus4KOyeast_rep1 | NanotRNAseq | S. *cerevisiae* Pus4 KO total tRNA | default | 5 | 2 | 72 | 512 | 376,606 | 1,00 | 248,061 | 65,87 | 1,00 |
| | | | custom | 2 | 1 | 72 | 512 | 784,714 | 2,08 | 378,299 | 48,21 | 1,53 |
| 9_NanotRNAseq_ Heatstressyeast_rep1 | NanotRNAseq | *S*. *cerevisiae* heat stress total tRNA | default | 5 | 2 | 72 | 512 | 588,336 | 1,00 | 384,900 | 65,42 | 1,00 |
| | | | custom | 2 | 1 | 72 | 512 | 1,716,06 5 | 2,92 | 788,830 | 45,97 | 2,05 |
| 10_NanotRNAseq_ Oxidativestressyeast_rep 1 | NanotRNAseq | *S*. *cerevisiae* oxidative stress total tRNA | default | 5 | 2 | 72 | 512 | 343,750 | 1,00 | 222,638 | 64,77 | 1,00 |
| | | | custom | 2 | 1 | 72 | 512 | 652,249 | 1,90 | 304,056 | 46,62 | 1,37 |
| 11_NanotRNAseq_ WTyeast_rep2 | NanotRNAseq | *S*. *cerevisiae* WT total tRNA | default | 5 | 2 | 72 | 512 | 495,919 | 1,00 | 322,138 | 64,96 | 1,00 |
| | | | custom | 2 | 1 | 72 | 512 | 1,702,24 0 | 3,43 | 786,434 | 46,20 | 2,44 |
| 12_NanotRNAseq_ pus4KOyeast_rep2 | NanotRNAseq | *S*. *cerevisiae* Pus4 KO total tRNA | default | 5 | 2 | 72 | 512 | 586,456 | 1,00 | 383,645 | 65,42 | 1,00 |
| | | | custom | 2 | 1 | 72 | 512 | 1,001,99 3 | 1,71 | 428,994 | 42,81 | 1,12 |
| 13_NanotRNAseq_ Heatstressyeast_rep2 | NanotRNAseq | *S*. *cerevisiae* heat stress total tRNA | default | 5 | 2 | 72 | 512 | 614,586 | 1,00 | 388,139 | 63,15 | 1,00 |
| | | | custom | 2 | 1 | 72 | 512 | 1,477,00 2 | 2,40 | 639,413 | 43,29 | 1,65 |
| 14_NanotRNAseq_Oxida tivestressyeast_rep2 | NanotRNAseq | *S*. *cerevisiae* oxidative stress total tRNA | default | 5 | 2 | 72 | 512 | 497,752 | 1,00 | 317,876 | 63,86 | 1,00 |
| | | | custom | 2 | 1 | 72 | 512 | 1,591,90 4 | 3,20 | 709,920 | 44,60 | 2,23 |

By lowering the MinKNOW strand minimum duration to 1 second and the adapter maximum duration to 2 seconds, a configuration herein referred to as "custom", we captured ~12 fold more basecalled and ~4.5 fold more uniquely mapped tRNA reads compared to the default MinKNOW configuration (Figure 5A-B, see also Table 8). Notably, we found that the default MinKNOW configuration does not only lead to low sequencing outputs, but actually causes significant biases in the relative abundances of tRNA molecules. Specifically, we found a greater representation of shorter tRNAs in our custom configuration (Figure 5C-D, see also Table 9), suggesting that default MinKNOW configuration is discarding shorter tRNA molecules, and preferentially capturing longer ones, such as tRNA molecules with variable arms (e.g. tRNA^{Leu}, tRNA^{Arg}, tRNA^{ser}). Moreover, the relative proportion of tRNA reads was much better recapitulated using the custom configuration than default settings (Figure 5E) and the reported tRNA abundances (ie. mapped tRNA reads) using custom settings correlated very well to the expected values (p=0.94) (Figure 5F, see also Tables 10-11).

**Table 9. Fold change of IVT tRNAs of various lengths when using default vs custom MinKNOW read capture configurations.**

| | **Run ID** | |
|---|---|---|
| | 3_NanotRNAseq_IVT+tRNAphe | |
| | **tRNA length** | **Fold change custom/default** |
| **IVT8_Hs_mitSer_GCU** | 62 | 8,7 |
| **IVT3_Dm_mitAla_UGC** | 68 | 7,2 |
| **IVT7_Hs Gly_ACC** | 74 | 2,4 |
| **IVT5_Nc_Thr_AGU** | 75 | 5,5 |
| **IVT1_Pf_Lys_UUU** | 76 | 4,3 |
| **IVT9_Hs_Gly_CCC** | 76 | 3,2 |
| **IVT2_Dm_Ser_GCU** | 85 | 3,3 |
| **IVT4_Ec_Ser_GCU** | 93 | 3,8 |
| **IVT6_Sp_Ser_UGA** | 93 | 2,7 |

**Table 10. Proportions of IVT tRNAs and S. cerevisiae tRNA^{Phe} when using default vs custom MinKNOW read capture configurations.**

| | | **Run ID** | | |
|---|---|---|---|---|
| | | 4_NanotRNAseq_IVT+tRNAphe | | |
| | **Configuration** | **Default** | **Custom** | **Expected** |
| **Sc_Phe (76 nt)** | **Mapped reads (#)** | 7,127 | 16,465 | - |
| | **Mapped reads (%)** | 40,48 | 30,29 | 33,00 |
| | **Mapped reads (#)** | 2,058 | 17,902 | - |
| **IVT3_Dm_mi tAla_UGC (68 nt)** | **Mapped reads (%)** | 11,69 | 32,83 | 33,00 |
| **IVT6_Sp_Ser _UGA (93 nt)** | **Mapped reads (#)** | 8,420 | 20,022 | - |
| | **Mapped reads (%)** | 47,83 | 36,88 | 33,00 |

**Table 11. Expected vs observed proportion of IVT tRNAs when using the custom MinKNOW read capture configuration.**

| | **Run ID** | |
|---|---|---|
| | 3_NanotRNAseq_IVT+tRNAphe | |
| | **Observed (log exp per 1000)** | **Expected (log exp per 1000)** |
| **IVT1_Pf Lys_UUU** | 0,255 | 0,000 |
| **IVT2_Dm_Ser_GCU** | 0,114 | 0,301 |
| **IVT3_Dm_mitAla_UGC** | 1,274 | 0,591 |
| **IVT4_Ec_Ser_GCU** | 0,826 | 0,892 |
| **!VT5_Nc_Thr_AGU** | 1,436 | 1,193 |
| **IVT6_Sp_Ser_UGA** | 1,602 | 1,797 |
| **IVT7_Hs Gly_ACC** | 2,250 | 2,097 |
| **IVT8_Hs_mitSer_GCU** | 2,707 | 2,398 |
| **IVT9_Hs_Gly_CCC** | 2,327 | 2,699 |

### Reverse transcription of tRNAs increases sequencing yield and decreases pore clogging

We next questioned whether using reverse transcription (RT) to remove tRNA structure would further improve the sequencing yield of the method of the invention. A linear tRNA molecule may (i) reduce the clogging of pores, allowing more reads to be sequenced and maintain the integrity of the flowcell longer, and/or (ii) stabilize the tRNA translocation speed through the pore, improving the accuracy of basecalling algorithms. We should note, however, that tRNAs are difficult to fully and accurately reverse transcribe due to their compact secondary and tertiary structures as well as their abundance of modifications that disrupt the Watson-Crick base pairing. To examine whether tRNA linearization might improve sequencing yield, we tested a range of commercial reverse transcriptases and incubation conditions on both IVT and native tRNAs, and examined their cDNA outputs (Figure 6A, see also Methods). We found that both Maxima and SuperScript IV at about 60°C offered the best performance in terms of production of full length cDNA products, and opted to use Maxima at about 60°C in our subsequent tRNA sequencing experiments (Figure 6B).

Next, we examined whether linearization of the tRNAs would increase our sequencing yields. To this end, total tRNA from *S. cerevisiae* was sequenced using Nano-tRNAseq with and without the reverse transcription step. Notably, the default MinKNOW configuration *without reverse transcription* condition resulted in more reads compared to the *with reverse transcription condition.* We hypothesize that these results may be explained by the fact that non-linearized tRNAs are highly structured, causing the helicase enzyme to process these molecules more slowly (Figure 6C), and ultimately making them more likely to be classified as a 'read' by the default MinKNOW configuration. Using the custom MinKNOW configuration (Figure 5B-E), the number of basecalled reads with reverse transcription was in fact ~1.5 fold higher, compared to without reverse transcription (Figure 6D, see also Table 12). Likewise, the number of reads uniquely mapped to tRNAs increased by ~1.5 fold with reverse transcription, and the relative abundance of tRNA isoacceptors was not affected by the linearization step. Overall, linearization of tRNA molecules improved the sequencing yields by increasing the helicase translocation rate.

**Table 12. Basecalling and mapping statistics of S. cerevisiae total tRNAs sequenced with and without Reverse Transcription (RT).**

| | **5_NanotRNAseq_WTyeast_r ep1_noRT** | | **6_NanotRNAseq_WTyeast_rep1 _RT** | | |
|---|---|---|---|---|---|
| | **Yeast tRNA without RT** | | **Yeast tRNA with RT** | | |
| | **Reads (#)** | **Reads (%)** | **Reads (#)** | **Reads (%)** | **Fold change (to no RT)** |
| Total basecalled | 1,568,000 | - | 2,316,266 | - | 1,48 |
| Total uniquely mapped | 59,408 | 3,79 | 90,866 | 3,92 | 1,53 |
| Total mapped | 208,369 | 13,29 | 350,802 | 15,15 | 1,68 |
| Ala-AGC | 780 | 1,31 | 1,252,00 | 1,38 | |
| Ala-TGC | 1,289 | 2,17 | 1,852,00 | 2,04 | |
| Arg-ACG | 2,009 | 3,38 | 3,200,00 | 3,52 | |
| Arg-CCG | 1,010 | 1,70 | 1,239,00 | 1,36 | |
| Arg-CCT | 1,900 | 3,20 | 2,415,00 | 2,66 | |
| Arg-TCT | 1,864 | 3,14 | 3,118,00 | 3,43 | |
| Asn-GTT | 686 | 1,15 | 1,039,00 | 1,14 | |
| Asp-GTC | 5,787 | 9,74 | 10,307,00 | 11,34 | |
| Cys-GCA | 1,702 | 2,86 | 2,282,00 | 2,51 | |
| Gln-CTG | 2,218 | 3,73 | 3,042,00 | 3,35 | |
| Gln-TTG | 759 | 1,28 | 1,208,00 | 1,33 | |
| Glu-CTC | 443 | 0,75 | 679,00 | 0,75 | |
| Glu-TTC | 2,079 | 3,50 | 3,022,00 | 3,33 | |
| Gly-CCC | 582 | 0,98 | 793,00 | 0,87 | |
| Gly-GCC | 14,699 | 24,74 | 21,533,00 | 23,70 | |
| Gly-TCC | 594 | 1,00 | 807,00 | 0,89 | |
| His-GTG | 1,048 | 1,76 | 1,483,00 | 1,63 | |
| !!e-AAT | 1,170 | 1,97 | 1,765,00 | 1,94 | |
| Ile-TAT | 212 | 0,36 | 302,00 | 0,33 | |
| Leu-CAA | 1,635 | 2,75 | 2,497,00 | 2,75 | |
| Leu-GAG | 239 | 0,40 | 374,00 | 0,41 | |
| Leu-TAA | 472 | 0,79 | 804,00 | 0,88 | |
| Leu-TAG | 652 | 1,10 | 1,044,00 | 1,15 | |
| Lys-CTT | 754 | 1,27 | 1,103,00 | 1,21 | |
| Lys-TTT | 1,025 | 1,73 | 1,688,00 | 1,86 | |
| Met-CAT | 154 | 0,26 | 246,00 | 0,27 | |
| Phe-GAA | 1,034 | 1,74 | 1,712,00 | 1,88 | |
| Pro-AGG | 275 | 0,46 | 431,00 | 0,47 | |
| Pro-TGG | 1,261 | 2,12 | 1,952,00 | 2,15 | |
| Ser-AGA | 4,103 | 6,91 | 6,713,00 | 7,39 | |
| Ser-CGA | 24 | 0,04 | 27,00 | 0,03 | |
| Ser-GCT | 271 | 0,46 | 375,00 | 0,41 | |
| Ser-TGA | 35 | 0,06 | 66,00 | 0,07 | |
| Thr-AGT | 812 | 1,37 | 1,235,00 | 1,36 | |
| Thr-CGT | 56 | 0,09 | 75,00 | 0,08 | |
| Thr-TGT | 117 | 0,20 | 193,00 | 0,21 | |
| Trp-CCA | 656 | 1,10 | 1,002,00 | 1,10 | |
| Tyr-GTA | 1,124 | 1,89 | 1,817,00 | 2,00 | |
| Val-AAC | 2,296 | 3,86 | 3,726,00 | 4,10 | |
| Val-CAC | 230 | 0,39 | 366,00 | 0,40 | |
| Val-TAC | 394 | 0,66 | 611,00 | 0,67 | |
| iMet-CAT | 195 | 0,33 | 276,00 | 0,30 | |
| RDN5 | 314 | 0,53 | 489 | 0,54 | |
| RDN58 | 300 | 0,50 | 419 | 0,46 | |
| oligo3 | 54 | 0,09 | 118 | 0,13 | |
| oligo5 | 6 | 0,01 | 4 | 0,44 | |
| antisense | 89 | 0,15 | 165 | 0,18 | |
| not-unique | 148,961 | - | 259,936 | - | |
| not mapped | 1,359,631 | - | 1,965,464 | - | |
| Total mapped tRNA | 58,645 | - | 89,671 | - | |

### tRNA abundances are accurately recapitulated using Nano-tRNAseq

Our results showed that Nano-tRNAseq, when used with optimized mapping settings and custom MinKNOW configuration, resulted in observed tRNA abundances highly similar to the expected values, using IVT tRNAs as input (ρ = 0.94) (Figure 5F). We then wondered whether Nano-tRNAseq observed abundances would correlate well with Illumina-based approaches. To this end, we compared S. cerevisiae tRNA abundances predicted using Nano-tRNAseq to those reported using three different Illumina-based methods: ARM-seq, Hydro-tRNAseq and mim-tRNAseq. In ARM-seq, tRNAs are pretreated with demethylating enzyme E. *coli* AlkB, which removes m1A, m3C, and a fraction of m1G modifications. Hydro-tRNAseq relies on partial alkaline RNA hydrolysis that generates fragments amenable for sequencing. In the case of mim-tRNAseq, the authors improved the efficiency of cDNA synthesis by optimizing TGIRT reverse transcription conditions and allowing for position-specific mismatch tolerance during read alignment.

Nano-tRNAseq correlated best with the Illumina-based methods that address the presence of RTtruncating modifications, namely ARM-seq (ρ=0.555) and mim-tRNAseq (ρ=0.525), and worst with Hydro-tRNAseq (ρ=0.182). The low correlation with Hydro-tRNAseq is probably due to the fact that (i) fragments which harbor such modifications are especially short and are less likely to be PCRamplified, and (ii) mapping fragmented samples is challenging and can lead to spurious tRNA counts. Overall, the generally low correlation of Illumina-based methods with Nano-tRNAseq is unsurprising given the substantial differences in library preparation and analysis. We should note that Illumina-based tRNA-sequencing methods also showed only modest correlations with each other (ρ=0.283-0.616). In any case, the advantages of Nano-pore sequencing compared to NGS-based approaches is that is cheaper faster and as it directly sequences the native RNA molecule, the need to remove modifications that perturb reverse transcription is circumvented. In addition, it does not require PCR amplification, which is known to introduce unwanted variation in the sequencing

### tRNA modification differences can be quantified using Nano-tRNAseq

Previous works have shown that basecalling errors, or mismatches to the reference, can be used to detect RNA modifications [Stephenson W, Razaghi R, Busan S, Weeks KM, Timp W, Smibert P. Direct detection of RNA modifications and structure using single molecule nanopore sequencing. doi:10.1101/2020.05.31.126763. Leger A, Amaral PP, Pandolfini L, Capitanchik C, Capraro F, Miano V, et al. RNA modifications detection by comparative Nanopore direct RNA sequencing. Nat Commun. 2021;12: 7198.Pratanwanich PN, Yao F, Chen Y, Koh CWQ, Wan YK, Hendra C, et al. Identification of differential RNA modifications from nanopore direct RNA sequencing with xPore. Nature Biotechnology. 2021. pp. 1394-1402. doi:10.1038/s41587-021-00949-w]. In agreement with these observations, biological S. *cerevisiae* tRNA^{Phe} showed considerably more mismatch errors than those seen in synthetic IVT tRNAs (Figure 3B). On closer inspection, the position of many of these mismatches coincided with specific RNA modifications, which can affect the signal with single base resolution, or can influence the signal of neighboring bases as well. These findings suggest that structure or short read length are not the major components causing errors in basecalling native tRNA reads, but it is rather the presence of RNA modifications.

On the basis of this premise, we aimed to validate that the mismatches we observe in native tRNAs were indeed the result of RNA modifications. To that end, we sequenced tRNAs from WT and a Pus4-deficient S. cerevisiae strain. Pus4 is an enzyme responsible for the synthesis of Ψ55 from U55 in the T-loop of tRNAs. Upon knockout of Pus4, we observed a striking loss of the characteristic U-to-C mismatch of Ψ at position 55 in tRNAs, while other known Ψ sites, which are not reported to be catalyzed by Pus4, were unaffected (Figure 7A,B, see also Table 13). Only Ψ55 of tRNATrp(CCA) remained unchanged in the absence of Pus4, however we noted that this tRNA does not contain the canonical Pus4 motif RRUUCNA, and therefore may not be targeted by this enzyme. Despite the loss of Ψ55 in Pus4-deficient S. cerevisiae, we observed only modest changes in tRNA isoacceptor expression (Figure 8A).

**Table 13. Sum of basecalling errors at known Ψ sites in WT and Pus4 KO S. cerevisiae tRNAs**

| | | | | **Sum of basecalling errors** | | | | **Pus4 KO sum of basecalling error relative to WT** | |
|---|---|---|---|---|---|---|---|---|---|
| **Refere nce** | **Ψ start** | **Ψ end** | **Position** | **Pus4 KO rep 1** | **WT rep 1** | **Pus4 KO rep 2** | **WT rep 2** | **Rep 1 differenc e** | **Rep 2 differe nce** |
| | **posit ion** | **posi tion** | | | | | | | |
| Ala-AGC | 61 | 62 | Ala-AGC:62 | 0,18 | 0,19 | 0,20 | 0,15 | 0,01 | -0,05 |
| Ala-AGC | 78 | 79 | Ala-AGC:79 | 0,05 | 0,90 | 0,06 | 0,90 | 0,85 | 0,85 |
| Arg-TCT | 49 | 50 | Arg-TCT:50 | 0,55 | 0,47 | 0,59 | 0,48 | -0,08 | -0,11 |
| Arg-TCT | 61 | 62 | Arg-TCT:62 | 0,41 | 0,46 | 0,42 | 0,45 | 0,05 | 0,03 |
| Arg-TCT | 77 | 78 | Arg-TCT:78 | 0,11 | 0,86 | 0,14 | 0,87 | 0,74 | 0,73 |
| Arg-ACG | 24 | 25 | Arg-ACG:25 | 0,41 | 0,38 | 0,42 | 0,35 | -0,03 | -0,07 |
| Arg-ACG | 50 | 51 | Arg-ACG:51 | 0,48 | 0,43 | 0,47 | 0,42 | -0,05 | -0,05 |
| Arg-ACG | 78 | 79 | Arg-ACG:79 | 0,14 | 0,57 | 0,16 | 0,58 | 0,43 | 0,42 |
| Asn-GTT | 63 | 64 | Asn-GTT:64 | 0,09 | 0,07 | 0,09 | 0,06 | -0,02 | -0,03 |
| Asn-GTT | 79 | 80 | Asn-GTT:80 | 0,03 | 0,51 | 0,03 | 0,54 | 0,48 | 0,51 |
| Asp-GTC | 36 | 37 | Asp-GTC:37 | 0,14 | 0,16 | 0,13 | 0,17 | 0,03 | 0,04 |
| Asp-GTC | 55 | 56 | Asp-GTC:56 | 0,71 | 0,68 | 0,71 | 0,69 | -0,03 | -0,02 |
| Asp-GTC | 77 | 78 | Asp-GTC:78 | 0,08 | 0,94 | 0,09 | 0,94 | 0,85 | 0,86 |
| Cys-GCA | 54 | 55 | Cys-GCA:55 | 0,22 | 0,26 | 0,22 | 0,30 | 0,04 | 0,08 |
| Cys-GCA | 61 | 62 | Cys-GCA:62 | 0,12 | 0,16 | 0,16 | 0,16 | 0,04 | 0,00 |
| Cys-GCA | 77 | 78 | Cys-GCA:78 | 0,11 | 0,52 | 0,11 | 0,53 | 0,41 | 0,42 |
| Glu-TTC | 36 | 37 | Glu-TTC:37 | 0,56 | 0,60 | 0,57 | 0,59 | 0,04 | 0,02 |
| Glu-TTC | 50 | 51 | Glu-TTC:51 | 0,07 | 0,07 | 0,08 | 0,07 | 0,00 | -0,01 |
| Glu-TTC | 77 | 78 | Glu-TTC:78 | 0,02 | 0,92 | 0,02 | 0,93 | 0,91 | 0,91 |
| Gly-GCC | 36 | 37 | Gly-GCC:37 | 0,42 | 0,41 | 0,39 | 0,39 | -0,01 | -0,01 |
| Gly-GCC | 54 | 55 | Gly-GCC:55 | 0,66 | 0,54 | 0,68 | 0,54 | -0,13 | -0,14 |
| Gly-GCC | 60 | 61 | Gly-GCC:61 | 0,26 | 0,28 | 0,24 | 0,28 | 0,02 | 0,04 |
| Gly-GCC | 76 | 77 | Gly-GCC:77 | 0,03 | 0,72 | 0,03 | 0,71 | 0,69 | 0,68 |
| Gly-TCC | 36 | 37 | Gly-TCC:37 | 0,57 | 0,62 | 0,57 | 0,66 | 0,05 | 0,09 |
| Gly-TCC | 77 | 78 | Gly-TCC:78 | 0,01 | 0,75 | 0,01 | 0,78 | 0,73 | 0,77 |
| His-GTG | 36 | 37 | His-GTG:37 | 0,06 | 0,09 | 0,07 | 0,07 | 0,03 | 0,00 |
| His-GTG | 55 | 56 | His-GTG:56 | 0,37 | 0,30 | 0,35 | 0,27 | -0,07 | -0,08 |
| His-GTG | 62 | 63 | His-GTG:63 | 0,74 | 0,76 | 0,74 | 0,77 | 0,03 | 0,03 |
| His-GTG | 77 | 78 | His-GTG:78 | 0,04 | 0,89 | 0,04 | 0,87 | 0,85 | 0,83 |
| IIe-AAT | 79 | 80 | Ile-AAT:80 | 0,10 | 0,63 | 0,10 | 0,64 | 0,53 | 0,53 |
| Ile-TAT | 50 | 51 | Ile-TAT:51 | 0,52 | 0,45 | 0,56 | 0,37 | -0,06 | -0,20 |
| Ile-TAT | 57 | 58 | Ile-TAT:58 | 0,12 | 0,09 | 0,11 | 0,11 | -0,02 | 0,00 |
| Ile-TAT | 59 | 60 | Ile-TAT:60 | 0,11 | 0,11 | 0,15 | 0,11 | 0,00 | -0,04 |
| Ile-TAT | 78 | 79 | Ile-TAT:79 | 0,29 | 0,56 | 0,32 | 0,60 | 0,27 | 0,28 |
| Ile-TAT | 90 | 91 | Ile-TAT:91 | 0,20 | 0,13 | 0,17 | 0,14 | -0,07 | -0,03 |
| Leu-TAG | 51 | 52 | Leu-TAG:52 | 0,74 | 0,73 | 0,78 | 0,76 | -0,01 | -0,03 |
| Leu-TAG | 56 | 57 | Leu-TAG:57 | 0,43 | 0,40 | 0,41 | 0,45 | -0,03 | 0,04 |
| Leu-TAG | 63 | 64 | Leu-TAG:64 | 0,37 | 0,28 | 0,39 | 0,27 | -0,08 | -0,11 |
| Leu-TAG | 87 | 88 | Leu-TAG:88 | 0,07 | 0,74 | 0,07 | 0,74 | 0,67 | 0,66 |
| Leu-CAA | 56 | 57 | Leu-CAA:57 | 0,60 | 0,59 | 0,59 | 0,59 | -0,02 | 0,00 |
| Leu-CAA | 63 | 64 | Leu-CAA:64 | 0,22 | 0,29 | 0,26 | 0,28 | 0,07 | 0,03 |
| Leu-CAA | 87 | 88 | Leu-CAA:88 | 0,02 | 0,89 | 0,02 | 0,90 | 0,87 | 0,88 |
| Leu-TAA | 63 | 64 | Leu-TAA:64 | 0,36 | 0,48 | 0,38 | 0,47 | 0,12 | 0,09 |
| Leu-TAA | 89 | 90 | Leu-TAA:90 | 0,07 | 0,91 | 0,07 | 0,91 | 0,84 | 0,84 |
| Lys-CTT | 50 | 51 | Lys-CTT:51 | 0,30 | 0,30 | 0,30 | 0,26 | 0,00 | -0,04 |
| Lys-CTT | 62 | 63 | Lys-CTT:63 | 0,15 | 0,14 | 0,15 | 0,11 | -0,01 | -0,04 |
| Lys-CTT | 78 | 79 | Lys-CTT:79 | 0,05 | 0,75 | 0,04 | 0,74 | 0,70 | 0,70 |
| Met-CAT | 50 | 51 | Met-CAT:51 | 0,46 | 0,40 | 0,51 | 0,38 | -0,07 | -0,14 |
| Met-CAT | 54 | 55 | Met-CAT:55 | 0,20 | 0,18 | 0,17 | 0,17 | -0,02 | 0,00 |
| Met-CAT | 62 | 63 | Met-CAT:63 | 0,10 | 0,08 | 0,09 | 0,06 | -0,02 | -0,02 |
| Met-CAT | 78 | 79 | Met-CAT:79 | 0,17 | 0,56 | 0,16 | 0,59 | 0,39 | 0,44 |
| Phe-GAA | 62 | 63 | Phe-GAA:63 | 0,41 | 0,46 | 0,37 | 0,40 | 0,05 | 0,03 |
| Phe-GAA | 78 | 79 | Phe-GAA:79 | 0,02 | 0,66 | 0,03 | 0,68 | 0,64 | 0,65 |
| Pro-TGG | 36 | 37 | Pro-TGG:37 | 0,50 | 0,50 | 0,50 | 0,48 | 0,00 | -0,02 |
| Pro-TGG | 54 | 55 | Pro-TGG:55 | 0,63 | 0,64 | 0,62 | 0,64 | 0,02 | 0,02 |
| Pro-TGG | 60 | 61 | Pro-TGG:61 | 0,10 | 0,13 | 0,11 | 0,11 | 0,03 | 0,00 |
| Pro-TGG | 77 | 78 | Pro-TGG:78 | 0,22 | 0,75 | 0,24 | 0,77 | 0,53 | 0,53 |
| Ser-CGA | 62 | 63 | Ser-CGA:63 | 0,15 | 0,15 | 0,16 | 0,14 | 0,00 | -0,02 |
| Ser-CGA | 87 | 88 | Ser-CGA:88 | 0,12 | 0,68 | 0,19 | 0,70 | 0,56 | 0,51 |
| Ser-TGA | 62 | 63 | Ser-TGA:63 | 0,07 | 0,16 | 0,09 | 0,10 | 0,09 | 0,02 |
| Ser-TGA | 87 | 88 | Ser-TGA:88 | 0,03 | 0,78 | 0,01 | 0,76 | 0,75 | 0,75 |
| Ser-AGA | 55 | 56 | Ser-AGA:56 | 0,14 | 0,16 | 0,14 | 0,21 | 0,02 | 0,07 |
| Ser-AGA | 62 | 63 | Ser-AGA:63 | 0,01 | 0,02 | 0,01 | 0,02 | 0,00 | 0,00 |
| Ser-AGA | 87 | 88 | Ser-AGA:88 | 0,05 | 0,85 | 0,05 | 0,86 | 0,81 | 0,81 |
| Ser-GCT | 87 | 88 | Ser-GCT:88 | 0,09 | 0,69 | 0,10 | 0,67 | 0,59 | 0,58 |
| Thr-AGT | 62 | 63 | Thr-AGT:63 | 0,16 | 0,15 | 0,17 | 0,15 | -0,02 | -0,02 |
| Thr-AGT | 78 | 79 | Thr-AGT:79 | 0,15 | 0,56 | 0,13 | 0,53 | 0,40 | 0,40 |
| Trp-CCA | 48 | 49 | Trp-CCA:49 | 0,52 | 0,49 | 0,54 | 0,36 | -0,02 | -0,18 |
| Trp-CCA | 49 | 50 | Trp-CCA:50 | 0,53 | 0,51 | 0,52 | 0,47 | -0,02 | -0,05 |
| Trp-CCA | 50 | 51 | Trp-CCA:51 | 0,65 | 0,55 | 0,67 | 0,43 | -0,10 | -0,23 |
| Trp-CCA | 61 | 62 | Trp-CCA:62 | 0,04 | 0,04 | 0,03 | 0,02 | 0,00 | -0,01 |
| Trp-CCA | 77 | 78 | Trp-CCA:78 | 0,23 | 0,65 | 0,20 | 0,60 | 0,43 | 0,40 |
| Trp-CCA | 87 | 88 | Trp-CCA:88 | 0,63 | 0,46 | 0,67 | 0,36 | -0,16 | -0,31 |
| Tyr-GTA | 60 | 61 | Tyr-GTA:61 | 0,32 | 0,36 | 0,29 | 0,36 | 0,03 | 0,07 |
| Tyr-GTA | 64 | 65 | Tyr-GTA:65 | 0,05 | 0,05 | 0,05 | 0,05 | 0,00 | 0,00 |
| Tyr-GTA | 80 | 81 | Tyr-GTA:81 | 0,10 | 0,63 | 0,09 | 0,65 | 0,53 | 0,56 |
| Val-TAC | 51 | 52 | Val-TAC:52 | 0,21 | 0,20 | 0,19 | 0,18 | -0,01 | 0,00 |
| Val-TAC | 56 | 57 | Val-TAC:57 | 0,42 | 0,38 | 0,43 | 0,36 | -0,04 | -0,06 |
| Val-TAC | 79 | 80 | Val-TAC:80 | 0,22 | 0,75 | 0,19 | 0,72 | 0,52 | 0,53 |
| Val-CAC | 36 | 37 | Val-CAC:37 | 0,73 | 0,73 | 0,76 | 0,72 | -0,01 | -0,04 |
| Val-CAC | 50 | 51 | Val-CAC:51 | 0,32 | 0,23 | 0,33 | 0,20 | -0,09 | -0,12 |
| Val-CAC | 51 | 52 | Val-CAC:52 | 0,44 | 0,36 | 0,38 | 0,34 | -0,09 | -0,04 |
| Val-CAC | 55 | 56 | Val-CAC:56 | 0,07 | 0,07 | 0,07 | 0,08 | 0,00 | 0,01 |
| Val-CAC | 78 | 79 | Val-CAC:79 | 0,09 | 0,69 | 0,11 | 0,69 | 0,60 | 0,59 |
| Val-AAC | 36 | 37 | Val-AAC:37 | 0,53 | 0,58 | 0,53 | 0,52 | 0,05 | -0,01 |
| Val-AAC | 51 | 52 | Val-AAC:52 | 0,20 | 0,17 | 0,23 | 0,15 | -0,03 | -0,08 |
| Val-AAC | 56 | 57 | Val-AAC:57 | 0,34 | 0,29 | 0,33 | 0,27 | -0,06 | -0,07 |
| Val-AAC | 79 | 80 | Val-AAC:80 | 0,14 | 0,57 | 0,12 | 0,58 | 0,43 | 0,46 |
| | | | **Read ID** | **8_NanotR NAseq_p us4KOye ast_rep1** | **7_NanotR NAseq_W Tyeast_re p1** | **12_Nanot RNAseq_ pus4KOy east_rep2** | **11_Nanot RNAseq_ WTyeast_ rep2** | | |

### Nano-tRNAseq identifies tRNA modification interdependencies

tRNA modifications are introduced in a defined sequential order, and the chronology is controlled by the cross-talk between modification events and RNA modifying enzymes. Using time-resolved nuclear magnetic resonance (NMR) monitoring of tRNA maturation, Barraud et al., 2019 reported a robust modification hierarchy in the T-loop of S. cerevisiae tRNAPhe, with Ψ55 positively influencing the introduction of both m5U54 and m¹A58, and m⁵U54 positively influencing the introduction of m¹A58. To explore whether our method could capture the effect of Ψ55 loss on other modifications, we plotted the summed basecalling error (base mismatch, insertion and deletion) for each nucleotide position and tRNA molecule reference, for each Pus4 knockout tRNA isoacceptor, relative to WT (Figure 7C, see also Figure 8B). In addition to the decrease in mismatch frequency at position 55, we observed a decrease at position 54 and 57-59 (Figure 7C-E). LC-MS/MS was used to confirm that this observation is due to a bona fide reduction in m⁵U54 and m¹A58 modifications (Figure 7E). Moreover, IGV tracks show that the Ψ mismatch error is typically restricted to a single base (Figure 7A), and the m¹A and m⁵U mismatch error signal spills over to neighboring bases. Although Nano-tRNAseq does not allow us to dissect the specific order of a modification circuit, it can reveal RNA modification interdependencies at distinct tRNA sites in the same transcript and quantify site-specific changes in RNA modifications across tRNA isoacceptors in a high-throughput manner with higher sensitivity than orthogonal methods, with the concurrent benefit of measuring tRNA abundance, confirmed that Nano-tRNAseq was able recapitulate the known relationship between loss of Ψ55, which is catalyzed by Pus4, and the subsequent loss of m¹A58 and m⁵U54 (Figure 7C-D), With the generation of yeast knockout strains of every tRNA modifying enzyme nearly complete, the method of the invention presents an excellent opportunity to describe tRNA modification circuits in a holistic manner, providing invaluable insights into how these processes are regulated and impact health and disease.

### Nano-tRNAseq reveals tRNA deadenylation upon oxidative stress, but not heat stress

tRNA modifying enzymes are dysregulated in a multitude of human diseases, and are subject to change under certain cellular conditions. Indeed, previous studies provide evidence that tRNA modification profiles are re-programmed under stressful conditions, such as elevated temperature and oxidative stress. However, chromatography-coupled mass spectrometry-based methods do not provide information about from which tRNA isoacceptor the modification originates, nor the sequence context. To examine the changes that occur in tRNA abundances and modifications at single nucleotide and single tRNA isoacceptor resolution, we sequenced tRNAs from WT S. *cerevisiae* that were exposed to heat stress or oxidative stress (see Methods). While we found that the tRNA abundances across biological replicates were highly replicable (ρ=0.981-0.993) (Figure 3C for WT, Figure 9A for heat and oxidative stress, see also Table 14), we observed only modest differences in the expression of tRNAs under heat or oxidative stress, compared to WT (Figure 9A, see also Table 15 and Table 16). Moreover, when we plotted the summed basecalling error frequency for each nucleotide position and tRNA isoacceptor, relative to WT, we observed no discernable changes in RNA modification profiles in any of the conditions tested Figure 9B. This finding was corroborated by our LC-MS/MS results (Figure 7E). By contrast, we observed a strong increase in basecalling error frequency of the last nucleotide, position 76 (Figure 9B), which corresponds to the terminal A of the CCA tail (Figure 9C). IGV tracks showed that the terminal A had reduced coverage relative to its neighboring bases (Figure 9D), which is indicative of a deletion. We then calculated the deletion frequency of the terminal A for each tRNA isoacceptor and found that the deletion frequency in tRNAs subjected to oxidative stress was significantly higher compared to WT, Pus4 KO and heat stress, suggesting that deadenylation of the terminal A is an oxidative stress specific phenomenon (Figure 9E). This result is consistent with a previous study which also found that the terminal A of the 3' CCA tail is rapidly removed during oxidative stress, which is reversible and quickly repairable, thus making the tRNAs chargeable again, representing a rapid mechanism of suppressing and reactivating translation at a low metabolic cost. The method of the invention is able to demonstrate this rapid and dynamic repression of translation via tRNA deacylation, by quantifying the level of terminal A deadenylation, with tRNA isoacceptor resolution.

**Table 14. Read counts per tRNA isoacceptor for WT, stressed and Pus4 KO S. cerevisiae tRNAs. Reads (#)**

| **Referenc e** | **WT rep 1** | **Pus4 KO rep 1** | **Heat stress rep 1** | **Oxidativ e stress rep 1** | **WT rep 2** | **Pus4 KO rep 2** | **Heat stress rep 2** | **Oxidativ e stress rep 2** |
|---|---|---|---|---|---|---|---|---|
| Ala-AGC | 16,567 | 8,335 | 16,107 | 20,619 | 12,195 | 11,304 | 18,043 | 8,780 |
| Ala-TGC | 8,521 | 4,212 | 7,511 | 10,176 | 4,665 | 3,089 | 8,033 | 4,017 |
| Arg-ACG | 38,993 | 12,776 | 28,405 | 24,776 | 14,877 | 13,428 | 29,806 | 10,600 |
| Arg-CCG | 7,692 | 3,685 | 6,109 | 8,388 | 5,560 | 3,116 | 8,075 | 3,045 |
| Arg-CCT | 2,679 | 1,237 | 3,031 | 3,702 | 1,390 | 1,791 | 2,992 | 1,528 |
| Arg-TCT | 12,487 | 7,585 | 11,677 | 13,700 | 7,536 | 5,307 | 12,721 | 4,824 |
| Asn-GTT | 4,711 | 5,097 | 5,013 | 7,325 | 4,031 | 2,940 | 5,830 | 3,364 |
| Asp-GTC | 96,009 | 39,684 | 66,346 | 90,559 | 57,467 | 46,213 | 92,123 | 43,090 |
| Cys-GCA | 24,314 | 12,692 | 19,080 | 18,259 | 16,136 | 9,462 | 21,215 | 6,155 |
| Gln-CTG | 56,227 | 46,226 | 43,394 | 37,235 | 38,127 | 32,320 | 52,070 | 13,781 |
| Gln-TTG | 25,080 | 17,151 | 12,999 | 16,605 | 19,879 | 13,472 | 17,773 | 6,385 |
| Glu-CTC | 3,620 | 2,704 | 3,186 | 4,761 | 2,674 | 1,752 | 4,520 | 1,950 |
| Glu-TTC | 17,525 | 13,412 | 18,086 | 26,277 | 13,886 | 8,816 | 26,688 | 11,645 |
| Gly-CCC | 4,537 | 2,025 | 3,162 | 3,973 | 3,260 | 1,870 | 4,382 | 1,699 |
| Gly-GCC | 79,417 | 45,882 | 61,046 | 79,432 | 61,414 | 52,269 | 94,468 | 38,043 |
| Gly-TCC | 11,049 | 5,060 | 8,024 | 5,007 | 7,748 | 3,976 | 11,308 | 1,848 |
| His-GTG | 17,820 | 8,825 | 16,409 | 18,260 | 11,136 | 6,107 | 15,960 | 7,191 |
| Ile-AAT | 7,239 | 7,236 | 8,473 | 12,468 | 4,179 | 7,533 | 8,383 | 5,451 |
| Ile-TAT | 1,147 | 696 | 1,291 | 1,762 | 623 | 650 | 1,172 | 527 |
| Leu-CAA | 66,410 | 34,251 | 46,376 | 56,526 | 38,526 | 19,954 | 48,296 | 23,796 |
| Leu-GAG | 2,385 | 1,302 | 2,119 | 2,612 | 1,973 | 802 | 2,508 | 1,118 |
| Leu-TAA | 11,300 | 4,498 | 8,728 | 8,895 | 7,976 | 3,230 | 9,022 | 3,017 |
| Leu-TAG | 8,365 | 8,921 | 7,425 | 7,856 | 5,617 | 5,899 | 7,146 | 2,901 |
| Lys-CTT | 5,947 | 4,567 | 6,324 | 10,883 | 3,681 | 3,145 | 5,674 | 3,927 |
| Lys-TTT | 6,540 | 4,803 | 5,685 | 8,066 | 3,520 | 4,801 | 5,658 | 3,540 |
| Met-CAT | 2,048 | 1,000 | 1,802 | 2,098 | 1,616 | 556 | 1,750 | 807 |
| Phe-GAA | 9,097 | 5,269 | 8,113 | 11,186 | 4,819 | 3,761 | 6,523 | 4,308 |
| Pro-AGG | 5,597 | 1,620 | 4,857 | 6,179 | 4,618 | 1,461 | 6,942 | 2,131 |
| Pro-TGG | 31,654 | 24,813 | 31,560 | 24,708 | 19,705 | 21,416 | 34,524 | 7,882 |
| Ser-AGA | 32,185 | 14,017 | 24,495 | 26,505 | 20,581 | 9,506 | 30,873 | 11,355 |
| Ser-CGA | 185 | 106 | 191 | 253 | 205 | 47 | 193 | 134 |
| Ser-GCT | 8,571 | 8,229 | 7,593 | 9,784 | 12,793 | 4,806 | 10,513 | 5,342 |
| Ser-TGA | 356 | 246 | 352 | 527 | 457 | 126 | 454 | 252 |
| Thr-AGT | 4,517 | 3,173 | 5,462 | 6,314 | 2,491 | 2,057 | 4,647 | 2,441 |
| Thr-CGT | 730 | 362 | 1,041 | 917 | 479 | 346 | 764 | 362 |
| Thr-TGT | 1,328 | 952 | 1,318 | 1,815 | 1,156 | 556 | 1,403 | 759 |
| Trp-CCA | 5,988 | 3,956 | 4,498 | 5,144 | 4,493 | 2,482 | 4,975 | 1,817 |
| Tyr-GTA | 8,017 | 5,767 | 7,120 | 10,444 | 4,395 | 4,247 | 7,575 | 4,159 |
| Val-AAC | 18,565 | 10,123 | 16,297 | 23,019 | 12,904 | 8,391 | 18,173 | 8,920 |
| Val-CAC | 3,520 | 1,613 | 2,926 | 3,249 | 2,828 | 962 | 3,371 | 1,219 |
| Val-TAC | 3,798 | 3,382 | 2,874 | 5,266 | 2,228 | 2,803 | 2,929 | 2,092 |
| iMet-CAT | 2,014 | 1,107 | 2,556 | 2,884 | 881 | 1,796 | 1,929 | 1,427 |
| RDN5 | 106,30 5 | 34,857 | 90,799 | 59,097 | 83,728 | 47,583 | 126,817 | 31,327 |
| RDN58 | 5,378 | 5,540 | 9,553 | 12,409 | 14,318 | 2,151 | 10,609 | 5,100 |
| oligo3 | 16 | 6 | 9 | 9 | 1 | 7 | 8 | 1 |
| oligo5 | 18 | 5 | 6 | 4 | 6 | 3 | 8 | 0 |
| antisense | 611 | 270 | 571 | 548 | 389 | 179 | 632 | 174 |
| not-unique | 212,22 3 | 106,94 5 | 179,481 | 185,103 | 168,658 | 84,681 | 228,815 | 76,781 |
| * | 702,93 8 | 465,77 3 | 657,522 | 696,320 | 450,468 | 321,545 | 697,772 | 271,237 |
| **Read ID** | **7_Nano tRNAse q_WTy east_re p1** | **8_Nano tRNAse q_pus4 KOyea st_rep1** | **9_Nanot RNAseq _Heatstr essyeast _rep1** | **10_Nanot RNAseq_ Oxidative stressye ast_rep1** | **11_Nan otRNAs eq_WTy east_rep 2** | **12_Nanot RNAseq_ pus4KOy east_rep2** | **13_Nanot RNAseq_ Heatstres syeast_re p2** | **14_Nanot RNAseq_ Oxidative stressye ast_rep2** |

**Table 15. Differential expression analysis of oxidative stress S. cerevisiae tRNAs.**

| **Reference** | **base Mean** | **log2FoldChange** | **IfcSE** | **stat** | **pvalue** | **padj** |
|---|---|---|---|---|---|---|
| Gly-TCC | 5616,21 | -1,434 | 0,154 | -9,334 | 1,02E-20 | 3,88E-19 |
| Gln-CTG | 31804,63 | -0,861 | 0,143 | -6,035 | 1,59E-09 | 2,01E-08 |
| Gln-TTG | 15051,90 | -0,952 | 0,157 | -6,069 | 1,29E-09 | 2,01E-08 |
| IIe-AAT | 6551,56 | 0,748 | 0,163 | 4,588 | 4,48E-06 | 4,26E-05 |
| Cys-GCA | 14077,88 | -0,727 | 0,161 | -4,500 | 6,80E-06 | 5,17E-05 |
| Leu-TAA | 6797,09 | -0,699 | 0,165 | -4,227 | 2,37E-05 | 0,000 |
| Lys-CTT | 5335,85 | 0,652 | 0,161 | 4,051 | 5,10E-05 | 0,000 |
| Pro-TGG | 18075,01 | -0,661 | 0,175 | -3,780 | 0,000 | 0,001 |
| Trp-CCA | 3827,08 | -0,591 | 0,170 | -3,486 | 0,000 | 0,002 |
| iMet-CAT | 1619,06 | 0,745 | 0,251 | 2,967 | 0,003 | 0,011 |
| Ser-AGA | 20047,35 | -0,401 | 0,141 | -2,837 | 0,005 | 0,016 |
| Val-CAC | 2394,63 | -0,499 | 0,181 | -2,761 | 0,006 | 0,018 |
| Gly-CCC | 2998,54 | -0,398 | 0,160 | -2,496 | 0,013 | 0,037 |
| Arg-CCT | 2046,70 | 0,456 | 0,199 | 2,293 | 0,022 | 0,055 |
| Leu-TAG | 5416,66 | -0,351 | 0,153 | -2,297 | 0,022 | 0,055 |
| Thr-AGT | 3449,26 | 0,388 | 0,178 | 2,181 | 0,029 | 0,069 |
| Glu-TTC | 15649,49 | 0,330 | 0,159 | 2,084 | 0,037 | 0,078 |
| Val-TAC | 2944,75 | 0,355 | 0,169 | 2,101 | 0,036 | 0,078 |
| Leu-CAA | 40739,23 | -0,300 | 0,152 | -1,967 | 0,049 | 0,098 |
| Asn-GTT | 4419,74 | 0,349 | 0,185 | 1,887 | 0,059 | 0,113 |
| Arg-ACG | 19183,75 | -0,462 | 0,248 | -1,861 | 0,063 | 0,114 |
| Tyr-GTA | 5926,09 | 0,311 | 0,171 | 1,815 | 0,070 | 0,120 |
| Lys-TTT | 4802,00 | 0,314 | 0,179 | 1,752 | 0,080 | 0,132 |
| Pro-AGG | 4076,39 | -0,319 | 0,185 | -1,720 | 0,086 | 0,135 |
| Met-CAT | 1456,45 | -0,317 | 0,190 | -1,668 | 0,095 | 0,145 |
| Arg-CCG | 5419,33 | -0,201 | 0,158 | -1,275 | 0,202 | 0,290 |
| Phe-GAA | 6413,13 | 0,226 | 0,179 | 1,264 | 0,206 | 0,290 |
| Gly-GCC | 58467,12 | -0,176 | 0,162 | -1,084 | 0,279 | 0,378 |
| Ala-TGC | 5992,69 | 0,180 | 0,172 | 1,049 | 0,294 | 0,385 |
| Leu-GAG | 1816,94 | -0,182 | 0,190 | -0,958 | 0,338 | 0,428 |
| His-GTG | 11952,18 | -0,130 | 0,145 | -0,900 | 0,368 | 0,451 |
| Glu-CTC | 2896,73 | 0,137 | 0,161 | 0,850 | 0,395 | 0,469 |
| Ser-GCT | 8644,11 | -0,461 | 0,581 | -0,793 | 0,428 | 0,493 |
| Ala-AGC | 13003,32 | 0,088 | 0,145 | 0,607 | 0,544 | 0,608 |
| Arg-TCT | 8363,27 | -0,085 | 0,163 | -0,519 | 0,604 | 0,650 |
| Asp-GTC | 64165,56 | -0,080 | 0,160 | -0,501 | 0,616 | 0,650 |
| Val-AAC | 13994,44 | 0,058 | 0,141 | 0,409 | 0,683 | 0,701 |
| Thr-TGT | 1137,68 | 0,080 | 0,214 | 0,373 | 0,709 | 0,709 |
| Ile-TAT | 862,32 | 0,368 | 0,252 | 1,461 | 0,144 | NA |
| Ser-CGA | 181,37 | 0,055 | 0,415 | 0,132 | 0,895 | NA |
| Ser-TGA | 369,93 | -0,045 | 0,364 | -0,124 | 0,901 | NA |
| Thr-CGT | 548,91 | 0,131 | 0,231 | 0,569 | 0,569 | NA |

Differentially expressed tRNAs were inferred using DESeq2. Base mean (the average normalized counts taken over all samples), log2FoldChange (log2 fold change between groups), IfcSE (standard error of log2FoldChange), stat (Walk Statistic), pvalue (Wald test p-value), padj (Benjamini-Hochberg adjusted p-value).

**Table 16. Differential expression analysis of heat stress S. cerevisiae tRNAs.**

| **Reference** | **base Mean** | **log2FoldChange** | **IfcSE** | **stat** | **pvalue** | **padj** |
|---|---|---|---|---|---|---|
| Gln-TTG | 15867,91 | -0,739 | 0,190 | -3,893 | 9.91E-05 | 4,16E-03 |
| IIe-AAT | 5728,49 | 0,426 | 0,182 | 2,338 | 1,94E-02 | 0,407 |
| Arg-CCT | 2036,02 | 0,443 | 0,216 | 2,055 | 3,98E-02 | 0,440 |
| Thr-AGT | 3471,19 | 0,404 | 0,217 | 1,860 | 6,29E-02 | 0,440 |
| Trp-CCA | 4141,22 | -0,322 | 0,170 | -1,897 | 0,058 | 0,440 |
| iMet-CAT | 1484,22 | 0,541 | 0,290 | 1,866 | 0,062 | 0,440 |
| Leu-CAA | 40896,47 | -0,287 | 0,169 | -1,703 | 0,089 | 0,465 |
| Leu-TAA | 7661,12 | -0,284 | 0,162 | -1,761 | 0,078 | 0,465 |
| Glu-TTC | 15565,49 | 0,316 | 0,204 | 1,548 | 0,122 | 0,534 |
| Thr-CGT | 616,40 | 0,435 | 0,285 | 1,526 | 0,127 | 0,534 |
| Ile-TAT | 857,72 | 0,351 | 0,244 | 1,435 | 0,151 | 0,577 |
| Pro-TGG | 23915,53 | 0,213 | 0,157 | 1,361 | 0,173 | 0,607 |
| Cys-GCA | 16602,36 | -0,165 | 0,153 | -1,081 | 0,280 | 0,745 |
| Gly-CCC | 3161,52 | -0,226 | 0,195 | -1,160 | 0,246 | 0,745 |
| Met-CAT | 1500,70 | -0,223 | 0,206 | -1,083 | 0,279 | 0,745 |
| Val-CAC | 2624,86 | -0,199 | 0,188 | -1,058 | 0,290 | 0,745 |
| Val-TAC | 2421,79 | -0,194 | 0,188 | -1,033 | 0,302 | 0,745 |
| Gln-CTG | 39018,12 | -0,149 | 0,155 | -0,960 | 0,337 | 0,756 |
| Lys-CTT | 4418,44 | 0,175 | 0,191 | 0,916 | 0,360 | 0,756 |
| Ser-GCT | 8502,90 | -0,518 | 0,565 | -0,917 | 0,359 | 0,756 |
| Ala-TGC | 5831,25 | 0,105 | 0,186 | 0,567 | 0,571 | 0,787 |
| Arg-TCT | 9046,22 | 0,139 | 0,165 | 0,845 | 0,398 | 0,787 |
| Asn-GTT | 4047,43 | 0,114 | 0,194 | 0,590 | 0,555 | 0,787 |
| Asp-GTC | 63573,23 | -0,108 | 0,186 | -0,578 | 0,564 | 0,787 |
| Glu-CTC | 2867,00 | 0,109 | 0,203 | 0,535 | 0,593 | 0,787 |
| Gly-TCC | 7831,62 | -0,136 | 0,189 | -0,719 | 0,472 | 0,787 |
| Leu-GAG | 1863,11 | -0,106 | 0,201 | -0,525 | 0,600 | 0,787 |
| Leu-TAG | 5877,74 | -0,096 | 0,172 | -0,560 | 0,575 | 0,787 |
| Ser-AGA | 22126,21 | -0,090 | 0,165 | -0,546 | 0,585 | 0,787 |
| Ser-CGA | 163,68 | -0,246 | 0,419 | -0,587 | 0,557 | 0,787 |
| Ser-TGA | 343,86 | -0,266 | 0,375 | -0,709 | 0,479 | 0,787 |
| Tyr-GTA | 5502,01 | 0,111 | 0,186 | 0,599 | 0,549 | 0,787 |
| Ala-AGC | 12934,42 | 0,073 | 0,159 | 0,463 | 0,644 | 0,812 |
| Arg-CCG | 5641,44 | -0,080 | 0,180 | -0,444 | 0,657 | 0,812 |
| Gly-GCC | 60957,61 | -0,050 | 0,210 | -0,239 | 0,811 | 0,908 |
| Lys-TTT | 4349,28 | 0,046 | 0,196 | 0,234 | 0,815 | 0,908 |
| Thr-TGT | 1082,37 | -0,063 | 0,226 | -0,280 | 0,779 | 0,908 |
| Val-AAC | 13552,44 | -0,035 | 0,154 | -0,226 | 0,821 | 0,908 |
| Phe-GAA | 5822,39 | -0,044 | 0,228 | -0,194 | 0,846 | 0,911 |
| Arg-ACG | 22436,26 | 0,027 | 0,263 | 0,103 | 0,918 | 0,955 |
| His-GTG | 12555,20 | 0,014 | 0,169 | 0,085 | 0,932 | 0,955 |
| Pro-AGG | 4540,22 | 0,009 | 0,210 | 0,042 | 0,967 | 0,967 |

Differentially expressed tRNAs were inferred using DESeq2. Base mean (the average normalized counts taken over all samples), log2FoldChange (log2 fold change between groups), IfcSE (standard error of log2FoldChange), stat (Walk Statistic), pvalue (Wald test p-value), padj (Benjamini-Hochberg adjusted p-value).

### Custom MinKNOW configuration capture shorter and longer molecules

The default MinKNOW configuration is suboptimal in detecting reads originating from short molecules such as tRNA. We developed an alternative MinKNOW configuration to capture about 10x more reads from tRNA experiments. This is achieved by reporting as reads molecules that are classified as adapter or strand (See methods).

### Demultiplexing tRNA reads sequenced using nanopore direct RNA sequencing

The method of the invention is able to produce millions of tRNA reads from a single sequencing reaction. The library preparation and sequencing is expensive. And for downstream analyses, having tens-to-hundreds of thousands of reads would be sufficient. Therefore, the availability of a multiplexing method allowing sequencing of multiple samples in a single reaction would be desirable for tRNA sequencing.

The method to demultiplex tRNA reads consists of the following steps:
1. Identification of "barcode" region via segmentation of the read, based on the alignment information
2. Basecalling of the DNA barcode region
3. Assignment of barcode based on alignment of the basecalled DNA barcode regions to reference set of DNA barcodes

### Step 1: Identification of the barcode (segmentation of the read based on alignment information)

In contrast to previous algorithms (e.g. DeePlexiCon), the segmentation step does not rely on the presence of polyA signals in the read. Instead, here we define the end of the barcode as the signal corresponding to the last aligned base of the read.

To know the location of the last aligned base of the read location in signal space, the read has to be first basecalled and aligned, and precise read start in the signal space can then be identified by checking which is the last aligned base reported by the basecaller. This information is extracted from the "move" table, that is provided by the basecaller.

### Step 2: DNA Barcode basecalling using Guppy

Next, we developed a custom function for basecalling DNA adapters from direct RNA sequencing libraries. We should note that RNA reads (i.e. those arising from direct RNA sequencing) are typically basecalled with RNA basecalling models, and therefore, they remove the DNA adapter and barcode regions, which cannot be basecalled under an "RNA basecalling model". Therefore, we used a Bonito-trained DNA model to basecall the DNA adapter regions of the reads sequenced using direct RNA sequencing. The basecalling itself was performed using guppy, but using as DNA basecalling model a pre-trained DNA model that was trained in-house using Bonito (see below for details on training the DNA model).

### Step 3: Barcode assignment via sequence alignment

Basecalled barcode sequences are mapped onto barcode reference sequences using minimap2 with following parameters: -xmap-ont -k6 -w3 -n1 -m10 -s13 -A1 -B1 -O1 -E1. Only best, unique (map quality above 0) alignments are reported. Only barcodes that were aligned uniquely to barcode reference were kept as "true". Barcodes that are mapped not uniquely are reported as unknown (bc_0).

In a particular embodiment a software to perform steps 1,2 and 3 iSn one sole program can be used. Such all-in-one design performs much faster than performing all operations sequentially. For example, 4K reads can be basecalled, aligned and demultiplexed in 2-5seconds using 1CPU and 1GPU. To perform the same operation sequentially, it is necessary to store the basecalled FAST5 reads, which occupy a lot of space and are not actually needed for any additional steps once the demultiplexing has been accomplished. Briefly, the sequential steps performed by this software are:
- i) tRNA basecalling using guppy_basecall via pyguppy client (part of step 1 above)
- ii) tRNA sequence alignment to reference using minimap2 via mappy (part of step1 above)
- iii) barcode identification in the signal space (part of step1 above)
- iv) barcode basecalling via our custom barcode basecalling model (CTC-CRF model trained from bonito) (step 2 above)
- v) barcode classification via barcode sequence alignment to barcode references using minimap2 via mappy (step 3 above)

### Evaluating the performance of the tRNA demultiplexing algorithm

We tested 7 independent Nano-tRNAseq runs, where each human tRNA sample was sequenced with an individual barcode. The method reached 0.979 accuracy and 0.996 recovery, overall. The bc_82 was performing the worst (accuracy of 0.958). Per barcode statistics can be seen in the Table 17 below.

**Table 17: Oligos used in the multiplexing experiment**

| | | |
|---|---|---|
| SEQ ID No 28 | OligoA_bc2 | /5Phos/GTGATTCTCGTCTTTCTGCGTAGTAGGTTC |
| SEQ ID No 29 | OligoA_bc3 | /5Phos/GTACTTTTCTCTTTGCGCGGTAGTAGGTTC |
| SEQ ID No 30 | OligoA_bc4 | /5Phos/GGTCTTCGCTCGGTCTTATTTAGTAGGTTC |
| SEQ ID No 31 | OligoA_bc23 | /5Phos/GCAGCTGGATGCGAGAGGGAAGCTGGTTGAAATAATATA GTAGGTTC |
| SEQ ID No 32 | OligoA_bc48 | /5Phos/GTTCCGCGCACGCTTTTCTCGTTTTGTCTTTTACACTTAGT AGGTTC |
| SEQ ID No 33 | OligoA_bc82 | /5Phos/GGGAAAGAGCGGTACACTCTCCGAGGGAGAGGCCCACT AGTAGGTTC |
| SEQ ID No 34 | OligoB_bc2 | GAGGCGAGCGGTCAATTTTCGCAGAAAGACGAGAATCACTTTTTT TTTT |
| SEQ ID No 35 | OligoB_bc3 | GAGGCGAGCGGTCAATTTCCGCGCAAAGAGAAAAGTACTTTTTT TTTT |
| SEQ ID No 36 | OligoB_bc4 | GAGGCGAGCGGTCAATTTTAATAAGACCGAGCGAAGACCTTTTTT TTTT |
| SEQ ID No 37 | OligoB_bc23 | GAGGCGAGCGGTCAATTTTTATTATTTCAACCAGCTTCCCTCTCGC ATCCAGCTGCTTTTTTTTTT |
| SEQ ID No 38 | OligoB_bc48 | GAGGCGAGCGGTCAATTTTAGTGTAAAAGACAAAACGAGAAAAGC GTGCGCGGAACTTTTTTTTTT |
| SEQ ID No 39 | OligoB_bc82 | GAGGCGAGCGGTCAATTTTGTGGGCCTCTCCCTCGGAGAGTGTA CCGCTCTTTCCCTTTTTTTTTT |

| | | |
|---|---|---|
| Shown in bold - barcode Shown underlined - oligodT overhang required to anneal with tRNA template that has 3' adapter annealed, via splint. This overhang is used by default ONT library preps (originally designed to capture mRNAs). | | |

**Table 18. Validation of the demultiplexing method using independent Nano-tRNAseq datasets**

| **Run ID** | **Expect ed barco de** | **Predicted barcode** | | | | | | **Accur acy** | **Recov ery** |
|---|---|---|---|---|---|---|---|---|---|
| | | **bc_2** | **bc_3** | **bc_4** | **bc_2 3** | **bc_4 8** | **bc_8 2** | | |
| RNA150622_MCF7_ BR2 | **bc_2** | **214,6 72** | 1,336 | 541 | 371 | 254 | 298 | 0.987 | 0.994 |
| RNA140622_MCF10 BR1 | **bc_3** | 1,497 | **548,5 78** | 1,319 | 833 | 557 | 847 | 0.991 | 0.994 |
| RNA150622_MCF10 BR2 | **bc_4** | 358 | 898 | **109,6 79** | 252 | 246 | 377 | 0.981 | 0.992 |
| RNA010722_SKBR3 BR1 | **bc_23** | 1,244 | 3,578 | 1,543 | **325,8 04** | 2,198 | 1,020 | 0.971 | 0.999 |
| RNA010722_T47D_ BR1 | **bc_48** | 1,236 | 5,050 | 1,452 | 870 | **329,1 71** | 2,109 | 0.968 | 0.998 |
| RNA110722_GM108 63 BR2 | | 154 | 546 | 125 | 154 | **48,18 8** | 168 | 0.977 | 0.998 |
| RNA110722_GM128 78 BR2 | **bc_82** | 898 | 2,980 | 858 | 670 | 928 | **144,8 01** | 0.958 | 0.998 |

The model reaches 0.978 accuracy and over 0.999 recovery on validation set. Per-barcode accuracy: bc_2 0.982, bc_3 0.985, bc_4 0.977, bc_23 0.98, bc_48 0.979, bc_82 0.962

### Comparison to using DeePlexiCon for demultiplexing tRNA reads

DeePlexiCon proceeds in 3 steps: i) barcode identification in the signal space (segmentation), ii) signal to image conversion (GASF) and barcode classification using convolutional neural network typically applied in image analysis (ResNet). Currently, DeePlexiCon allows pooling up to 4 samples in a sequencing reaction, lowering the sequencing cost nearly 4 times. DeePlexiCon reaches 95% accuracy with 93% recovery (Smith MA, Ersavas T, Ferguson JM, et al. Molecular barcoding of native RNAs using nanopore sequencing and deep learning. Genome Res. 2020;30(9):1345-1353. doi:10.1101/gr.260836.120). However, DeePlexiCon performed very poorly with tRNA samples, reaching only 0.696 accuracy (Table 19), making it completely unusable for tRNA multiplexing. The most likely explanation of such low performance is the problem with segmentation due to the short polyA tail used in the Nano-RNA seq protocol (10 bases). The segmentation method used in DeePlexiCon was developed for long mRNA reads and those tend to have much longer polyA tails, often in the range of hundreds of bases.

**Table 19. Demultiplexing accuracy of tRNA reads using DeePlexiCon**

| **Reference** | **Expected barcode** | **Predicted barcode** | | | | **Accuracy** |
|---|---|---|---|---|---|---|
| | | **bc_1** | **bc_2** | **bc_3** | **bc_4** | |
| IVT_pDP-10 | bc_1 | **695** | 131 | 214 | 137 | 0.590 |
| IVT_pG76 | bc_2 | 474 | **3,173** | 818 | 486 | 0.641 |
| tRNAphe | bc_3 | 329 | 324 | **3,825** | 453 | 0.776 |

### Training a Bonito DNA basecalling model

We rely on the CTC-CRF model trained with bonito, using a chunk size of 3000 signal samples with 31 window and 10 stride. The CTC-CRF models consisting of 25 (sup) or 0.5 (fast) million parameters were trained using bonito for 50 epochs with learning rate 2e⁻³ using over 500 thousand chunks in total for 6 barcodes. 3% of the dataset was used as a validation set. The best performance on validation set was reached after 32 epochs and we use this as a final barcode basecalling model.

## Claims

1. A method to quantify tRNA abundance and tRNA modifications that comprises the following steps:
a) contacting an RNA sample containing tRNA, in the presence of an agent with ligating activity, with:
a pre-annealed splinted double-stranded oligonucleotide comprising:
- a first splinted oligonucleotide, comprising a second splinted oligonucleotide hybridization region and a tRNA hybridization region, said tRNA hybridization region being located on its 3' end,
- a second splinted oligonucleotide, comprising a first splinted oligonucleotide hybridization region, a second adapter DNA oligonucleotide hybridization region, such that at the end of the step, the first splinted oligonucleotide is adjacent to the 5' end of the tRNA and complementary annealed to both the 3' end of the tRNA by the tRNA hybridization region and to the second splinted oligonucleotide by the second splinted oligonucleotide hybridization region,
b) contacting the product of step a) in the presence of an agent with ligating activity with
a pre-annealed adapter DNA double-stranded oligonucleotide comprising:
- a first adapter DNA oligonucleotide, comprising a second DNA adapter oligonucleotide hybridization region, and
- a second adapter DNA oligonucleotide, comprising a first DNA adapter oligonucleotide hybridization region and a second splinted oligonucleotide hybridization region, being said second splinted oligonucleotide hybridization region complementary to the second adapter DNA oligonucleotide hybridization region of the second splinted oligonucleotide,
such that at the end of the step, the first adapter DNA oligonucleotide is adjacent to the 3' end of the terminal region of the second splinted RNA oligonucleotide, and the second DNA oligonucleotide is complementary annealed to both the second splinted RNA oligonucleotide and the first adapter DNA oligonucleotide,
c) performing reverse transcription to linearize the product of step b) to obtain a library and
d) carrying out nanopore direct sequencing with the library of step c), to obtain the abundance of tRNA and its modifications in said RNA sample.

2. The method according to the preceding claim, wherein step d) comprises:
- contacting the library of step c), in the presence of an agent with ligating activity, with an oligonucleotide adapter configured to perform nanopore direct sequencing,
- loading the product of the previous step to a flow cell which contains a membrane in which is present a nanopore that provides a channel through said membrane, coupled to a current intensity, wherein the product of the previous step passes through the nanopore, causes disruptions in the current intensity, and
- analyzing said sequences to obtain the abundance of tRNA and its modifications in said sample.

3. The method according to any of the preceding claims, wherein the ligating agent of step a) is an enzyme, preferably *E. coli* T4 RNA ligase2 with SEQ ID N° 2 or a variant having at least 80 % identity, more preferably 85% identity, even more preferably at least 90 % identity, and even more preferably 91 % or 92 % or 93 % or 94 % or 95 % or 96 % or 97 % or 98 % or even up to 99 % identity with respect to the SEQ ID No 2 polynucleotide sequence.

4. The method according to any of the preceding claims, wherein the duration of step a) is between 1hr and 3hr at a temperature comprised between 20°C and 26°C.

5. The method according to any of the preceding claims, wherein the second adapter DNA oligonucleotide hybridization region of the second splinted oligonucleotide is any sequence of at least 10 nucleotides complementary to the second adapter DNA oligonucleotide hybridization region of the second splinted oligonucleotide, preferably a poly-A tail of at least 10 A nucleotides.

6. The method according to any of the preceding claims, wherein the second splinted oligonucleotide is a RNA:DNA oligonucleotide, preferably having between 1 and 15 DNA nucleotides starting from the 3' end of said nucleotide.

7. The method according to any of the preceding claims 1 to 6, wherein the first RNA splinted oligonucleotide is SEQ ID No 3 and the second splinted oligonucleotide is SEQ ID No 4 or SEQ ID No 5
or a variant having at least 80%, preferably at least 85%, more preferably at least 90 % and even more preferably 91 % or 92 % or 93 % or 94 % or 95 % or 96 % or 97 % or 98 % or even up to 99 % identity with respect to SEQ ID No 3, SEQ ID No 4, SEQ ID No 5.

8. The method according to any of the preceding claims 2 to 7, wherein the parameters of the software configured to analyze the sequencing results of the nanopore direct sequencing are adjusted for up to 1 second definition for adapter and a maximum of 2 seconds for the strand.

9. The method according to any of the preceding claims 2 to 8, wherein the parameters for the analysis of the nanopore direct sequencing results use the BWA configuration, and the parameters are selected from the group consisting of:
j) bwa mem -W13 -k6 -xont2d,
ii) bwa mem -W13 -k6 -xont2d -T20,
iii) bwamem -W13 -k6 -xont2d -T10,
iv) bwamem -W9 -k5 -xont2d -T10 and
v) bwasw-z10 -a2 -b1 -q2 -r1,
preferably bwa mem -W13 -k6 -xont2d -T20

10. The method according to any of the preceding claims 1 to 7, wherein the hybridization region of both first and second DNA adapter oligonucleotide is a random nucleotide sequence between 15 and 45 nucleotides, unique for each pair of first and second DNA adapter oligonucleotide.

11. The method according to the preceding claim wherein more than one RNA sample containing tRNA are processed simultaneously through step d).

12. The method according to any of the preceding claims 10 or 11, wherein the performing algorithm for the analysis of the nanopore direct sequencing results is minimap2 with adjusted parameters: - xmap-ont -k6 -w3 -n1 -m10 -s13 -A1 -B1 -O1 -E1

13. A kit for performing the method described in any of the claims 1 to 12, comprising:
- a first splinted oligonucleotide, comprising a second splinted oligonucleotide hybridization region and a tRNA hybridization region, said tRNA hybridization region being located on its 3' end,
- a second splinted oligonucleotide, comprising a first splinted oligonucleotide hybridization region, a second adapter DNA oligonucleotide hybridization region,
- a first adapter DNA oligonucleotide, comprising a second DNA adapter oligonucleotide hybridization region, and
- a second adapter DNA oligonucleotide, comprising a first DNA adapter oligonucleotide hybridization region and a second splinted oligonucleotide hybridization region, being said second splinted oligonucleotide hybridization region complementary to the second adapter DNA oligonucleotide hybridization region of the second splinted oligonucleotide,

14. The Kit according to the preceding claim, wherein the second splinted oligonucleotide is an RNA:DNA oligonucleotide with at least 1 terminal DNA base on its 3' end.

15. The Kit according to any of the preceding claims 13 to 14, comprising a T4 RNA ligase 2 with the oligonucleotide sequence of SEQ ID N° 1 or SEQ ID N° 2 or a variant having at least 80 % identity, more preferably 85% identity, even more preferably at least 90 % identity, and even more preferably 91 % or 92 % or 93 % or 94 % or 95 % or 96 % or 97 % or 98 % or even up to 99 % identity with respect to the SEQ ID No 1 or SEQ ID No 2 sequence.
